(19) Europäisches Patentamt
European Patent Office
Office européen des brevets

(11) EP 1 164 142 A1

(12) **EUROPEAN PATENT APPLICATION**
published in accordance with Art. 158(3) EPC

(43) Date of publication:
**19.12.2001 Bulletin 2001/51**

(21) Application number: **00908064.9**

(22) Date of filing: **14.03.2000**

(51) Int Cl.[7]: **C07K 14/47**, C07K 7/06, C12N 15/12, C12P 21/02, C07K 16/18, A61K 38/17, G01N 33/50, C12N 5/18 // C12P21:02, C12R1:91

(86) International application number:
**PCT/JP00/01542**

(87) International publication number:
**WO 00/55197 (21.09.2000 Gazette 2000/38)**

(84) Designated Contracting States:
**AT BE CH CY DE DK ES FI FR GB GR IE IT LI LU MC NL PT SE**
Designated Extension States:
**AL LT LV MK RO SI**

(30) Priority: **15.03.1999 JP 6830299**
**28.07.1999 JP 21363599**
**05.08.1999 JP 22220099**

(71) Applicant: **Takeda Chemical Industries, Ltd.**
**Osaka-shi, Osaka 541-0045 (JP)**

(72) Inventors:
- **ONDA, Haruo**
  **Tsuchiura-shi, Ibaraki 300-0812 (JP)**
- **OGI, Kazuhiro**
  **Tsukuba-shi, Ibaraki 305-0045 (JP)**
- **KITADA, Chieko**
  **Sakai-shi, Osaka 590-0073 (JP)**
- **SUZUKI, Nobuhiro**
  **Tsukuba-shi, Ibaraki 305-0861 (JP)**
- **OHKUBO, Shoichi**
  **Usiku-shi, Ibaraki 300-1234 (JP)**
- **SHINTANI, Yasushi**
  **Tsukuba-shi, Ibaraki 305-0821 (JP)**
- **KIKUCHI, Kuniko**
  **Toride-shi, Ibaraki 302-0024 (JP)**

(74) Representative: **Best, Michael, Dr. et al**
**Lederer, Keller & Riederer Patentanwälte**
**Prinzregentenstrasse 16**
**80538 München (DE)**

# (54) NOVEL PROTEINS AND USES THEREOF

(57) The present invention relates to a novel secretory protein or a partial peptide derived therefrom, or a salt thereof, a method for producing the protein, a pharmaceutical composition the protein or the like, an antibody against the protein, a method/kit for screening for a compound, or a salt thereof, promoting or inhibiting the activity of the protein, the compound obtained by such screening, and a pharmaceutical composition the compound, and the like.

The protein, partial peptide or the like of the present invention can be used as a tissue regenerating agent after excision of a diseased tissue or a diagnostic agent for cancer, and the like. The antibody of the present invention can be used in quantifying the protein of the present invention in test samples. Further, the protein of the present invention is useful as a reagent for screening for a compound promoting or inhibiting the activity of the protein of the present invention



Printed by Jouve, 75001 PARIS (FR)

SEQUENCE LISTING

<110> Takeda Chemical Industries, Ltd.

<120> Novel Protein and Its Use

<130> B00033

<150> JP 11-068302

<151> 1999-03-15

<150> JP 11-213635

<151> 1999-07-28

<150> JP 11-222200

<151> 1999-08-05

<160> 9

<210> 1

<211> 252

<212> PRT

<213> Human

<400> 1

Met Ala Ala Ala Ala Ala Thr Lys Ile Leu Leu Cys Leu Pro Leu Leu
1 5 10 15

Leu Leu Leu Ser Gly Trp Ser Arg Ala Gly Arg Ala Asp Pro His Ser
20 25 30

Leu Cys Tyr Asp Ile Thr Val Ile Pro Lys Phe Arg Pro Gly Pro Arg
35 40 45

Trp Cys Ala Val Gln Gly Gln Val Asp Glu Lys Thr Phe Leu His Tyr
50 55 60

Asp Cys Gly Asn Lys Thr Val Thr Pro Val Ser Pro Leu Gly Lys Lys
65 70 75 80

Leu Asn Val Thr Thr Ala Trp Lys Ala Gln Asn Pro Val Leu Arg Glu
85 90 95

Val Val Asp Ile Leu Thr Glu Gln Leu Arg Asp Ile Gln Leu Glu Asn
100 105 110
Tyr Thr Pro Lys Glu Pro Leu Thr Leu Gln Ala Arg Met Ser Cys Glu
115 120 125
Gln Lys Ala Glu Gly His Ser Ser Gly Ser Trp Gln Phe Ser Phe Asp
130 135 140
Gly Gln Ile Phe Leu Leu Phe Asp Ser Glu Lys Arg Met Trp Thr Thr
145 150 155 160
Val His Pro Gly Ala Arg Lys Met Lys Glu Lys Trp Glu Asn Asp Lys
165 170 175
Val Val Ala Met Ser Phe His Tyr Phe Ser Met Gly Asp Cys Ile Gly
180 185 190
Trp Leu Glu Asp Phe Leu Met Gly Met Asp Ser Thr Leu Glu Pro Ser
195 200 205
Ala Gly Ala Pro Leu Ala Met Ser Ser Gly Thr Thr Gln Leu Arg Ala
210 215 220
Thr Ala Thr Pro Ser Ser Phe Ala Ala Ser Ser Ser Ser Ser Pro Ala
225 230 235 240
Ser Ser Ser Leu Ala Ser Glu Glu Ser Pro Leu Glu
245 250 252

<210> 2
<211> 756
<212> DNA
<213> Human
<400> 2

ATGGCAGCAG CCGCCGCTAC CAAGATCCTT CTGTGCCTCC CGCTTCTGCT CCTGCTGTCC 60
GGCTGGTCCC GGGCTGGGCG AGCCGACCCT CACTCTCTTT GCTATGACAT CACCGTCATC 120
CCTAAGTTCA GACCTGGACC ACGGTGGTGT GCGGTTCAAG GCCAGGTGGA TGAAAAGACT 180
TTTCTTCACT ATGACTGTGG CAACAAGACA GTCACACCTG TCAGTCCCCT GGGGAAGAAA 240

CTAAATGTCA CAACGGCCTG GAAAGCACAG AACCCAGTAC TGAGAGAGGT GGTGGACATA 300

CTTACAGAGC AACTGCGTGA CATTCAGCTG GAGAATTACA CACCCAAGGA ACCCCTCACC 360

CTGCAGGCCA GGATGTCTTG TGAGCAGAAA GCTGAAGGAC ACAGCAGTGG ATCTTGGCAG 420

TTCAGTTTCG ATGGGCAGAT CTTCCTCCTC TTTGACTCAG AGAAGAGAAT GTGGACAACG 480

GTTCATCCTG GAGCCAGAAA GATGAAAGAA AAGTGGGAGA ATGACAAGGT TGTGGCCATG 540

TCCTTCCATT ACTTCTCAAT GGGAGACTGT ATAGGATGGC TTGAGGACTT CTTGATGGGC 600

ATGGACAGCA CCCTGGAGCC AAGTGCAGGA GCACCACTCG CCATGTCCTC AGGCACAACC 660

CAACTCAGGG CCACAGCCAC CCCCTCATCC TTTGCTGCCT CCTCATCATC CTCCCCTGCT 720

TCATCCTCCC TGGCATCTGA GGAGAGTCCT TTAGAG 756

<210> 3

<211> 40

<212> DNA

<213> Artifical Sequence

<220>

<223>

<400> 3

GCGCTCGAAT TCCACCATGG CAGCAGCCGC CGCTACCAAG 40

<210> 4

<211> 76

<212> DNA

<213> Artifical Sequence

<220>

<223>

<400> 4

GCGGCCGCTC ACTTGTCATC GTCGTCCTTG TAGTCCTCTA AAGGACTCTC CTCAGATGCC 60

AGGGAGGATG AAGCAG 76

<210> 5

<211> 246

<212> PRT

<213> Human

<400> 5

Met Ala Ala Ala Ala Ala Thr Lys Ile Leu Leu Cys Leu Pro Leu Leu
1 5 10 15

Leu Leu Leu Ser Gly Trp Ser Arg Ala Gly Arg Ala Asp Pro His Ser
20 25 30

Leu Cys Tyr Asp Ile Thr Val Ile Pro Lys Phe Arg Pro Gly Pro Arg
35 40 45

Trp Cys Ala Val Gln Gly Gln Val Asp Glu Lys Thr Phe Leu His Tyr
50 55 60

Asp Cys Gly Asn Lys Thr Val Thr Pro Val Ser Pro Leu Gly Lys Lys
65 70 75 80

Leu Asn Val Thr Thr Ala Trp Lys Ala Gln Asn Pro Val Leu Arg Glu
85 90 95

Val Val Asp Ile Leu Thr Glu Gln Leu Arg Asp Ile Gln Leu Glu Asn
100 105 110

Tyr Thr Pro Lys Glu Pro Leu Thr Leu Gln Ala Arg Met Ser Cys Glu
115 120 125

Gln Lys Ala Glu Gly His Ser Ser Gly Ser Trp Gln Phe Ser Phe Asp
130 135 140

Gly Gln Ile Phe Leu Leu Phe Asp Ser Glu Lys Arg Met Trp Thr Thr
145 150 155 160

Val His Pro Gly Ala Arg Lys Met Lys Glu Lys Trp Glu Asn Asp Lys
165 170 175

Val Val Ala Met Ser Phe His Tyr Phe Ser Met Gly Asp Cys Ile Gly
180 185 190

Trp Leu Glu Asp Phe Leu Met Gly Met Asp Ser Thr Leu Glu Pro Ser
195 200 205

Ala Gly Ala Pro Leu Ala Met Ser Ser Gly Thr Thr Gln Leu Arg Ala

210 215 220

Thr Ala Thr Thr Leu Ile Leu Cys Cys Leu Leu Ile Ile Leu Pro Cys

225 230 235 240

Phe Ile Leu Pro Gly Ile

245 246

<210> 6

<211> 738

<212> DNA

<213> Human

<400> 6

ATGGCAGCAG CCGCCGCTAC CAAGATCCTT CTGTGCCTCC CGCTTCTGCT CCTGCTGTCC 60

GGCTGGTCCC GGGCTGGGCG AGCCGACCCT CACTCTCTTT GCTATGACAT CACCGTCATC 120

CCTAAGTTCA GACCTGGACC ACGGTGGTGT GCGGTTCAAG GCCAGGTGGA TGAAAAGACT 180

TTTCTTCACT ATGACTGTGG CAACAAGACA GTCACACCTG TCAGTCCCCT GGGGAAGAAA 240

CTAAATGTCA CAACGGCCTG GAAAGCACAG AACCCAGTAC TGAGAGAGGT GGTGGACATA 300

CTTACAGAGC AACTGCGTGA CATTCAGCTG GAGAATTACA CACCCAAGGA ACCCCTCACC 360

CTGCAGGCCA GGATGTCTTG TGAGCAGAAA GCTGAAGGAC ACAGCAGTGG ATCTTGGCAG 420

TTCAGTTTCG ATGGGCAGAT CTTCCTCCTC TTTGACTCAG AGAAGAGAAT GTGGACAACG 480

GTTCATCCTG GAGCCAGAAA GATGAAAGAA AAGTGGGAGA ATGACAAGGT TGTGGCCATG 540

TCCTTCCATT ACTTCTCAAT GGGAGACTGT ATAGGATGGC TTGAGGACTT CTTGATGGGC 600

ATGGACAGCA CCCTGGAGCC AAGTGCAGGA GCACCACTCG CCATGTCCTC AGGCACAACC 660

CAACTCAGGG CCACAGCCAC CACCCTCATC CTTTGCTGCC TCCTCATCAT CCTCCCCTGC 720

TTCATCCTCC CTGGCATC 738

<210> 7

<211> 63

<212> DNA

<213> Artifical Sequence

<220>

<223>

<400> 7

GCGCTGAATT CCCACCATGG CAGCAGCCGC CGCTACCAAG ATCCTTCTGT GCCTCCCGCT 60

TCT 63

<210> 8

<211> 71

<212> DNA

<213> Artifical Sequence

<220>

<223>

<400> 8

TTGCGGCCGC TCACTTGTCA TCGTCGTCCT TGTAGTCGAT GCCAGGGAGG ATGAAGCAGG 60

GGAGGATGAT G 71

<210> 9

<211> 47

<212> DNA

<213> Artifical Sequence

<220>

<223>

<400> 9

TTGCGGCCGC TCAGATGCCA GGGAGGATGA AGCAGGGGAG GATGATG 47

## Description

TECHNICAL FIELD

The present invention relates to a novel secretory protein, among others.

BACKGROUND ART

The oncogenes so far discovered include a large number of cell proliferation factor genes or receptor genes such as PDGF associated with brain tumor (Ross R., Nature 1993, 362:801), erb-B and erb-B2 associated with mammary cancer (Cell 1983; 35, 718, Burden S., Neuron 1997; 18, 847) and the like and, in addition, a large number of genes such as signal transduction promoting Ki-ras associated with colorectal cancer, N-ras involved in leukemia, c-myc relating with cell proliferation promoting gene activating transcription factor and involved in leukemia, mammary cancer and stomach cancer, among others (Maheswaran, Mol. Cell. Biol. 1994; 14 (2) 1147), N-myc involved in neuroblastoma (Schwab, Nature 1983; 305, 245), L-myc involved in lung cancer (Nau, Nature 1985; 318, 69) and, further, Bcl-2, Bcl-1, MDM2 and like proteins associated with follicular B cell lymphoma, mammary cancer, cervical cancer or proteins antagonizing p53 tumor suppressor protein (Reed, Nature 1997; 387, 773; Donehower, Nature 1992, 356, 215). Apart from these genes, a number of genes functioning to suppress cancer cell proliferation, such as APC, DPC4, NF-1, NF-2, MTS1, RB, P53 and WT1, have been found (Weinberg, Scientific American, 1996, September, 32). These genes have been identified based on the amino acid sequence information provided by analyzing specific proteins found upon malignant transformation of cells or tissues and the details of the discoveries are variegated. These genes so far discovered are by themselves not enough to explain oncogenesis and/or recovery from cancer.

Further, as a result of recent human gene analysis, a number of nucleotide sequences have been reported for ESTs (expressed sequence tags) with unknown function.

It has therefore been desired that a novel treating or preventing agent for cancer be developed by finding out genes coding for tumor-specific proteins from among ESTs whose functions are unknown.

DISCLOSURE OF INVENTION

The present inventors made intensive investigations in an attempt to solve the above problem and, as a result, found a secretory protein gene having a novel nucleotide sequence on the basis of the nucleotide sequences of ESTs and found that the secretory protein encoded thereby is expressed specifically in tumor cells.

As a result of further investigations based on such and other findings, the present inventors have now completed the present invention.

Thus, the present invention provides:

(1) A protein which comprises an amino acid sequence identical or substantially identical with the amino acid sequence shown by SEQ ID NO:1, or a salt thereof;
(2) A protein, or a salt thereof, as mentioned above under (1) in which the amino acid sequence substantially identical with the amino acid sequence shown by SEQ ID NO:1 is the amino acid sequence shown by SEQ ID NO:5;
(3) A protein, or a salt thereof, as mentioned above under (1) which is produced in cancer cells;
(4) A partial peptide derived from the protein mentioned above under (1), or a salt thereof;
(5) A partial peptide, or a salt thereof, as mentioned above under (4) which has the amino acid sequence from the 26th to 34th amino acid residues in the amino acid sequence shown by SEQ ID NO:1;
(6) A partial peptide, or a salt thereof, as mentioned above under (4) which has the amino acid sequence from the 166th to 190th amino acid residues in the amino acid sequence shown by SEQ ID NO:5;
(7) A method for producing the protein mentioned above under (1) or the partial peptide mentioned above under (4), or a salt thereof, which comprises cultivating a transformant harboring a recombinant vector containing a DNA coding for the protein mentioned above under (1) or the partial peptide mentioned above under (4) to thereby cause formation of said protein or partial peptide;
(8) An antibody against the protein mentioned above under (1) or the partial peptide mentioned above under (4), or a salt thereof;
(9) An antibody as mentioned above under (8) which is a monoclonal antibody;
(10) An antibody as mentioned above under (8) which is an antibody against the partial peptide as mentioned above under (4), or a salt thereof, having the amino acid sequence from the 26th to 34th amino acid residues in the amino acid sequence shown by SEQ ID NO:1;
(11) An antibody as mentioned above under (8) which is an antibody against the partial peptide as mentioned above under (4), or a salt thereof, having the amino acid sequence from the 166th to 190th amino acid residues in the amino acid sequence shown by SEQ ID NO:5;

(12) A diagnostic agent which comprises the antibody mentioned above under (8);

(13) A pharmaceutical composition which comprises the protein mentioned above under (1) or the partial peptide mentioned above under (4), or a salt thereof, or the antibody mentioned above under (8);

(14) A method for screening for a compound, or a salt thereof, enhancing or inhibiting an activity of the protein mentioned above under (1) or the partial peptide mentioned above under (4), or a salt thereof, which method is characterized by using the protein mentioned above under (1) or the partial peptide mentioned above under (4), or a salt thereof;

(15) A method for screening as mentioned above under (14) in which the activity is cell proliferating activity;

(16) A kit for screening for a compound, or a salt thereof, enhancing or inhibiting an activity of the protein mentioned above under (1) or the partial peptide mentioned above under (4), or a salt thereof, which kit comprises the protein mentioned above under (1) or the partial peptide mentioned above under (4), or a salt thereof;

(17) A compound, or a salt thereof, enhancing or inhibiting an activity of the protein mentioned above under (1) or the partial peptide mentioned above under (4), or a salt thereof, which are obtainable by using the screening method mentioned above under (14) or the screening kit mentioned above under (16);

(18) A pharmaceutical composition which comprises a compound, or a salt thereof, enhancing or inhibiting an activity of the protein mentioned above under (1) or the partial peptide mentioned above under (4), or a salt thereof, which are obtainable by using the screening method mentioned above under (14) or the screening kit mentioned above under (16); and

(19) A pharmaceutical composition as mentioned above under (18) which is for preventing or treating a cancer; among others.

The present invention further provides:

(20) A protein, or a salt thereof, as mentioned above under (1) in which the amino acid sequence substantially identical with the amino acid sequence shown by SEQ ID NO:1 is an amino acid sequence having not less than about 70%, preferably not less than about 80%, more preferably not less than about 90%, still more preferably not less than about 95%, homology with the amino acid sequence shown by SEQ ID NO:1;

(21) A protein, or a salt thereof, as mentioned above under (1) in which the amino acid sequence substantially identical with the amino acid sequence shown by SEQ ID NO:1 is (1) an amino acid sequence resulting from deletion of 1 to 5 (preferably 1 to 3) amino acid residues from the amino acid sequence shown by SEQ ID NO:1, (2) an amino acid sequence resulting from addition of 1 to 10 (preferably 1 to 5 (more preferably 1 to 3)) amino acid residues to the amino acid sequence shown by SEQ ID NO:1, (3) an amino acid sequence resulting from substitution of some other amino acid residue or residues for 1 to 5 (preferably 1 to 3) amino acid residues in the amino acid sequence shown by SEQ ID NO:1 or (4) an amino acid sequence resulting from a combination thereof;

(22) A screening method as mentioned above under (14) which comprises (i) bringing a substrate into contact with the protein mentioned above under (1) or the partial peptide mentioned above under (4), or a salt thereof, (ii) bringing the substrate and a test compound into contact with the protein mentioned above under (1) or the partial peptide mentioned above under (4), or a salt thereof, measuring the activity of the protein mentioned above under (1) or the partial peptide mentioned above under (4), or a salt thereof, in each case and comparing the activity values obtained;

(23) A pharmaceutical composition which comprises a compound, or a salt thereof, enhancing the activity of the protein mentioned above under (1) or the partial peptide mentioned above under (4), or a salt thereof, which are obtainable by using the screening method mentioned above under (14) or the screening kit mentioned above under (16) ;

(24) A pharmaceutical composition as mentioned above under (23) which is a tissue regenerating agent for use after excision of a diseased tissue;

(25) A pharmaceutical composition which comprises a compound, or a salt thereof, inhibiting the activity of the protein mentioned above under (1) or the partial peptide mentioned above under (4), or a salt thereof, which are obtainable by using the screening method mentioned above under (14) or the screening kit mentioned above under (16);

(26) A pharmaceutical composition as mentioned above under (25) which is for treating or preventing a cancer;

(27) A method for quantifying the protein mentioned above under (1) or the partial peptide mentioned above under (4), or a salt thereof, which comprises reacting the antibody mentioned above under (8) competitively with the test solution and a labeled form of the protein mentioned above under (1) or of the partial peptide mentioned above under (4), or a salt thereof and determining the proportion of the labeled form of the protein mentioned above under (1) or of the partial peptide mentioned above under (4), or a salt thereof, as bound to said antibody; and

(28) A method for quantifying the protein mentioned above under (1) or the partial peptide mentioned above under (4), or a salt thereof, which comprises reacting the test solution with the antibody mentioned above under (8) as immobilized on a carrier and a labeled form of the antibody mentioned above under (8) simultaneously or successively and measuring the activity of the label on the immobilizing carrier; among others.

The present invention also provides:

(29) A hybridoma having the ability to produce the monoclonal antibody mentioned above under (9);
(30) An antibody as mentioned above under (8) which is a neutralizing antibody;
(31) An antibody as mentioned above under (8) which is a humanized antibody;
(32) A pharmaceutical composition which comprises the antibody mentioned above under (31);
(33) A pharmaceutical composition as mentioned above under (32) which is for treating or preventing a cancer;
(34) A pharmaceutical composition which comprises a compound, or a salt thereof, having an inhibitory activity on the inhibition of natural killer cell proliferation by the protein mentioned above under (1) or the partial peptide mentioned above under (4), or a salt thereof;
(35) A pharmaceutical composition as mentioned above under (34) which is for treating or preventing a cancer; and so forth.

BRIEF DESCRIPTION OF THE DRAWINGS

Fig. 1 shows the nucleotide sequence of the DNA obtained in Example 1, coding for a protein of the present invention as well as the amino acid sequence deduced therefrom.

Fig. 2 is an electrophoretogram showing the results of the northern blotting carried out in Example 2. In A (for normal tissues), lane 1 is for brain, lane 2 - heart, lane 3 - skeletal muscle, lane 4 - large intestine, lane 5 - thymus, lane 6 - spleen, lane 7 - kidney, lane 8 - liver, lane 9 - small intestine, lane 10 - placenta, lane 11 - lung and lane 12 - peripheral blood and, in B (for cancel cells), lane 1 is for promyelocytic leukemia cell HL60, lane 2 - cervical cancer cell HeLa, lane 3 - chronic myelogenous leukemia cell K-562, lane 4 - lymphoblastic leukemia cell MOLT4, lane 5 - Burkitt lymphoma cell Raji, lane 6 - colon cancer cell SW480, lane 7 - lung cancer cell A549, and lane 9 - melanoma cell G361.

Fig. 3 is an electrophoretogram showing the results of the northern blotting carried out in Example 2. In C-1, lane 1 is for human lung cancer cell RERF-LC-A1, lane 2 - human lung cancer cell NCI-H345, lane 3 - human lung cancer cell NCI-H460, lane 4 - human lung cancer cell NCI-H1299, lane 5 - normal human fetal fibroblastic cell MRC-5, lane 6 - normal human fetal fibroblastic cell WI-38, lane 7 - colorectal cancer cell HT-29, lane 8 - colorectal cancer cell WiDr, and lane 9 - mammary cancer cell MCF-7.
In C-2, lane 1 is for neuroblastoma cell GOTO-P3, lane 2 - neuroblastoma cell IMR-32, lane 3 - glioma cell U-251, lane 4 - glioma cell KNS42, and lane 5 - glioma cell KNS81.
In C-3, lane 1 is for lung cancer cell NCI-H345, lane 2 - neuroblastoma cell IMR-32, lane 3 - prostatic cancer cell LNCap, lane 4 - prostatic cancer cell PC-3, lane 5 - neuroblastoma cell GOTO-P3, lane 6 - promyelocytic leukemia cell HL-60, lane 7 - myeloma cell Bowes, and lane 8 - mammary cancer cell MCF-7.

Fig. 4 is an electrophoretogram showing the results of the western blotting carried out in Example 4.
In the figure, lanes 1 and 2 each is for cell lysate, lanes 3 and 4 each - culture supernatant, and lane 5 - a mock (culture supernatant of COS-7 cells alone).

Fig. 5 shows the nucleotide sequence of the DNA obtained in Example 5, coding for a protein of the present invention as well as the amino acid sequence deduced therefrom.

Fig. 6 is an electrophoretogram showing the results of the northern blotting carried out in Example 6.
In the figure, lane 9 is for human gastric cancer cell AGS, lane 10 - human pancreatic carcinoma cell PANC-1, lane 11 - human endometrioma cell AN3CA, lane 12 - human endometrioma cell KLE, lane 13 and lane 14 each - human chondroma cell W1353, lane 15 - human endometrioma cell SKN, and lane 16 - renal adenocarcinoma cell ACHN.

Fig. 7 shows the results of the determination of antibody titers in mouse antisera as performed in Example 14. In the figure, ■ shows the absorbance of the well with mouse 1 antiserum added, □ with mouse 2 antiseum added, ▲ with mouse 3 antiserum added, ∆with mouse 4 antiserum added, ● with mouse 5 antiserum added, ○ with mouse 6 antiserum added, | with mouse 7 antiserum added, and ◊with mouse 8 antiserum added.

Fig. 8 shows the results of the competitive EIA conducted in Example 17.
In the figure, ■ shows the $B/B_0$ value for the monoclonal antibody produced by hybridoma No. 39-35, □ by hybridoma No. 53-16, ▲ by hybridoma No. 61-2, ∆by hybridoma No. 76-12, ● by hybridoma No. 85-16, ○ by hybridoma No. 112-3, ♦by hybridoma No. 128-18, ◊by hybridoma No. 139-26, and ×by hybridoma No. 151-2.

The dilution factor for each culture supernatant was as follows:

| | |
|---|---|
| No. 39-35 | 50 times; |
| No. 53-16 | 50 times; |
| No. 61-2 | 120 times; |
| No. 76-12 | 60 times; |
| No. 85-16 | 120 times; |
| No. 112-3 | 450 times; |
| No. 128-18 | 450 times; |
| No. 139-26 | 120 times; |
| No. 151-2 | 60 times. |

Fig. 9 shows the result of the western blot analysis of the TGC838 protein and TGC839 protein as carried out in Example 24.
In the figure, lanes 1, 3 and 5 each is a lane obtained by electrophoresis of the culture supernatant of CHO-K1/TGC838N-4, and lanes 2, 4 and 6 each of the culture supernatant of CHO-K1/618/839F6-3. Antibody 128-18 (Example 18) was used as the primary antibody for lanes 1 and 2, antibody 112-3 (Example 18) for lanes 3 and 4, and rabbit antiserum (Example 19) for lanes 5 and 6.
Fig. 10 shows the result of the western blot analysis of the TGC838 protein and TGC839 protein as carried out in Example 24.
In the figure, lanes 1 and 2 each is a lane obtained by electrophoresis of the culture supernatant of COS7 with the pCAN618/H838 DNA introduced therein, lanes 3 and 4 each of COS7 with the pCAN618/H838F DNA introduced therein, lanes 5 and 6 each of COS7 with the pCAN618/H839F DNA introduced therein, and lanes 7 and 8 each of COS7 with the pCAN618 DNA introduced therein. Mouse antiserum (Example 12) was used as the primary antibody.
Fig. 11 shows the result of the western blot analysis of the TGC838 protein and TGC839 protein as carried out in Example 24.
In the figure, lanes 3 and 4 each is a lane obtained by electrophoresis of the culture supernatant of COS7 with the pCAN618/H838F DNA introduced therein, lanes 5 and 6 each of COS7 with the pCAN618/H839F DNA introduced therein, and lanes 7 and 8 each of COS7 with the pCAN618 DNA introduced therein. Anti-FLAG mouse IgG was used as the primary antibody.
Fig. 12 shows the result of the immunoprecipitation test using antibodies against the TGC838 protein and TGC839 protein as carried out in Example 25.
In the figure, lanes 1, 3 and 5 each is for the culture supernatant of COS7 cells with the pCAN618/H838F DNA introduced therein, and lanes 2, 4 and 6 each of COS7 cells with the pCAN618/H839F DNA introduced therein. The immunoprecipitation was effected using antibody 128-18 (Example 18) for lanes 1 and 2, and rabbit antiserum (Example 19) for lanes 3 and 4; lanes 5 and 6 are controls (no antibody added).
Fig. 13 shows the results of the testing for confirming sugar chain addition as carried out in Example 26.
In the figure, lanes 1 and 2 each is a lane obtained by electrophoresis of the culture supernatant of COS7 cells with the pCAN618/H838 DNA introduced therein, lanes 3 and 4 each of COS7 cells with the pCAN618/H838F DNA introduced therein, lanes 5 and 6 each of COS7 cells with the pCAN618/H839F DNA introduced therein, and lanes 7 and 8 each of COS7 cells with the pCAN618 DNA introduced therein. Lanes 1, 3 and 5 are lanes obtained after treatment with N-glycosidase.
Fig. 14 shows the working curve obtained in Example 28 and corresponds to Table 1.
Fig. 15 shows the results of the quantifying TGC838 as performed in Example 28.
In the figure, □ indicates the amount of TGC838 in the culture supernatant of CHO-K1/TGC838N-4, ◊of COS7 with the pCAN618/H838 DNA introduced therein, ○ of SW1353-1, and Δof SW1353-2.
Fig. 16 shows the results of the quantifying TGC838 as performed in Example 28.
In the figure, ○ indicates the plasma level of TGC838 in healthy normal subjects, ◊in patients with hepatocarcinoma, and Δin patients with colorectal carcinoma.
Fig. 17 shows the results of the FACS analysis performed in Example 29.
In the figure, the solid line indicates the cell group with the TGC839FLAG protein added, and the broken line the cell group without addition of the TGC839FLAG protein.
Fig. 18 shows the results of the FACS analysis performed in Example 29.
In the figure, +FITC-TGC839F indicates the cell group with the TGC839FIAG protein added, and -FITC-TGC839F the cell group without addition of the TGC839FLAG protein.
Fig. 19 shows the results of the testing of the TGC839 protein conducted in Example 30 for influences on the cytotoxicity of NK cells.

In the figure, ○ indicates the TGC839FLAG concentration of 0 ng/ml, □ 50 ng/ml, Δ150 ng/ml, and ◊500 ng/ml.

Fig. 20 shows the results of the testing of the TGC839 protein conducted in Example 31 for influences on the proliferation of NK cells.

In the figure, □ and ○ denote peripheral blood samples derived from different individuals.

Fig. 21 shows the results of the testing of the TGC839 protein conducted in Example 31 for influences on the proliferation of K562 cells.

Fig. 22 shows the results of the quantifying conducted in Example 31 of the culture supernatant for IFN-γ.

BEST MODES FOR CARRYING OUT THE PRESENT INVENTION

The protein (hereinafter referred to as "protein of the present invention ") which comprises an amino acid sequence identical or substantially identical with the amino acid sequence shown by SEQ ID NO:1 may be a protein derived from human or warm-blooded animal (e.g. guinea pig, rat, murine, chicken, rabbit, porcine, ovine, bovine, simian) cells (e.g. hepatocytes, splenocytes, nerve cells, glia cells, pancreatic β cells, bone marrow cells, mesangium cells, Langerhans cells, epidermic cells, epithelial cells, endothelial cells, fibroblasts, fibrocytes, myocytes, adipocytes, immunocytes (e.g. macrophages, T cells, B cells, natural killer cells, mast cells, neutrophils, basophils, eosinophils, monocytes), megakaryocytes, synovial cells, chondrocytes, osteocytes, osteoblasts, osteoclasts, mammary gland cells, hepatocytes or interstitial cells, or progenitor cells thereof, stem cells or cancer cells), or any tissue comprising such cells, for example brain, any segment of brain (e.g. olfactory bulb, amygdala, basal ganglia, hippocampus, thalamus, hypothalamus, cerebral cortex, medulla oblongata, cerebellum), spinal cord, pituitary gland, stomach, pancreas, kidney, liver, gonad, thyroid, gallbladder, bone marrow, adrenal, skin, muscle, lung, digestive tract (e.g. large intestine, small intestine), blood vessel, heart, thymus, spleen, submandibular gland, peripheral blood, prostate, testis, ovary, placenta, uterus, bone, joint and skeletal muscle, or from blood cells or cultured cells thereof (e.g. MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01), or may be a synthetic protein. The protein of the present invention is preferably of fetal human or warm-blooded animal (e.g. guinea pig, rat, murine, chicken, rabbit, porcine, ovine, bovine, simian) cell (in particular fetal brain or lung cell) origin or of cancer cell origin.

As the amino acid sequence of the present invention which is substantially identical with the amino acid sequence shown by SEQ ID NO:1, there may be mentioned, among others, amino acid sequences not less than about 70%, preferably not less than about 80%, more preferably not less than about 90%, still more preferably not less than about 95%, homologous with said amino acid sequence shown by SEQ ID NO:1.

Preferred as the protein of the present invention which has an amino acid sequence substantially identical with the amino acid sequence shown by SEQ ID NO:1 are, for example, proteins having an amino acid sequence substantially identical with said amino acid sequence shown by SEQ ID NO:1 and having substantially the same activity as that of the protein having the amino acid sequence shown by SEQ ID NO:1.

More specifically, there may be mentioned the protein having the amino acid sequence shown by SEQ ID NO:5 and the like.

Substantially the same activity as mentioned above is, for example, cell proliferating activity.

When mention is made of "substantially the same activity", it is meant that both under comparison are of the same quality (e.g. physiologically or pharmacologically). Therefore, it is preferred that the activity such as cell proliferating activity is comparable (e.g. about 0.1-100 times, preferably about 0.5-10 times, more preferably about 0.5-2 times). Quantitative elements such as the level of such activity and the molecular weight of the protein may differ, however.

The cell proliferation can be determined by any of the publicity known methods, for example by the screening method to be mentioned later herein.

The protein of the present invention also includes the so-called muteins such as, for example, proteins comprising (1) an amino acid sequence derived from the amino acid sequence shown by SEQ ID NO:1 by deletion of 1 to 5 (preferably 1 to 3) amino acid residues, (2) an amino acid sequence derived from the amino acid sequence shown by SEQ ID NO:1 by addition of 1 to 10 (preferably 1 to 5 (more preferably 1 to 3)) amino acid residues, (3) an amino acid sequence derived from the amino acid sequence shown by SEQ ID NO:1 by insertion of 1 to 5 (preferably 1 to 3) amino acid residues, (4) an amino acid sequence derived from the amino acid sequence shown by SEQ ID NO:1 by substitution of some other amino acid residue or residues for 1 to 5 (preferably 1 to 3) amino acid residues, or (5) an amino acid sequence derived by a combination thereof.

In cases where there is an insertion, deletion or substitution in the amino acid sequence, as mentioned above, the site or sites of the insertion, deletion or substitution are not particularly restricted.

In the present specification, proteins are described according to the conventional practice in describing peptides, with the N terminus (amino terminus) at the left end and the C terminus (carboxyl terminus) at the right end. In the protein of the present invention , typically the protein comprising the amino acid sequence shown by SEQ ID NO: 1, the C terminus is generally a carboxyl group (-COOH) or carboxylate ($-COO^-$). The C terminus may also be an amide ($-CONH_2$) or ester (-COOR), however.

Usable as R in the above ester are a $C_{1-6}$ alkyl group such as methyl, ethyl, n-propyl, isopropyl or n-butyl, a $C_{3-8}$ cycloalkyl group such as cyclopentyl or cyclohexyl, a $C_{6-12}$ aryl group such as phenyl or α-naphthyl, a $C_{7-14}$ aralkyl group, for example a phenyl-$C_{1-2}$ alkyl group such as benzyl or phenethyl or an α-naphthyl-$C_{1-2}$ alkyl group such as α-naphthylmethyl and, further, a pivaloyloxymethyl group and the like, which are generally used as esters for oral administration.

In cases where the protein of the present invention has a carboxyl (or carboxylate) group(s) at other sites than the C terminus, those proteins in which such carboxyl group have been amidated or esterified also fall within the scope of the present invention. The ester in such a case may be the same as the C-terminal ester mentioned above.

The protein of the present invention further includes those whose N-terminal amino acid residue (e.g. methionine residue) is protected with a protective group (e.g. a $C_{1-6}$ acyl group, for example formyl or $C_{1-6}$ alkanoyl such as acetyl), those in which the N-terminal glutamine residue formed upon in vivo cleavage has been converted to a pyroglutamic acid residue, those in which a substituent(s) on the side chain(s) of an amino acid residue within the molecule (e.g. -OH, -SH, amino group, imidazole group, indole group, guanidino group) have been protected with an appropriate protective group (e.g. a $C_{1-6}$ acyl group, for example, formyl or $C_{1-6}$ alkanoyl group such as acetyl), and conjugated proteins, for example the so-called glycoproteins resulting from binding of a sugar chain.

Useful as specific examples of the protein of the present invention are the human-derived protein (Fig. 1) having the amino acid sequence shown by SEQ ID NO:1 and the human-derived protein (Fig. 5) having the amino acid sequence shown by SEQ ID NO:5, among others.

The protein of the present invention is characterized in that it is expressed specifically in cancer cells, or in the fetal brain, fetal lung or the like, preferably in cancer cells.

The partial peptide of the present invention which is derivative of the above protein may be any partial peptide derivative of the above-mentioned protein of the present invention that preferably has the same activity (e.g. cell proliferating activity) as the above-mentioned protein of the present invention . For example, use may be made of peptides having at least 20%, preferably not less than 50%, more preferably not less than 70%, still more preferably not less than 90%, most preferably not less than 95%, of the amino acid sequence constituting the protein of the present invention and having cell proliferating activity.

Among these peptides, those having an amino acid sequence comprising the amino acid sequence covering the 22nd-252nd, 26th-252nd or 26th-34th (in particular 26th-252nd or 26th-34th, particularly preferably 26th-34th) amino acid residues of the amino acid sequence shown by SEQ ID NO:1 or the amino acid sequenced covering the 22nd-246th, 26th-246th or 166th-190th (in particular 26th-246th or 166th-190th, particularly preferably 166th-190th) amino acid residues of the amino acid sequence shown by SEQ ID NO:5, for instance, are useful.

In the partial peptide of the present invention , 1 to 5 (preferably 1 to 3) amino acid residues may have been deleted from the amino acid sequence thereof, or 1 to 10 (preferably 1 to 5 (more preferably 1 to 3)) amino acid residues may have been added to the amino acid sequence thereof, or 1 to 5 (preferably 1 to 3) amino acid residues may have been inserted in the amino acid sequence thereof, or 1 to 5 (preferably 1 to 3) amino acid residues in the amino acid sequence thereof may have been substituted by some other amino acid residues.

The partial peptide of the present invention generally has a carboxyl group (-COOH) or carboxylate ($-COO^-$) as the C terminus but, as mentioned hereinabove with regard to the protein of the present invention , its C terminus may be an amide ($-CONH_2$) or ester (-COOR) (R being as defined above).

Further, like the above-mentioned protein of the present invention , the partial peptide of the present invention also includes those in which the amino group of an N-terminal amino acid residue (e.g. methionine residue) is protected with a protective group, those in which a glutamine residue on the N-terminal side as formed upon in vivo cleavage has been converted to a pyroglutamic acid residue, those in which a substituent on a side chain of an amino acid residue within the molecule is protected with an appropriate protective group, or conjugated peptides such as the so-called glycopeptides resulting from binding of a sugar chain.

The partial peptide of the present invention can be used as an antigen for antibody production and therefore need not always have cell proliferating activity.

As regards the salt form of the protein or partial peptide of the present invention , salts with physiologically acceptable acids (e.g. inorganic acids, organic acids) or bases (e.g. alkali metals) or the like are used and, among others, physiologically acceptable acid addition salts are preferred. As such salts, use is made of salts with inorganic acids (e.g. hydrochloric acid, phosphoric acid, hydrobromic acid, sulfuric acid), or salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid), among others.

The protein of the present invention , inclusive of salts thereof, can be produced from the above-mentioned human or warmblooded animal cells (in particular cancer cells or the like) or tissues (in particular fetal brain or lung or the like) by a publicity known method for protein purification or can be produced by cultivating a transformant harboring a DNA coding for the protein, as mentioned later herein. It can also be produced by a method for peptide synthesis, as mentioned later herein.

In the production from a human or mammalian tissue or cells (in particular cancer cells, fetal brain or lung or the like), the human or mammalian tissue or cells are homogenized and then extracted with an acid, for instance, and the extract is subjected to combined chromatographic techniques, such as reversed-phase chromatography and ion-exchange chromatography, whereby the protein can be isolated and purified.

In synthesizing the protein or partial peptide of the present invention , inclusive of salts thereof or the amide form thereof, a synthetic resin for protein synthesis, which is generally available on the market, can be used. As such resin, there may be mentioned, for example, chloromethyl resins, hydroxymethyl resins, benzhydrylamine resins, aminomethyl resins, 4-benzyloxybenzyl alcohol resins, 4-methylbenzhydrylamine resins, PAM resins, 4-hydroxymethylmethylphenylacetamidomethyl resins, polyacrylamide resins, 4-(2',4'-dimethoxyphenyl-hydroxymethyl)phenoxy resins, 4-(2',4'-dimethoxyphenyl-Fmoc-aminoethyl)phenoxy resins and the like. Using such a resin, amino acids with the $\alpha$-amino group and side chain functional group adequately protected are subjected to condensation on the resin by a publicity known condensation method according to the sequence of the desired protein. After the last reaction, the protein is excised from the resin and, at the same time, various protective groups are eliminated and, further, the intramolecular disulfide bond formation reaction is carried out in a highly diluted solution, whereby the desired protein or the amide form thereof can be obtained.

Referring to the above-mentioned condensation of protected amino acids, various activating reagents usable in protein synthesis can be used and, in particular, carbodiimides are preferred. Useful carbodiimides are DCC, N,N'-diisopropylcarbodiimide and N-ethyl-N'-(3-dimethylaminopropyl)carbodiimide, among others. For the activation with these, each protected amino acid may be added directly to the resin together with a racemization inhibiting additive (e.g. HOBt, HOOBt), or each protected amino acid may be activated as a symmetric acid anhydride or an HOBt or HOOBt ester in advance and then added to the resin.

The solvent to be used in the activation of protected amino acids or the condensation thereof with the resin can adequately be selected from among those known to be usable in protein condensation reactions. Thus, for example, use may be made of acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, pyridine, ethers such as dioxane and tetrahydrofuran, nitriles such as acetonitrile and propionitrile, esters such as methyl acetate and ethyl acetate, and appropriate mixtures of these. The reaction temperature is adequately selected from the range known to be usable in protein bond formation reactions, generally within the range of about -20°C to 50°C. Each activated amino acid derivative is used generally in 1.5 to 4 times excess. If testing by the ninhydrin reaction shows that the condensation is insufficient, the condensation reaction can be repeated without eliminating the protective group to thereby attain sufficient condensation. If sufficient condensation cannot be attained even by repetition of the reaction, the unreacted amino acid may be rendered inert to the succeeding reactions by acetylating the same using acetic anhydride or acetylimidazole.

Useful as the protective group for the amino group of each starting material are, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl, Fmoc and the like.

The carboxyl group can be protected, for example, by alkyl esterification (e.g. methyl, ethyl, propyl, butyl, t-butyl, cyclopentyl, cyclohexyl, cycloheptyl, cyclooctyl, 2-adamantyl or like straight, branched or cyclic alkyl esterification), aralkyl esterification (e.g. benzyl esterification, 4-nitrobenzyl esterification, 4-methoxybenzyl esterification, 4-chlorobenzyl esterification or benzhydryl esterification), phenacyl esterification, benzyloxycarbonylhydrazide formation, t-butoxycarbonylhydrazide formation, or tritylhydrazide formation.

The hydroxyl group of serine can be protected by esterification or etherification. Usable as the group suited for this esterification are, for example, lower ($C_{1-6}$) alkanoyl groups such as acetyl, aroyl groups such as benzoyl, and carbonic acid-derived groups such as benzyloxycarbonyl and ethoxycarbonyl. As the group suited for the etherification, there may be mentioned, for example, benzyl, tetrahydropyranyl and t-butyl.

Useful as the protective group for the phenolic hydroxyl group of tyrosine are, for example, Bzl, $Cl_2$-Bzl, 2-nitrobenzyl, Br-Z and t-butyl.

Useful as the protective group for the imidazole of histidine are, for example, Tos, 4-methoxy-2,3,6-trimethylbenzenesulfonyl, DNP, benzyloxymethyl, Bum, Boc, Trt and Fmoc.

The activated form of the carboxyl group of each starting material includes, among others, the corresponding acid anhydride, azide and activated esters [esters with an alcohol (e.g. pentachlorophenol, 2,4,5-trichlorophenol, 2,4-dinitrophenol, cyanomethylalcohol, paranitrophenol, HONB, N-hydroxysuccinimide, N-hydroxyphthalimide, HOBt)]. The activated form of the amino group of each starting material is, for example, the corresponding phosphoric amide.

As for the method for protective group removal (elimination), catalytic reduction in a hydrogen stream in the presence of a catalyst such as Pd black or Pd on carbon, treatment with an acid such as anhydrous hydrogen fluoride, methanesulfonic acid, trifluoromethanesulfonic acid, trifluoroacetic acid or a mixture thereof, treatment with a base such as diisopropylethylamine, triethylamine, piperidine or piperazine, or reduction with sodium in liquid ammonia may be employed, among others. The above acid treatment for elimination reaction is generally carried out at a temperature of about -20°C to 40°C and, in the acid treatment, the addition of a cation scavenger such as anisole, phenol, thioanisole, metacresol, paracresol, dimethyl sulfide, 1,4-butanedithiol or 1,2-ethanedithiol is effective. The 2,4-dinitrophenyl group used as a imidazole-protecting group for histidine can be eliminated by thiophenol treatment, and the formyl group used as an indole-protecting group for tryptophan can be eliminated by deprotecting acid treatment in the presence of the above-mentioned 1,2-ethanedithiol, 1,4-butanedithiol or the like as well as by treatment with an alkali such as dilute aqueous sodium hydroxide, dilute ammonia or the like.

The protection of a functional group which should not be involved in the reaction, the protective group to be used, the elimination of the protective group and the activation of the functional groups to be involved in the reaction, among others, can be selected from among publicity known groups or publicity known means.

An alternative method for obtaining the protein in an amide form comprises, for example, first protecting the α-carboxyl group of a carboxyl-terminal amino acid by amidation, then extending the peptide (protein) chain to a desired chain length on the amino group side, thereafter producing a protein derived from said peptide chain by eliminating only the protective group on the N-terminal α-amino group and a protein derived by eliminating only the protective group on the C-terminal carboxyl group, and subjecting the both proteins in a mixed solvent to condensation reaction, as mentioned above. The particulars of the condensation reaction are as mentioned above. The protected protein obtained by condensation is purified and then all the protective groups are eliminated by the methods mentioned above, whereby the desired crude protein can be obtained. This crude protein can be purified by making full use of various known purification means and the main fraction is lyophilized to give the desired protein in amide form.

For obtaining the protein in ester form, the α-carboxyl group of the carboxy-terminal amino acid, for example, is subjected to condensation with a desired alcohol to give an amino acid ester, for instance, which is followed by production of the desired ester-form protein in the same manner as the amide-form protein.

The partial peptide, or a salt thereof, of the present invention can be produced by any of publicity known methods of peptide synthesis or by cleaving the protein of the present invention with an appropriate peptidase. The method for peptide synthesis may be the solid phase one or liquid phase one. Thus, a partial peptide or amino acid capable of constituting the partial peptide of the present invention is condensed with the remaining portion of the partial peptide, followed by deprotection when the condensation product has protective group(s), whereby the desired peptide can be obtained. As the publicity known methods of condensation and the methods of protective group elimination, there may be mentioned, for example, those described in the following:

(1) M. Bodansky and M. A. Ondetti, Peptide Synthesis, Interscience Publishers, New York, 1966;
(2) Schroeder and Luebke, The Peptide, Academic Press, New York (1965) ;
(3) Nobuo Izumiya et al., Peputido Gosei no Kiso to Jikken (Peptide Synthesis, Fundamentals and Experiments), Maruzen (1975) ;
(4) Haruaki Yajima and Shumpei Sakakibara, Seikagaku Jikken Koza (Lectures in Biochemistry) 1, Tampakushitsu no Kagaku (Chemistry of Proteins) IV, 205 (1977);
(5) Haruaki Yajima (editor), Zoku Iyakuhin no Kaihatsu (Development of Drugs, 2nd series), vol. 14, Peptide Synthesis, Hirokawa Shoten.

After completion of all reactions, the partial peptide of the present invention can be purified and isolated, for example, by an appropriate combination of techniques selected from among solvent extraction, distillation, column chromatography, liquid chromatography, recrystallization and so forth. When the partial peptide obtained by the above methods is in a free form, it can be converted to an appropriate salt by a publicity known method or a modification thereof. When, conversely, it is obtained in a salt form, the salt can be converted to the free form or another salt by a publicity known method or a modification thereof.

The DNA coding for the protein of the present invention may be any one provided that it comprises the nucleotide sequence coding for the above-mentioned protein of the present invention . It may be a genomic DNA, a genomic DNA library, a cDNA derived from any of the above-mentioned cells or tissues, a cDNA library derived from any of the above-mentioned cells or tissues, or a synthetic DNA.

The vector to be used for library construction may be any one, for example a bacteriophage, plasmid, cosmid or phagimid. Direct amplification by reverse transcriptase polymerase chain reaction (hereinafter briefly referred to as RT-PCR) is also possible using a total RNA or mRNA fraction prepared from any of the cells or tissues mentioned above.

The DNA coding for the protein of the present invention is, for example, (1) a DNA containing the nucleotide sequence shown by SEQ ID NO:2, or a DNA having a nucleotide sequence hybridizable with the nucleotide sequence shown by SEQ ID NO:2 under highly stringent conditions and coding for a protein having substantially the same activity (e.g. cell proliferating activity) as that of the protein of the present invention , (2) a DNA containing the nucleotide sequence shown by SEQ ID NO:6, or a DNA having a nucleotide sequence hybridizable with the nucleotide sequence shown by SEQ ID NO:6 under highly stringent conditions and coding for a protein having substantially the same activity (e.g. cell proliferating activity) as that of the protein of the present invention , or any other like one.

More specifically, use is made, as the DNA coding for the protein having the amino acid sequence shown by SEQ ID NO:1, of a DNA having the nucleotide sequence shown by SEQ ID NO:2, for instance, and, as the DNA coding for the protein having the amino acid sequence shown by SEQ ID NO:5, of a DNA having the nucleotide sequence shown by SEQ ID NO:6, among others.

The DNA coding for the partial peptide of the present invention may be any one provided that it contains a nucleotide sequence coding for the above-mentioned partial peptide of the present invention . It may be a genomic DNA, a genomic DNA library, a cDNA derived from any of the above-mentioned cells or tissues, a cDNA library derived from any of the above-mentioned cells or tissues, or a synthetic DNA.

The DNA coding for the partial peptide of the present invention is, for example, (1) a DNA having a partial nucleotide sequence of the DNA having the nucleotide sequence shown by SEQ ID NO:2, or a DNA having a nucleotide sequence hybridizable with the nucleotide sequence shown by SEQ ID NO:2 under highly stringent conditions and having a part of the nucleotide sequence of a DNA coding for a protein having substantially the same activity as that of the protein of the present invention , (2) a DNA having a partial nucleotide sequence of the DNA having the nucleotide sequence shown by SEQ ID NO:6, or a DNA having a nucleotide sequence hybridizable with the nucleotide sequence shown by SEQ ID NO:6 under highly stringent conditions and having a part of the nucleotide sequence of a DNA coding for a protein having substantially the same activity as that of the protein of the present invention , or any other like one.

The means of cloning a DNA fully coding for the protein or partial peptide of the present invention (hereinafter, in explaining the cloning and expression of a DNA coding for the protein or the like, the protein or the like is sometimes referred to as "protein of the present invention " for short) may comprise effecting amplification by the publicity known PCR method using synthetic DNA primers having respective partial nucleotide sequences of the protein of the present invention , or by sorting out the DNA incorporated in an appropriate vector by hybridization with a labeled form of a DNA fragment or synthetic DNA coding for a part or the whole range of the protein of the present invention . The hybridization can be carried out, for example, according to the method described in Molecular Cloning, 2nd edition (J. Sambrook et al., Cold Spring Harbor Lab. Press, 1989). When a commercial library is used, the procedure described in the manual attached thereto can be followed.

The nucleotide sequence of a DNA can be modified, for example, by the gapped duplex method or Kunkel method or a like publicity known method or a modification thereof using a publicity known kit, for example Mutan™-G (Takara Shuzo) or Mutan™-K (Takara Shuzo).

The cloned, protein-encoding DNA can be used as such or, when desired, after restriction enzyme digestion or linker addition, for instance. Said DNA has ATG as a translation initiation codon on the 5'-terminal side and may have TAA, TGA or TAG as a translation termination codon on the 3'-terminal side. These translation initiation codon and/or translation termination codon can also be added using an appropriate synthetic DNA adapter.

The expression vector for the protein of the present invention can be produced, for example, by (a) excising the desired DNA fragment from a DNA coding for the protein of the present invention and (b) joining the DNA fragment into an appropriate expression vector at a site downstream from the promoter therein.

Useful as the vector are Escherichia coli-derived plasmids (e.g. pBR322, pBR325, pUC12, pUC13), Bacillus subtilis-derived plasmids (e.g. pUB110, pTP5, PC194), yeast-derived plasmids (e.g. pSH19, pSH15), bacteriophages such as λ phage, retroviruses, vaccinia viruses, baculoviruses and other animal viruses and, further, pAl-11, pXT1, pRc/CMV, pRc/RSV, pcDNAI/Neo and so on.

The promoter to be used in the practice of the present invention may be any promoter provided that it is suited for the host employed for gene expression, and includes, among others, the SRα promoter, SV40 early promoter, HIV LTR promoter, CMV promoter, and HSV-TK promoter for animal cell hosts.

Among these, the CMV (cytomegalovirus) promoter, SRα promoter and the like are preferably used. Where the host is an Escherichia, the trp promoter, lac promoter, recA promoter, λPL promoter, lpp promoter, T7 promoter and the like are preferred and, where the host is a Bacillus, the SPO1 promoter, SPO2 promoter, penP promoter and the like are preferred and, where the host is a yeast, the PHO5 promoter, PGK promoter, GAP promoter, ADH promoter and the like are preferred. Where the host is an insect cell, the polyhedrin promoter, P10 promoter and the like are preferred.

Also useful, as desired, as the expression vector in addition to the above are those containing an enhancer, splicing signal, poly(A) addition signal, selective marker, SV40 origin of replication (hereinafter sometimes referred to as SV40ori) or the like. As the selective marker, there may be mentioned, for example, the dihydrofolate reductase (hereinafter sometimes referred to as dhfr) gene [methotrexate (MTX) resistance gene], ampicillin resistance gene (hereinafter sometimes referred to as $Amp^r$), neomycin resistance gene (hereinafter sometimes referred to as $Neo^r$, G418 resistance) and the like. In particular when the dhfr gene is used as the selective marker by using dhfr gene-deficient Chinese hamster cells, the recombinant cells can also be selected by using a thymidine-free medium.

Further, where necessary, a signal sequence suited for the host is added to the N terminal side of the protein of the present invention . When the host is an Escherichia, the PhoA signal sequence, OmpA signal sequence or the like can be utilized. When the host is a Bacillus, the α-amylase signal sequence, subtilicin signal sequence or like sequence can be utilized, and when the host is a yeast, the MFα signal sequence, SUC2 signal sequence or like sequence can be used, and when the host is an animal cell, the insulin signal sequence, α-interferon signal sequence, antibody molecule signal sequence or like sequence can be utilized.

Using the thus-constructed vector containing a DNA coding for the protein of the present invention , a transformant can be produced.

Usable as the host are, for example, Escherichia, Bacillus, yeasts, insect cells, insects, animal cells and so forth.

As specific examples of the Escherichia, use may be made of Escherichia coli K12 DH1 [Proceedings of the National Academy of Sciences of the USA (Proc. Natl. Acad. Sci. USA), vol. 60, 160 (1968)], JM103 [Nucleic Acids Research, vol. 9, 309 (1981)], JA221 [Journal of Molecular Biology, vol. 120, 517 (1978)], HB101 [Journal of Molecular Biology, vol. 41, 459 (1969)], C600 [Genetics, vol. 39, 440 (1954)] and the like.

As examples of the Bacillus which are to be used, there may be mentioned Bacillus subtilis MI114 [Gene, vol. 24, 255 (1983)] and 207-21[Journal of Biochemistry, vol. 95, 87 (1984)], for instance.

As examples of the yeast, use may be made of Saccharomyces cerevisiae AH22, AH22R$^-$, NA87-11A, DKD-5D, 20B-12, Schizosaccharomyces pombe NCYC1913, NCYC2036, Pichia pastoris KM71 and the like.

As examples of the insect cells, use may be made of larval cabbage armyworm-derived established cell lines (Spodoptera frugiperda cells; Sf cells), MG1 cells derived from the mesenteron of Trichoplusia ni, High Five TM cells derived from the ovum of Trichoplusia ni, Mamestra brassicae-derived cells, Estigmenta acrea-derived cells and the like when the virus is AcNPV, and silkworm-derived established cell lines (Bombyx mori N cells; BmN cells) and the like when the virus is BmNPV. As the Sf cells, use may be made of Sf9 cells (ATCC CRL 1711) and Sf21 cells (for both, Vaughn, J. L. et al., In Vivo, 13, 213-217 (1977), among others.

Useful as the insect are silkworm larvae and the like [Maeda et al., Nature, vol. 315, 592 (1985)].

Useful as the animal cells are, for example, simian COS-7 cells, Vero, chinese hamster CHO cells (hereinafter briefly referred to as CHO cells), dhfr gene-deficient chinese hamster CHO cells (hereinafter briefly referred to as CHO (dhfr$^-$) cells), murine L cells, murine AtT-20, murine myeloma cells, rat GH3, and human FL cells. Further, various normal human cells, such as hepatocytes, splenocytes, nerve cells, glia cells, pancreatic β cells, bone marrow cells, mesangium cells, Langerhans' cells, epidermic cells, epithelial cells, endothelial cells, fibroblasts, fibrocytes, myocytes, fat cells, immunocytes (e.g. macrophages, T cells, B cells, natural killer cells, mast cells, neutrophils, basophils, eosinophils, monocytes), megakaryocytes, synovial cells, chondrocytes, osteocytes, osteoblasts, osteoclasts, mammary gland cells, hepatocytes and interstitial cells, precursor cells of these cells, stem cells, cancer cells, etc.) can also be used.

The transformation of Escherichia can be effected, for example, by the method described in Proc. Natl. Acad. Sci. USA, vol. 69, 2110 (1972) or Gene, vol. 17, 107 (1982), for instance.

The transformation of Bacillus can be effected, for example, by the method described in Molecular & General Genetics, vol. 168, 111 (1979), for instance.

The transformation of yeast can be effected, for example, by the method described in Methods in Enzymology, vol. 194, 182-187 (1991) or Proc. Natl. Acad. Sci. USA, vol. 75, 1929 (1978), for instance.

The transformation of insect cells or insects can be effected, for example, by the method described in Bio/Technology, 6, 47-55 (1988), for instance.

The transformation of animal cells can be effected, for example, by the method described in Saibo Kogaku Bessatsu (Cell Engineering, Extra Number) 8, Shin Saibo Kogaku Jikken Purotokoru (Protocols for Experiments in Cell Engineering, Revised), 263-267 (1995) (published by Shujunsha) or Virology, vol. 52, 456 (1973), for instance.

In this way, a transformant as transformed with an expression vector containing a protein-encoding DNA can be obtained.

In cultivating a host which is a transformant belonging to the genus Escherichia or Bacillus, a liquid medium is suited for use as the medium for the cultivation and the medium contains a carbon source(s), a nitrogen source(s), an inorganic substance(s) and so on. The carbon source includes, among others, glucose, dextrin, soluble starch and sucrose, the nitrogen source includes, among others, ammonium salts, nitrate salts, corn steep liquor, peptone, casein, meat extract, soybean cake, potato extracts and like inorganic and organic substances, and the inorganic substance include, among others, potassium chloride, sodium dihydrogen phosphate and magnesium chloride. An yeast extract, vitamins and/or growth promoting factors may also be added. The medium preferably has a pH of about 5 to 8.

Preferred as the medium for cultivating Escherichia is, for example, M9 medium (Miller, Journal of Experiments in Molecular Genetics, 431-433, Cold Spring Harbor Laboratory, New York, 1972) containing glucose and casamino acids. For efficient promoter functioning, such an agent as 3β-indolylacrylic acid, for instance, may be added thereto, when necessary.

Where the host is an Escherichia, the cultivation is carried out generally at about 15-43°C for about 3-24 hours. If necessary, aeration and/or agitation may be applied.

Where the host is a Bacillus, the cultivation is carried out generally at about 30-40°C for about 6-24 hours. If necessary, aeration and/or agitation may be applied.

In cultivating a host which is a yeast transformant, the medium is, for example, Burkholder's minimum medium [Bostian, K. L. et al., Proc. Natl. Acad. Sci. USA, vol. 77, 4505 (1980)] or SD medium [Bitter, G. A. et al., Proc. Natl. Acad. Sci. USA, vol. 81, 5330 (1984)] containing 0.5% casamino acids. The pH of the medium is preferably adjusted to about 5 to 8. The cultivation is carried out generally at about 20-35°C for about 24-72 hours. If necessary, aeration and/or agitation is applied.

Useful as the medium for cultivating a host which is an insect cell- or insect-derived transformant is, for example, Grace's insect medium (Grace, T. C. C., Nature, 195, 788 (1962)) supplemented with 10% inactivated bovine serum or a like additive. The pH of the medium is preferably adjusted to about 6.2 to 6.4. The cultivation is carried out generally at about 27°C for about 3-5 days. If necessary, aeration and/or agitation is applied.

Useful as the medium for cultivating a host which is an animal cell transformant are, for example, MEM medium [Science, vol. 122, 501 (1952)], DMEM medium [Virology, vol. 8, 396 (1959)], RPMI 1640 medium [The Journal of the American Medical Association, vol. 199, 519 (1967)], 199 medium [Proceedings of the Society for the Biological Medicine, vol. 73, 1 (1950), each supplemented with about 5-20% fetal calf serum. The pH is preferably about 6 to 8. The cultivation is carried out generally at about 30-40°C for about 15-60 hours. If necessary, aeration and/or agitation is applied.

In this manner, it is possible to cause the transformant to extracellularly produce the protein of the present invention

The protein of the present invention can be separated and purified from the cultivation mixture, for example, in the following manner.

An adequate method for extracting the protein of the present invention from cultured bacterial or other cells comprises, for example, harvesting the bacterial or other cells after cultivation by a publicity known method, then suspending them in an appropriate buffer, and disrupting them by sonication, lysozyme treatment and/or freezing-thawing, for instance, and recovering the protein-containing crude extract solution by centrifugation or filtration. The buffer may contain a protein denaturing agent such as urea or guanidine hydrochloride, or a surfactant such as Triton X-100™. Where the protein is secreted into the culture fluid, the cultivation mass after cultivation is separated into bacterial or other cells and the supernatant and the supernatant is collected.

The protein contained in the thus-obtained culture supernatant or extract can be purified by an appropriate combination of publicity known separation/purification methods. These known methods include techniques utilizing the difference in solubility, such as salting out and solvent precipitation, techniques principally utilizing the difference in molecular weight, such as dialysis, ultrafiltration, gel filtration and SDS-polyacrylamide gel electrophoresis, techniques utilizing the difference in charge, such as ion exchange chromatography, techniques utilizing the specific affinity, such as affinity chromatography, techniques utilizing the difference in hydrophobicity, such as reversed-phase high-performance liquid chromatography, and techniques utilizing the difference in isoelectric point, such as isoelectric focusing, among others.

In cases where the thus-obtained protein is in free form, it can be converted to a salt by a publicity known method or a modification thereof. When, conversely, it is obtained in the form of a salt, the salt may be converted to the free form or another salt by a publicity known method or a modification thereof.

It is also possible to arbitrarily modify, or delete a partial polypeptide from, the protein produced by the transformant by causing an appropriate protein-modifying enzyme to act thereon before or after purification thereof. Usable as the protein modifying enzyme are, for example, trypsin, chymotrypsin, arginine endopeptidase, protein kinase and glycosidase.

The presence or activity of the thus-formed protein, or a salt thereof, of the present invention can be determined, for example, by assaying for binding with a labeled ligand and enzyme immunoassay using a specific antibody.

The antibody against the protein or partial peptide, or a salt thereof, of the present invention may be a polyclonal or monoclonal antibody provided that it can recognize the protein or partial peptide, or a salt thereof, of the present invention

Preferred as the antibody against the partial peptide, or a salt thereof, of the present invention are, for example, antibodies to (1) a partial peptide, or a salt thereof, having an amino acid sequence containing the amino acid sequence covering the 22nd to 252nd, 26th to 252nd or 26th to 34th (preferably 26th to 252nd or 26th to 34th, more preferably 26th to 34th) amino acid residues in the amino acid sequence shown by SEQ ID NO:1 and (2) a partial peptide, or a salt thereof, having an amino acid sequence containing the amino acid sequence covering the 22nd to 246th, 26th to 246th or 166th to 190th (preferably 26th to 246th or 166th to 190th, more preferably 166th to 190th) amino acid residues in the amino acid sequence shown by SEQ ID NO:5.

The antibody against the protein or partial peptide, or a salt thereof, of the present invention (hereinafter, in explaining the antibody, the protein or the like are sometimes referred to merely as "protein of the present invention ") can be produced by a publicity known method for producing antibodies or antisera, using the protein of the present invention as an antigen.

[Monoclonal antibody production]

(a) Making of monoclonal antibody-producing cells

The protein of the present invention is administered, as such or together with a carrier or diluent, to a warm-blooded animal at a site capable of antibody production upon administration. On the occasion of administration, complete Freund's adjuvant or incomplete Freund's adjuvant may be administered for increasing the antibody production. Generally, the administration is performed once per 2 to 6 weeks and a total of about 2 to 10 times. The warm-blooded animal to be used includes, among others, monkeys, rabbits, dogs, guinea pigs, mice, rats, sheep, goats, chickens, among which mice and rats are preferred.

In making of monoclonal antibody-producing cells, an individual in which an antibody titer is recognizable is selected from among warm-blooded animals (e.g. mouse) immunized with the antigen and, 2 to 5 days after the final immunization, the spleen or lymph node is excised and the antibody-producing cells contained therein are fused with myeloma cells of a homozoic or heterozoic animal, whereby monoclonal antibody-producing hybridomas can be prepared. The antibody titer of an antiserum sample can be determined, for example, by reacting the labeled protein to be mentioned later herein with the antiserum and measuring the activity of the antibody-bound label. The procedure for fusion can be carried out following the method for Koehler and Milstein [Nature, 256, 495 (1975)], for instance. As the fusion promoter, there may be mentioned, for example, polyethylene glycol (PEG) and Sendai virus, among which PEG is preferred.

As the myeloma cells, there may be mentioned, for example, warm-blooded animal-derived myeloma cells such as NS-1, P3U1, SP2/0 and AP-1, among which P3U1 is preferred. The ratio between the number of antibody-producing cells (splenocytes) and the number of myeloma cells is preferably about 1:1 to 20:1 and PEG (preferably PEG 1000 to PEG 6000) is added to a concentration of about 10-80%, and the cell fusion can be efficiently effected by incubating at 20-40°C, preferably 30-37°C, for 1-10 minutes.

Various methods can be used in screening for monoclonal antibody-producing hybridomas. For example, there may be mentioned the method comprising adding the hybridoma culture supernatant to a solid phase (e.g. micro plate) with a protein antigen adsorbed thereon directly or together with a carrier, then adding a radiolabeled or enzyme-labeled anti-immunoglobulin antibody (anti-mouse immunoglobulin antibody when the cells used for cell fusion are murine cells) or Protein A and detecting the solid phase-bound monoclonal antibody, and the method comprising adding the hybridoma culture supernatant to a solid with an anti-immunoglobulin antibody or Protein A adsorbed thereon, adding a radiolabeled or enzyme-labeled protein and detecting the solid phase-bound monoclonal antibody.

Monoclonal antibody sorting can be carried out by a publicity known method or a modification thereof. Generally, it can be effected using a medium for animal cells supplemented with HAT (hypoxanthine, aminopterin and thymidine). The medium for sorting and breeding may be any one provided that the hybridoma can grow thereon. For example, PRMI 1640 medium supplemented with 1-20%, preferably 10-20%, fetal calf serum, GIT medium (Wako Pure Chemical Ind.) supplemented with 1-10% fetal calf serum, or serum-free medium for hybridoma cultivation (SFM-101, Nissui Pharmaceutical) and the like may be used. The cultivation temperature is generally 20-40°C, preferably about 37°C. The cultivation time is generally 5 days to 3 weeks, preferably 1 to 2 weeks. Generally, the cultivation can be carried out in the presence of 5% carbon dioxide. The antibody titer of the hybridoma culture supernatant can be determined in the same manner as mentioned above with reference to antiserum antibody titer determination.

(b) Monoclonal antibody purification

Separation and purification of monoclonal antibodies can be carried out by a publicity known method, for example by a method for separating and purifying immnoglobulins [e.g. salting out, alcohol precipitation, isolectric precipitation, electrophoresis, adsorption/desorption using an ion exchanger (e.g. DEAE), ultracentrifugation, gel filtration, specific purification method comprising collecting an antibody alone using an antigen-bound solid phase or an active adsorbent such as Protein A or protein G and recovering the antibody by dissociating the bond.]

[Polyclonal antibody production]

The polyclonal antibody of the present invention can be produced by a publicity known method or a modification thereof. For example, it can be produced by immunizing a warm-blooded animal with the immunogen (protein antigen) itself or a conjugate prepared from the same and a carrier protein in the same manner as mentioned above with reference to monoclonal antibody production, collecting the material containing an antibody against the protein of the present invention from the immunized animal and separating and purifying the antibody.

Referring to the immunogen-carrier protein conjugate to be used for immunizing warm-blooded animals, while the species of the carrier protein and the mixing ratio between the carrier and hapten are not critical provided that an antibody can be produced efficiently against the hapten crosslinked with the carrier and used for immunization, bovine serum albumin, bovine thyroglobulin, hemocyanin or the like is coupled to the hapten in a weight ratio of about 0.1-20: 1, preferably about 1-5:1.

For the coupling of the hapten to the carrier, various condensing agents can be used, for example glutaraldehyde, carbodiimides, active maleimide esters, and thiol- or dithiopyridyl-containing active ester reagents.

The coupling product is administered, as such or together with a carrier or diluent, to a warm-blooded animal at a site capable of antibody production upon administration. On the occasion of administration, complete Freund's adjuvant or incomplete Freund's adjuvant may be administered for increasing the antibody production. Generally, the administration is performed once per 2 to 6 weeks and a total of about 3 to 10 times.

The polyclonal antibody can be recovered from the blood, ascitic fluid and so forth, preferably the blood, of the warm-blooded animal immunized by the method mentioned above.

The polyclonal antibody titer of an antisera can be determined in the same manner as the antiserum antibody titer determination mentioned above. The polyclonal antibody can be separated and purified by the same method for immunoglobulin separation and purification as in the monoclonal antibody separation and purification mentioned above.

In the following, the uses of the protein or partial peptide, or a salt thereof, of the present invention (hereinafter sometimes referred to as "protein or the like of the present invention " for short) and of the antibody against the protein or partial peptide, or a salt thereof, of the present invention (hereinafter sometimes referred to as "antibody of the present invention ") are described.

(1) Therapeutic and/or prophylactic composition for various diseases which contain the protein or the like of the present invention

The protein or the like of the present invention has cell proliferating activity and therefore can be used as a medicinal such as a tissue regenerating agent after extraction of a diseased tissue (including total and partial excision but preferably partial excision).

In using the protein or the like of the present invention in the above treating or preventing composition, it is used preferably in a state purified up to at least 90%, preferably not less than 95%, more preferably not less than 98%, still more preferably not less than 99%.

The protein or the like of the present invention can be used orally in the form of tablets (if necessary sugar-coated), capsules, elixirs, microcapsules and so forth, or nonorally in the form of parenteral solutions such as aseptic solutions in water or some other pharmaceutically acceptable liquid, or suspensions. It can be prepared, for example, by admixing the protein or the like of the present invention with a physiologically acceptable carrier, flavoring agent, excipient, vehicle, preservative, stabilizer and/or binder, among others, to give unit dosage forms as required in the generally established pharmaceutical practice. The amount of the active ingredient in these pharmaceutical preparations should be sufficient to give an adequate dose within a range indicated.

The additives which can be formulated in tablets and capsules, among others, include but are not limited to binders such as gelatin, corn starch, tragacanth and gum arabic, excipients such as crystalline cellulose, swelling agents such as corn starch, gelatin and alginic acid, lubricants such as magnesium stearate, sweeteners such as sucrose, lactose and saccharin, and flavoring agents such as peppermint, gaultheria oil and cherry. When the unit preparation form is a capsule, the capsules may further contain a liquid carrier, such as an oil or fat, in addition to the above-mentioned types of material. Sterile compositions for injection can be formulated, for example, by dissolving or suspending the active constituent in a vehicle such as water for injection, or a natural vegetable oil, such as sesame oil or coconut oil, and so forth, according to the conventional pharmaceutical practice.

The aqueous solution for injection includes, for example, physiological saline, isotonic solutions containing glucose and/or some other auxiliary agent (e.g. D-sorbitol, D-mannitol, sodium chloride), and the like. These may be used in combination with an appropriate dissolution aid, such as an alcohol (e.g. ethanol), a polyalcohol (e.g. propylene glycol, polyethylene glycol), or a nonionic surfactant (e.g. polysorbate 80™, HCO-50). As the oleaginous liquid, there may be mentioned, for example, sesame oil, soybean oil and the like and these may be used in combination with benzyl benzoate, benzyl alcohol or the like as a dissolution aid. A buffer (e.g. phosphate buffer, sodium acetate buffer), an analgesic (e.g. benzalkonium chloride, procaine hydrochloride), a stabilizer (e.g. human serum albumin, polyethylene glycol), a preservative (e.g. benzyl alcohol, phenol) and/or an antioxidant may further be incorporated. The parenteral solutions prepared are generally filled into appropriate ampules.

The thus-obtained preparations are safe and low in toxicity and can be administered to humans or warm-blooded animals (e.g. rats, mice, guinea pigs, rabbits, birds, sheep, swine, cattle, horses, cats, dogs, monkeys), for instance.

The dose of the protein or the like of the present invention may vary according to the disease to be treated and the administration target or the like. Generally, however, the protein or the like of the present invention , when administered to human adults (weighing 60 kg) as a tissue regenerating agent after excision of a diseased tissue, for instance, is administered in a daily dose of about 0.1 mg to 100 mg, preferably about 1.0 to 50 mg, more preferably about 1.0 to 20 mg. (2) Screening for candidate medicinals for diseases

Natural killer (NK) cells inhibit the proliferation of cancer cells. The protein or the like of the present invention does not influence the proliferation of normal cells but inhibit the proliferation of NK cells and, therefore, a compound, or a salt thereof, having inhibitory activity against the NK cell proliferation inhibition by the protein or the like of the present invention can be used as a medicinal such as a treating or preventing agent for such diseases as various types of cancer (e.g. cancer of the uterine body, endometrioma, mammary cancer, carcinoma of the colon and rectum, prostatic cancer, lung cancer, renal cancer, neuroblastoma, bladder cancer, myeloma), for instance.

Therefore, the protein or the like of the present invention is useful as a reagent for screening for a compound, or a salt thereof, having inhibitory activity against the NK cell proliferation inhibition by the protein or the like of the present invention .

Thus, the present invention provides:(1) 1) A method for screening for a compound, or a salt thereof, (hereinafter, said compound inclusive of its salt is sometimes referred to as "inhibitor" with reference to "(2) Screening for candidate medicinals for disease") having inhibitory activity against the NK cell proliferation inhibition by the protein or partial peptide, or a salt thereof, of the present invention which method is characterized by using the protein or partial peptide, or a salt thereof, of the present invention (hereinafter, said method is sometimes referred to as "screening method for the present invention " with reference to "(2) Screening for candidate medicinals for diseases") and 2) a kit for screening for an inhibitor which is characterized by comprising the protein or partial peptide, or a salt thereof, of the present invention (hereinafter, said kit is sometimes referred to as "screening kit of the present invention " with reference to "(2) Screening for candidate medicinals for diseases") and, more specifically, it provides:

> (2) 1) A method for screening for an inhibitor which comprises comparing (i) the case in which NK cells are contacted with the protein or partial peptide, or a salt thereof, of the present invention and (ii) the case in which NK cells and a test compound are contacted with the protein or partial peptide, or a salt thereof, of the present invention and 2) a kit for screening for an inhibitor which is characterized by containing the protein or partial peptide, or a salt thereof, of the present invention and NK cells.

Specifically, the above screening method and screening kit are characterized, for example, by measuring the NK cell proliferation inhibiting activity of the protein or the like of the present invention in the cases (i) and (ii) and comparing the measurement data.

The NK cell proliferation inhibiting activity of the protein or the like of the present invention can be measured, for example, by a publicity known method or a modification thereof, preferably by the method described later in the example section.

The test compound includes, among others, peptides, proteins, nonpeptide compounds, synthetic compounds, fermentation products, cell extracts, plant extracts, animal tissue extracts and so forth. These may be novel compounds or known compounds.

For example, a test compound inhibiting the NK cell proliferation inhibiting activity in the above case (ii) by not less than about 20%, preferably not less than 30%, more preferably not less than 50%, as compared with the above case (i) can be selected as a compound inhibiting the NK cell proliferation inhibiting activity of the protein or the like of the present invention .

The compound, or a salt thereof, obtained by using the screening method or screening kit of the present invention is a compound selected from among such test compounds as mentioned above, for example peptides, proteins, nonpeptide compounds, synthetic compounds, fermentation products, cell extracts, plant extracts, animal tissue extracts and plasma and is a compound having inhibitory activity against the NK cell proliferation inhibiting activity of the protein or the like of the present invention .

The salt of said compound may be the same as mentioned above with reference to the salt of the protein or the like of the present invention .

In cases where the compound obtained by using the screening method or screening kit of the present invention is used as the treating or preventing agent mentioned above, it can be used according to the established practice. For example, tablets, capsules, elixirs, microcapsules, aseptic solutions, suspensions and other preparations can be produced in the same manner as mentioned above with reference to the pharmaceutical composition containing the protein or the like of the present invention .

The thus-obtained preparations are safe and low in toxicity and can be administered to humans or warm-blooded animals (e.g. mice, rats, rabbits, sheep, swine, cattle, horses, birds, cats, dogs, monkeys), for instance.

The dose of said compound or a salt thereof may vary according to its activity, the disease to be treated, the administration target, the route of administration or the like. Generally, however, the compound having inhibitory activity against the NK cell proliferation inhibition by the protein or the like of the present invention , when orally administered to ordinary adults (weighing 60 kg) for the treatment of endometrioma, for instance, is administered in a daily dose of about 0.1 mg to 100 mg, preferably about 1.0 to 50 mg, more preferably about 1.0 to 20 mg. In the case of nonoral administration, when the compound having inhibitory activity against the NK cell proliferation inhibition by the protein or the like of the present invention is administered in the form of a parenteral solution to adults (weighing 60 kg) for the treatment of endometrioma, for instance, said compound is administered in a daily dose of about 0.01 to 30 mg, preferably about 0.1 to 20 mg, more preferably about 0.1 to 10 mg, by intravenous injection, although the unit dose of the compound may vary according to the administration target, the disease to be treated, or the like. In other animals, a dose calculated on the 60 kg body weight basis can be administered.

(3) Screening for candidate medicinals for diseases

Since the protein or the like of the present invention has cell proliferating activity, a compound, or a salt thereof, capable of promoting the function (e.g. cell proliferating activity) of the protein or the like of the present invention can be used, for instance, as a medicinal such as a tissue regenerating agent after excision of a diseased tissue.

On the other hand, a compound, or a salt thereof, capable of inhibiting the function of the protein or the like of the present invention can be used, for example, as a medicinal such as a treating or preventing agent for diseases such as various types of cancer (e.g. cancer of the uterine body, endometrioma, mammary cancer, carcinoma of the colon and rectum, prostatic cancer, lung cancer, renal cancer, neuroblastoma, bladder cancer, myeloma).

Therefore, the protein or the like of the present invention is useful as a reagent for screening for a compound, or a salt thereof, promoting or inhibiting the function of the protein or the like of the present invention .

Thus, the present invention provides:

(1) 1) A method for screening for a compound, or a salt thereof, promoting the function (e.g. cell proliferating activity) of the protein or partial peptide, or a salt thereof, of the present invention (in "(3) Screening for a candidate medicinal for diseases", said compound or a salt thereof is sometimes referred to as "promoter" for short) or a compound, or a salt thereof, inhibiting the function (e.g. cell proliferating activity) of the protein or partial peptide, or a salt thereof, of the present invention (in "(3) Screening for a candidate medicinal for diseases", said compound or a salt thereof is sometimes referred to as "inhibitor" for short), which method is characterized by using the protein or partial peptide, or a salt thereof, of the present invention , and 2) a kit for screening for such promoter or inhibitor which is characterized by containing the protein or partial peptide, or a salt thereof, of the present invention (in "(3) Screening for a candidate medicinal for diseases", said kit is sometimes referred to as "screening kit of the present invention " for short) and, more specifically, it provides, among others,

(2) 1) A method for screening for a promoter or inhibitor which is characterized by comparing (i) the case in which cells (e.g. normal cells derived from any of the various tissues mentioned above (preferably a human tissue) or cancer cells derived from any of the above-mentioned types of cancer) are contacted with the protein or partial peptide, or a salt thereof, of the present invention and (ii) the case in which cells (e.g. normal cells derived from any of the various tissues mentioned above (preferably a human tissue) or cancer cells derived from any of the above-mentioned types of cancer) and a test compound are contacted with the protein or partial peptide, or a salt thereof, of the present invention and 2) a kit for screening for a promoter or inhibitor which is characterized by containing the protein or partial peptide, or a salt thereof, of the present invention and cells (e.g. normal cells derived from any of the various tissues mentioned above (preferably a human tissue) or cancer cells derived from any of the above-mentioned types of cancer).

To be concrete, it is a characteristic feature in the above screening method that the cell proliferating activity of the protein or the like of the present invention , for example, is measured in the cases (i) and (ii) and the data obtained are compared.

The cell proliferating activity of the protein or the like of the present invention can be measured by a publicity known method or a modification thereof.

Useful as the cells are, for example, normal cells derived from any of the various tissues mentioned above (preferably a human tissue) or cancer cells derived from any of the above-mentioned types of cancer.

The test compound includes, among others, peptides, proteins, nonpeptide compounds, synthetic compounds, fermentation products, cell extracts, plant extracts, animal tissue extracts and so forth. These may be novel compounds or known compounds.

For carrying out the above screening method, a sample of the protein or the like of the present invention is prepared by suspending the same in a buffer suited for the screening. The buffer may be any buffer that does not inhibit the reaction of the protein or the like of the present invention with the test compound; it may be phosphate buffer or Tris-hydrochloride buffer with a pH of about 4-10 (desirably, pH of about 6-8), for instance.

For example, a test compound promoting the cell proliferating activity in the above case (ii) by not less than about 20%, preferably not less than 30%, more preferably not less than 50%, as compared with the above case (i) can be selected as a compound promoting the cell proliferating activity of the protein or the like of the present invention and, on the other hand, a test compound inhibiting the cell proliferating activity in the above case (ii) by not less than about 20%, preferably not less than 30%, more preferably not less than 50%, as compared with the above case (i) can be selected as a compound inhibiting the cell proliferating activity of the protein or the like of the present invention .

The compound, or a salt thereof, obtained by using the screening method or screening kit of the present invention is a compound selected from among such test compounds as mentioned above, for example peptides, proteins, nonpeptide compounds, synthetic compounds, fermentation products, cell extracts, plant extracts, animal tissue extracts and plasma and is a compound promoting or inhibiting the function (e.g. cell proliferating activity) of the protein or the like of the present invention .

The salt of said compound may be the same as mentioned above with reference to the salt of the protein or the like of the present invention .

In cases where the compound obtained by using the screening method or screening kit of the present invention is used as the treating or preventing agent mentioned above, it can be used according to the established practice. For example, tablets, capsules, elixirs, microcapsules, aseptic solutions, suspensions and other preparations can be produced in the same manner as mentioned above with reference to the pharmaceutical composition containing the protein or the like of the present invention .

The thus-obtained preparations are safe and low in toxicity and can be administered to humans or warm-blooded animals (e.g. mice, rats, rabbits, sheep, swine, cattle, horses, birds, cats, dogs, monkeys), for instance.

The dose of said compound or a salt thereof may vary according to its activity, the disease to be treated, the administration target, the route of administration or the like. Generally, however, the compound promoting the function of the protein or the like of the present invention , when orally administered to human adults (weighing 60 kg) as a tissue regenerating agent after excision of a diseased tissue, for instance, is administered in a daily dose of about 0.1 mg to 100 mg, preferably about 1.0 to 50 mg, more preferably about 1.0 to 20 mg. In other animals, a dose calculated on the 60 kg basis can be administered.

On the other hand, when a compound inhibiting the function of the protein or the like of the present invention is orally administered for the treatment of endometrioma, it is administered to human adults (weighing 60 kg) in a daily dose of about 0.1 mg to 100 mg, preferably about 1.0 to 50 mg, more preferably about 1.0 to 20 mg. In the case of nonoral administration, when the compound inhibiting the function of the protein or the like of the present invention is administered in the form of a parenteral solution to ordinary adults (weighing 60 kg) for the treatment of endometrioma, for instance, said compound is administered in a daily dose of about 0.01 to 30 mg, preferably about 0.1 to 20 mg, more preferably about 0.1 to 10mg by intravenous injection, although the unit dose of said compound may vary according to the administration target, the disease to be treated or the like. In other animals, a dose calculated on the 60 kg basis can be administered.

(4) Quantifying of the protein or partial peptide, or a salt thereof, of the present invention

An antibody against the protein or the like of the present invention (hereinafter, sometimes referred to as "antibody of the present invention " for short) can specifically recognize the protein or the like of the present invention and therefore can be used in quantifying the protein or the like of the present invention in a test solution, in particular by sandwich immunoassay, for instance.

Thus, the present invention provides:

(i) A method for quantifying the protein or the like of the present invention in a test solution which comprises reacting the antibody of the present invention with the test solution and a labeled form of the protein or the like of the present invention competitively and determining the percentage of binding of the labeled form of the protein or the like of the present invention to said antibody and

(ii) A method for quantifying the protein or the like of the present invention in a test solution which comprises reacting the test solution with the antibody of the present invention as immobilized on a carrier and a labeled form of another antibody of the present invention simultaneously or successively and then measuring the activity of the label on the immobilizing carrier.

In the above quantifying method (ii), it is desirable that one of the antibodies be an antibody recognizing the N terminus of the protein or the like of the present invention and the other be an antibody reacting the C terminus of the protein or the like of the present invention .

Further, it is possible to quantify the protein or the like of the present invention using a monoclonal antibody against the protein or the like of the present invention (hereinafter sometimes referred to as "monoclonal antibody of the present invention ") and, in addition, to detect the same by tissue staining. For such purpose, the antibody molecule as such may be used or the $F(ab')_2$, Fab' or Fab fraction of the antibody molecule may be used.

The method for quantifying the protein or the like of the present invention using the antibody of the present invention is not particularly restricted but any assay technique may be used provided that the technique comprises detecting, by chemical or physical means, the amount of an antibody, antigen or antibody-antigen complex which corresponds to the amount of the antigen (e.g. protein) in the test solution, and converting the amount detected to the assay value using a standard curve constructed by using standard solutions each containing a known amount of the antigen. Suited are, for example, the nephelometric, competitive, immunometric and sandwich techniques. From the viewpoint of sensitivity and specificity, the use of the sandwich technique to be mentioned later herein is particularly preferred.

The label to be used in the assay techniques using a labeled substance is, for example, a radioactive isotope, enzyme, fluorescent substance, or luminescent substance. As the radioactive isotope, use is made, for example, of [$^{125}I$], [$^{131}I$], [$^{3}H$], [$^{14}C$] and so on. Preferred as the enzyme are those which are stable and have a high specific activity, such as, for example, β-galactosidase, β-glucosidase, alkaline phosphatase, peroxidase, malate dehydrogenase and the like. Useful as the fluorescent substance are, for example, fluorescamine and fluoresein isothiocyanate. Useful as the luminescent substance are luminol, luminol derivatives, luciferin and lucigenin, among others. Further, a biotin-avidin system can also be used for binding the antibody or antigen to the label.

For antigen or antibody insolubilization, physical adsorption may be utilized or a method generally utilizing chemical bonding for immobilizing and fixing a protein or enzyme may also be used. As the carrier, there may be mentioned insoluble polysaccharides such as agarose, dextran and cellulose, synthetic resins such as polystyrene, polyacrylamide and silicones, glass and so forth.

According to the sandwich technique, the protein of the present invention in a test solution can be quantitated by reacting the immobilized monoclonal antibody of the present invention with the test solution (primary reaction) and further with another labeled monoclonal antibody of the present invention (secondary reaction) and then measuring the activity of the label on the immobilizing carrier. The primary and secondary reactions may be carried out in a reversed order, or simultaneously, or at a time interval. The labeling agent and the method for immobilization may be selected from among those mentioned hereinabove. In the immunoassay by the sandwich technique the antibody for use as the solid phase antibody or labeling antibody need not always comprise one single species but a mixture of two or more antibodies may be used for improving the sensitivity of the measurement.

In assaying the protein or the like of the present invention by the sandwich technique according to the present invention , the monoclonal antibodies to be used in the primary reaction and secondary reaction preferably differ in the site of binding to the protein or the like of the present invention. Thus, regarding the antibodies to be used in the primary and secondary reactions, when the antibody employed for the secondary reaction recognizes the C terminal portion of the protein or the like of the present invention , the antibody to be used for the primary reaction is preferably one which recognizes other site than the C terminal portion, for example the N terminal portion.

The monoclonal antibody of the present invention can also be used in other assay systems than systems for the sandwich technique, for example in the competitive, immunometric or nephelometric method.

According to the competitive technique, the antigen in a test solution and a labeled antigen are competitively reacted with an antibody, then the unreacted labeled antigen (F) is separated from the antibody-bound labeled antigen (B) (B/F separation), and the label of B or F is assayed, to thereby determine the amount of the antigen in the test solution. The manner of this reaction includes the liquid phase technique which uses a soluble antibody as the antibody, the B/F separation is effected with polyethylene glycol, and uses a second antibody to the above antibody, among others, and the immobilization technique which uses an antibody immobilized on a solid phase as the first antibody or uses a soluble antibody as the first antibody and an immobilized antibody as the second antibody.

According to the immunometric technique, the antigen in a test solution and an immobilized antigen are competitively reacted with a given amount of a labeled antibody and then the solid phase and liquid phase are separated from each other, or the antigen in a test solution is reacted with an excess of a labeled antibody, then an immobilized antigen is added to thereby cause the unreacted labeled antibody to bind to the solid phase and, thereafter, the solid and liquid phases are separated from each other. Then, the label in one of the phases is assayed to determine the amount of the antigen in the test solution.

In nephelometry, the insoluble precipitate formed as a result of the antigen-antibody reaction within a gel or in a solution is quantitated. Even when the amount of the antigen in the test solution is slight and the precipitate is obtained only a small amount, laser nephelometry utilizing scattering of laser beams is judiciously used.

In applying these respective immunological assay methods to the quantifying method for the present invention , no particular conditions, procedure or like elements are required to be established. Assaying systems for the protein or the like of the present invention can be constructed giving technical considerations ordinary to those skilled in the art to the ordinary conditions and procedure. As regards the details of these general technical means, review articles and monographs, among others, can be referred to.

Reference may be made, for example, to Hiroshi Irie (ed.): "Radioimmunoassay" (publsihed 1974 by Kodansha); Hiroshi Irie (ed.): "Radioimmunoassay, Sequel" (publsihed 1979 by Kodansha); Eiji Ishikawa et al. (ed.): "Koso Men-eki Sokuteiho (Enzyme immunoassays)" (published 1978 by Igaku Shoin); Eiji Ishikawa et al. (ed.): "Koso Men-eki Sokuteiho (Enzyme immunoassays)" (2nd edition, published 1982 by Igaku Shoin); Eiji Ishikawa et al. (ed.): "Koso Men-eki Sokuteiho (Enzyme immunoassays)" (3rd edition, published 1987 by Igaku Shoin); "Methods in Enzymology", vol. 70 (Immunological Techniques (Part A)); ibid., vol. 73 (Immunochemical Techniques (Part B)); ibid., vol. 74 (Immunochemical Techniques (Part C)); ibid., vol. 84 (Immunochemical Techniques (Part D: Selected immunoassays); ibid., vol. 92 (Immunochemical Techniques (Part E: Monoclonal Antibodies and General Immunoassay Methods)); ibid., vol. 121 (Immunochemical Techniques (Part I: Hybridoma Technology and Monoclonal Antibodies)) (all above published by Academic Press); and so on.

In the above manner, the protein or the like of the present invention can be quantified with good sensitivity by using the antibody of the present invention .

Further, when an increase in the concentration of the protein or the like of the present invention is detected upon determining the concentration of the protein or the like of the present invention using the antibody of the present invention , it can be diagnosed that there is a disease such as, for example, various types of cancer (e.g. cancer of the uterine body, endometrioma, mammary cancer, carcinoma of the colon and rectum, prostatic cancer, lung cancer, renal cancer, neuroblastoma, bladder cancer, myeloma) or it is highly possible for the subject tested to suffer from such a disease in the future.

The antibody of the present invention can also be used in detecting the protein or the like of the present invention occurring in a test sample such as a body fluid or tissue. It can further be used in making an antibody column for use in purifying the protein or the like of the present invention , detecting the protein or the like of the present invention in each fraction in the step of purification thereof, and analyzing the behavior of the protein or the like of the present invention in cells tested, among others.

(5) Pharmaceutical compositions containing the antibody of the present invention

The antibody of the present invention which can neutralizes the activity of the protein or the like of the present invention (neutralizing antibody) can be used as a medicinal such as a treating or preventing agent for diseases such as, for example, various types of cancer (e.g. cancer of the uterine body, endometrioma, mammary cancer, carcinoma of the colon and rectum, prostatic cancer, lung cancer, renal cancer, neuroblastoma, bladder cancer, myeloma).

The humanized antibody against the protein or the like of the present invention can be used as a medicinal such as a treating or preventing agent for diseases such as, for example, various types of cancer (e.g. cancer of the uterine body, endometrioma, mammary cancer, carcinoma of the colon and rectum, prostatic cancer, lung cancer, renal cancer, neuroblastoma, bladder cancer, myeloma).

Said humanized antibody can be produced according to the method described in Nat. Biotechnol., 14, 845-851 (1996); Nat. Genet., 15, 146-156 (1997); or PNAS, 97 (2), 722-727 (2000), for instance.

Hereinafter, the neutralizing antibody and humanized antibody of the present invention are collectively referred to as "antibody of the present invention " with reference to "(5) Pharmaceutical composition containing the antibody of the present invention ".

The treating or preventing agent for the diseases mentioned above which comprises the antibody of the present invention can be administered, either as such in the form of a liquid preparation or in the form of an appropriate pharmaceutical composition, orally or nonorally to humans or mammals (e.g. rats, rabbits, sheep, swine, cattle, cats, dogs, monkeys). While the dose may vary according to the target of administration, target disease, symptom, route of administration or the like, the antibody of the present invention , when used for the treatment and/or prevention of endometrioma in human adults, is administered generally in a unit dose of about 0.01-20 mg/kg body weight, preferably about 0.1-10 mg/kg body weight, more preferably about 0.1-5 mg/kg body weight, about 1 to 5 times a day, preferably about 1 to 3 times a day, favorably by intravenous injection. In the case of other nonoral administration and oral administration, the corresponding dose can be administered. When the symptom is particularly severe, the dose may be increased according to the symptom.

The antibody of the present invention can be administered either as such or in the form of an appropriate pharmaceutical composition. The pharmaceutical composition used for the above administration comprises the above mentioned it or a salt thereof and a pharmacologically acceptable carrier, diluent or excipient. Such composition is provided in a dosage form suited for oral or nonoral administration.

Thus, for example, the composition for oral administration includes solid or liquid dosage forms, specifically tablets (inclusive of sugar-coated tablets and film-coated tablets), pills, granules, powders, capsules (inclusive of soft capsules), syrups, emulsions, suspensions and so on. Such compositions can be produced by publicity known methods and contain a carrier, diluent or excipient commonly used in the field of pharmaceutics. For example, lactose, starch, sucrose, magnesium stearate and the like are used as the carrier or excipient for tablets.

The composition for nonoral administration is, for example, a parenteral solution or a suppository. The parenteral solution includes such dosage forms as solutions for intravenous injection, subcutaneous injection, intradermal injection, intramuscular injection, and drip injection. Such parenteral solutions are prepared according to a publicity known method, for example by dissolving, suspending or emulsifying the above antibody or a salt thereof in a sterile aqueous or oleaginous vehicle which is commonly used for the preparation of parenteral products. Physiological saline or isotonic solutions containing glucose and other auxiliary agents, for instance, are used as the aqueous vehicle for injection, and they may be used in combination with an appropriate dissolution aid, such as an alcohol (e.g. ethanol), a polyalcohol (e.g. propylene glycol, polyethylene glycol) or a nonionic surfactant [e.g. polysorbate 80, HCO-50 (polyoxyethylene(50 mol) adduct of hydrogenated castor oil]. Useful as the oleaginous vehicle are, for example, sesame oil and soybean oil. These may be used in combination with benzyl benzoate, benzyl alcohol or the like as a dissolution aid. Generally, the parenteral solution prepared is filled into appropriate ampules. The suppository for rectal administration is prepared by admixing the above antibody or a salt thereof with an ordinary suppository base.

The above pharmaceutical composition for oral or nonoral administration is favorably prepared in the form of a unit dosage form conforming to the dose of the active ingredient. As examples of such unit dosage form, there may be mentioned tablets, pills, capsules, parenteral solutions (ampules) and suppositories, among others. Each unit dosage form generally contains 5-500 mg of the active ingredient and, in the case of parenteral solutions, in particular, each unit dosage form preferably contains 5-100 mg and, in the case of other dosage forms, 10-250 mg of the above antibody.

Each of the compositions mentioned above may contain some other active ingredient provided that the incorporation thereof does not cause any unfavorable interaction with the above antibody.

In the present specification and drawings, the abbreviations used to express the bases or amino acids or the like are based on the IUPAC-IUB Commission on Biochemical Nomenclaure or are according to the conventional abbreviations used in the relevant field of art. The following are examples thereof. Where there can be optical isomerism with regard to amino acids, it is to be construed that the L form is meant, unless otherwise specified.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| I | : inosine |
| R | : adenine (A) or guanine (G) |
| Y | : thymine (T) or cytosine (C) |
| M | : adenine (A) or cytosine (C) |
| K | : guanine (G) or thymine (T) |
| S | : guanine (G) or cytosine (C) |
| W | : adenine (A) or thymine (T) |
| B | : guanine (G), guanine (G) or thymine (T) |
| D | : adenine (A), guanine (G) or thymine (T) |
| V | : adenine (A), guanine (G) or cytosine (C) |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguannosine triphosphate |
| dCTP | : deoxycytidine triphosphate |
| ATP | : adenosine triphosphate |
| Gly | : glycine |
| Ala | : alanine |
| Val | : valine |
| Leu | : leucine |
| Ile | : isoleuicine |
| Ser | : serine |
| Thr | : threonine |

Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid

In the present specification, those substituents, protective groups and reagents which appear frequently are indicated by the symbols given below.

Me : methyl group
Et : ethyl group
Bu : butyl group
Ph : phenyl group
TC : thiazolidine-4(R)-carboxamido group
Tos : p-toluenesulfonyl
CHO : formyl
Bzl : benzyl
$Cl_2$-Bzl : 2,6-dichlorobenzyl
MBzl : methoxybenzyl
MeBzl : 4-methylbenzyl
OcHex : cyclohexyl ester
OBzl : benzyl ester
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenyl
Trt : trityl
Bum : t-butoxymethyl
Fmoc : N-9-fluorenylmethoxycarbonyl
HOBt : 1-hydroxybenzotriazol
HOOBt : 3,4-dihydro-3-hydroxy-4-oxo-1,2,3-benzotriazine
HONB : 1-hydroxy-5-norbornene-2,3-dicarboxiimide
DCC : N,N'-dicyclohexylcarbodiimide
DMF : N,N-dimethylformamide
TEA : triethylamine
WSCD : 1-ethyl-3-(3-dimethylaminopropyl)carbodiimide
EDTA : ethylenediaminetetraacetic acid
SDS : sodium dodecyl sulfate

In the sequence listing in the specification of the instant application, the sequence identifier numbers respectively show the following:

SEQ ID NO:1 shows the amino acid sequence of a human-derived protein (TGC839 protein) according to the present invention.
SEQ ID NO:2 shows the nucleotide sequence of a DNA coding for the human-derived protein according to the present invention which has the amino acid sequence shown by SEQ ID NO:1.
SEQ ID NO:3 shows the nucleotide sequence of a primer (synthetic) DNA used in Example 3.

SEQ ID NO:4 shows the nucleotide sequence of a primer (synthetic) DNA used in Example 3.
SEQ ID NO:5 shows the amino acid sequence of a human-derived protein (TGC838 protein) according to the present invention.
SEQ ID NO:6 shows the nucleotide sequence of a DNA coding for the human-derived protein according to the present invention which has the amino acid sequence shown by SEQ ID NO:5.
SEQ ID NO:7 shows the nucleotide sequence of a primer (synthetic) DNA used in Example 7 and Example 20.
SEQ ID NO:8 shows the nucleotide sequence of a primer (synthetic) DNA used in Example 7.
SEQ ID NO:9 shows the nucleotide sequence of a primer (synthetic) DNA used in Example 20.

The transformant Escherichia coli SURE/pCAN618/H839F obtained in Example 3 as described later herein has been deposited with the National Institute of Bioscience and Human Technology (NIBH), the Ministry of International Trade and Industry, 1-3 Higashi 1-chome, Tsukuba, Ibaraki, Japan, as of March 10, 1999 under the accession number FERM BP-6677 and with the Institute for Fermentation Osaka (IFO), 17-85 Jusohonmachi 2-chome, Yodogawa-ku, Osaka, Osaka, Japan, as of February 25, 1999 under the accession number IFO 16259.
The transformant Escherichia coli XL10-Gold/pCAN618/H838F obtained in Example 7 as described later herein has been deposited with the National Institute of Bioscience and Human Technology (NIBH), the Ministry of International Trade and Industry, as of August 2, 1999 under the accession number FERM BP-6809 and with the Institute for Fermentation Osaka (IFO) as of July 21, 1999 under the accession number IFO 16297.
The hybridoma clone No. 112-3 obtained in Example 15 as mentioned later herein has been deposited as 839N-112 with the National Institute of Bioscience and Human Technology (NIBH), the Ministry of International Trade and Industry, as of March 2, 2000 under the accession number FERM BP-7068.
The hybridoma clone No: 128-18 obtained in Example 15 as mentioned later herein has been deposited as 839N-128 with the National Institute of Bioscience and Human Technology (NIBH), the Ministry of International Trade and Industry, as of March 2, 2000 under the accession number FERM BP-7069.
The CHO-K1/TGC838N-4 obtained in Example 22 as mentioned later herein has been deposited with the National Institute of Bioscience and Human Technology (NIBH), the Ministry of International Trade and Industry, as of March 10, 2000 under the accession number FERM BP-7088.
The CHO-K1/618/839F6-3 obtained in Example 23 as mentioned later herein has been deposited with the National Institute of Bioscience and Human Technology (NIBH), the Ministry of International Trade and Industry, as of March 10, 2000 under the accession number FERM BP-7089.
The hybridoma 839-01 obtained in Example 28 as mentioned later herein has been deposited with the National Institute of Bioscience and Human Technology (NIBH), the Ministry of International Trade and Industry, as of March 10, 2000 under the accession number FERM BP-7086.
The hybridoma 839-02 obtained in Example 28 as mentioned later herein has been deposited with the National Institute of Bioscience and Human Technology (NIBH), the Ministry of International Trade and Industry, as of March 10, 2000 under the accession number FERM BP-7087.
The following examples illustrate the present invention more specifically. They are, however, by no means limitative of the scope of the present invention . The methods for gene manipulation using Escherichia coli are those described in Molecular Cloning.

Example 1 Clone selection from a data base

A clone having an amino acid sequence characteristic of a secretory sequence of proteins was selected from the data base supplied by SmithKline Beecham (SB).
Thus, after translation of the nucleotide sequence of each EST into the amino acid sequence, EST clones were selected in which Met occurred at the 5' terminus and a hydrophobic amino acid sequence was found on the 5'-terminal side and, further, hydrophilic and hydrophobic amino acid sequences were present downstream therefrom and the 10th-30th amino acid residues from the first residue Met constituted a signal sequence necessary for protein secretion. One of them, HGS:2772893, was thus found. This clone was ordered from SB and named TGC839. An E. coli strain transformed with a plasmid containing said gene was cultivated in the conventional manner, the plasmid was purified and said gene was excised with the restriction enzymes EcoRI and XhoI, whereby two gene fragments, about 0.9 kb and about 0.5 kb in size, were found. It was thus revealed that the size of this gene is about 1.4 kb.
Then, the nucleotide sequence of this gene was analyzed with an ABI 377 Prism fluorescence DNA sequencer using the primers T7 and T3. The nucleotide sequence and amino acid sequence as found are shown in Fig. 1.
This gene codes for a protein having 252 amino acid residues and it was estimated that signal sequence cleavage occur between the 25th (from the N terminus) amino acid residue (Ala) and the 26th amino acid residue (Gly).

Example 2 Confirmation of gene expression

For estimating the function of the gene obtained in Example 1, an investigation was made concerning its expression in various human tissues and in cancer cell lines.

First, using, as a probe, a DNA fragment about 900 bases in size which are obtainable by cleavage of the gene with EcoRI and XhoI, northern blot analysis was performed of mRNA samples which were extracted from the respective tissues and cells. Nylon filters blotted with Clontech's human multi tissue northern (MTN) blot or human cancer cell line mRNA blot or with mRNA of various human cancer cell lines were reacted with the isotope-labeled DNA probe in the routine manner and, after washing the filters, autoradiographs were taken and examined for tissues or cells showing expression of this gene.

The expression of this gene was hardly recognized in normal human tissues. Specific expression thereof was observed only in cancer cells and fetal brain and lung (Fig. 2, Fig. 3).

Example 3 Construction of an expression vector for the expression of the human TGC839 protein in animal cells

An expression vector was constructed for the expression of the human TGC839 protein in animal cells. On that occasion, for facilitating the later detection of the expression product, a FLAG sequence (DYKDDDDK) composed of 8 amino acid residues was introduced into the C-terminal side of the TGC839 protein.

Hereinafter, the TGC839 protein having the FLAG sequence added is sometimes referred to specifically as "TGC839FLAG protein".

First, using a synthetic DNA designed for an EcoRI restriction site to appear immediately upstream of the translation initiation codon ATG [5' GCGCTCGAATTCCACCATGGCAGCAGCCGCCGCTACCAAG 3': SEQ ID NO:3)] and a synthetic DNA designed for an amino acid sequence represented by DYKDDDDK to appear at the C terminus of the TGC839 protein and be followed by the termination codon and further by a NotI restriction site [5' GCGGCCGCTCACTTGTCATCGTCGTCCTTGTAGTCCTC TAAAGGACTCTCCTCAGATGCCAGGGAGGATGAAGCAG 3': (SEQ ID NO:4)], with the TGC839 protein-encoding cDNA fragment used as a template, the PCR was carried out, and a DNA fragment containing the ORF of TGC839 wwhich are obtainable. This DNA fragment was subjected to double digestion with the restriction enzymes EcoRI (Takara Shuzo and EagI (New England Biolabs), followed by introduction into pCAN618 at the EcoRI-NotI site thereof, whereby an expression vector, pCAN618/H839F, for expression of TGC839FLAG, the human TGC839 protein with the FLAG sequence on the C-terminal side, in animal cells wwhich are obtainable. The Escherichia coli SURE strain was transformed with said pCAN618/H839F by a publicity known method to give Escherichia coli SURE/pCAN618/H839F.

Example 4 Secretory expression of the human TGC839 protein into the COS-7 culture supernatant

For confirming the fact that the human TGC839 protein is a secretory protein, expression of the TGC839FLAG protein was caused using COS-7 cells and whether it was secreted into the medium or not was checked. On the day before transfection with the expression vector constructed in Example 3, COS-7 cells were sowed to a density of $4 \times 10^5$ cells/well and cultured in DMEM medium (Gibco-BRL) supplemented with 10% FBS (Hyclone) in a $CO_2$ incubator for 24 hours. At 24 hours after transfection using the pCAN618/H839F DNA and Effectene (Qiagen), the medium was exchanged for Opti-MEM medium (Gibco-BRL) (1 ml) supplemented with 0.1 mM ABSF (4-(2-aminoethyl)benzenesulfonyl fluoride (hydrochloride)) (Wako Pure Chemical) and 0.05% CHAPS (3-[3-cholamidopropyl)dimethylammonio] propanesulfonic acid) (Dojin Kagaku) and incubation was further continued for 36 hours. The culture supernatant was transferred to an Eppendorf sample tube and centrifuged and, after removal of floating cells, concentrated to 1/10 using Centricon 10 (Amicon), SDS-PAGE sample buffer containing an equal amount of DTT was then added, and the mixture was subjected to SDS-PAGE. Western blotting was carried out using anti-FLAG mouse IgG monoclonal antibody (M12; 5 mg/ml, Sigma) as the primary antibody and HRP (horseradish peroxidase)-labeled anti-mouse IgG antibody (1/2000, Amersham)as the secondary antibody.

As a result, the TGC839FLAG protein was detected as a single band of about 34 kDa intracellularly and, in the culture supernatant, as such and a band broader than that found intracellularly (Fig. 4). In view of this, it was estimated that the TGC839 protein is secreted into the medium with a sugar chain added at each of the two N type glycosylation sites occurring in the protein.

Example 5 Clone selection from a data base

A clone having an amino acid sequence characteristic of a secretory sequence of proteins was selected from the data base supplied by SmithKline Beecham (SB).

Thus, after translation of the nucleotide sequence of each EST into the amino acid sequence, EST clones were selected in which Met occurred at the 5' terminus and a hydrophobic amino acid sequence was found on the 5'-terminal side and, further, a hydrophilic and hydrophobic amino acid sequence was present downstream therefrom and the 10th-30th amino acid residues from the first residue Met constituted a signal sequence necessary for protein secretion. One of them, HGS:951123, was thus found. This clone was ordered from SB and named TGC838. An E. coli strain transformed with a plasmid containing said gene was cultivated in the conventional manner, the plasmid was purified and said gene was excised with the restriction enzymes EcoRI and XhoI, whereby two gene fragments, about 1.0 kb and about 0.4 kb in size, were found. It was thus revealed that the size of this gene is about 1.4 kb.

Then, the nucleotide sequence of this gene was analyzed with an ABI 377 Prism fluorescence DNA sequencer using the primers T7 and T3. The nucleotide sequence and amino acid sequence as found are shown in Fig. 5.

This gene codes for a protein having 246 amino acid residues and it was estimated that signal sequence cleavage occur between the 25th (from the N terminus) amino acid residue (Ala) and the 26th amino acid residue (Gly).

Example 6 Confirmation of gene expression

For estimating the function of the gene obtained in Example 5, an investigation was made concerning its expression in various human tissues and in cancer cell lines.

First, using, as a probe, a DNA fragment about 1.0 kb in size which are obtainable by cleavage of the gene with EcoRI and XhoI, northern blot analysis was performed of mRNA samples which were extracted from the respective tissues and cells. Nylon filters blotted with Clontech's human multi tissue northern (MTN) blot or human cancer cell line mRNA blot or with mRNAs of various human cancer cell lines were reacted with the isotope-labeled DNA probe in the routine manner and, after washing the filters, autoradiographs were taken and examined for tissues or cells showing expression of this gene.

The expression of this gene was hardly recognized in normal human tissues. Specific expression thereof was observed only in cancer cells and fetal brain and lung (Fig. 6).

Example 7 Construction of an expression vector for the expression of the human TGC838 protein in animal cells

An expression vector was constructed for the expression of the human TGC838 protein in animal cells. On that occasion, for facilitating the later detection of the expression product, a FLAG sequence (DYKDDDDK) composed of 8 amino acid residues was introduced into the C-terminal side of the TGC838 protein.

Hereinafter, the TGC838 protein having the FLAG sequence added is sometimes referred to specifically as "TGC838FLAG protein".

First, using a synthetic DNA designed for an EcoRI restriction site to appear immediately upstream of the translation initiation codon ATG [5' GCGCTGAATTCCCACCATGGCAGCAGCCGCCGCTACCAAGATCCTTCTGTGCCTCCC GCTTCT 3': (SEQ ID NO:7)] and a synthetic DNA designed for an amino acid sequence represented by DYKDDDDK to appear at the C terminus of the TGC838 protein and be followed by the termination codon and further by a NotI restriction site [5' TTGCGGCCGCTCACTTGTCATCGTCG TCCTTGTAGTCGATGCCAGGGAGGATGAAGCAGGGGAGGATGATG 3': (SEQ ID NO:8)], with the TGC838 protein-encoding cDNA fragment used as a template, the PCR was carried out, and a DNA fragment containing the ORF of TGC838 wwhich are obtainable. This DNA fragment was subjected to double digestion with the restriction enzymes EcoRI and NotI, followed by introduction into pCAN618 at the EcoRI-NotI site thereof, whereby an expression vector, pCAN618/H838F, for expression of TGC838FLAG, the human TGC838 protein with the FLAG sequence on the C-terminal side, in animal cells wwhich are obtainable.

The plasmid pCAN618/H838F of the present invention was introduced into the Escherichia coli strain XL10-Gold, whereby a transformant, Escherichia coli XL10-Gold/pCAN618/H838F, wwhich are obtainable.

Example 8 Purification of the human TG839 protein from the COS-7 cell culture supernatant

Since, in Example 4, the TGC839 protein proved to be secreted into the COS-7 cell culture supernatant, the human TGC839FLAG protein was purified from the COS-7 cell culture supernatant. On the day before transfection with the expression vector constructed in Example 3, ten 10-cm dishes were sown with COS-7 cells to a density of 2 $\times 10^6$ cells/plate and cultured in DMEM medium (Gibco-BRL) supplemented with 10% FBS (Hyclone) in a $CO_2$ incubator for 24 hours. At 24 hours after transfection using the pCAN618/H839F DNA and Effectene (Qiagen), the medium was exchanged for Opti-MEM medium (Gibco-BRL) (4 ml) supplemented with 0.1 mM ABSF (Wako Pure Chemical) and 0.05% CHAPS (Dojin Kagaku) and incubation was continued further for 36 hours. The culture supernatant collected from the ten 10-cm dishes was transferred to a 50-ml centrifuge tube and centrifuged to thereby remove floating cells, and filtered through a 0.2-μm ultrafiltration membrane. To the filtrate was added 20-fold concentrated TBS to a final concentration of one-fold and, after two repetitions of adsorption on ANTI FLAG M2-agarose affinity gel (Sigma), the desired protein was eluted with glycine-HCl buffer (pH 3.5). The eluate was subjected to buffer exchange for PBS using a desalting column (PD-10, AmershamPharmacia) and then concentrated with Centriplus-10, Centricon-10 (Amicon) to about 60 µl to give a purified sample. This purified sample was subjected to SDS-PAGE, followed by staining with CBB, whereupon a single broad band of about 45 kDa was observed.

Example 9 N-Terminal amino acid sequence determination of the purified human TGC839FLAG protein

A 2-µl portion of the purified sample obtained in Example 8 was applied to a gaseous phase amino acid sequencer, LF3000 protein sequencer (Beckman-Coulter) for N-terminal amino acid sequence determination. As a result, the amino acid residues 1. glycine (5.10 pmol), 2. arginine (6.53 pmol), 3. alanine (3.97 pmol) and 4. asparagine (6.92 pmol) were detected in that order from the N terminal.

Based on the above findings, it was found that the TGC839 protein is secreted as the human mature TGC839 protein beginning with the 26th amino acid (glycine) residue into the medium after cleavage of the signal sequence composed of 25 N-terminal amino acid residues of the human TGC839 precursor protein.

Example 10 Production of Gly-Arg-Ala-Asp-Pro-His-Ser-Leu-Cys: TGC-839 peptide (26-34)

The Boc group of 2.27 g of the starting material Boc-Cys(MBzl)-OBzl was eliminated with 4 N HCl·ethyl acetate, the HCl salt was neutralized with an equimolar amount of TEA and condensed with Boc-Leu·$H_2O$ in the presence of HONB/WSCD·HCl. The reaction mixture was extracted with ethyl acetate, the extract was washed with 0.1 N HCl and 5% aqueous solution of sodium bicarbonate and dried over anhydrous sodium sulfate and the solvent was then removed. The residue was crystallized from ethyl acetate and hexane and the crystals were collected by filtration. 2.3 g (80.3%). Using this Boc-Leu-Cys(MBzl)-OBzl, condensation was effected with Boc-Ser(Bzl) and with Boc-His(Bom) to give 1.23 g of Boc-His(Bom)-Ser(Bzl)-Leu-Cys(MBzl)-OBzl: (I). Starting with Pro-OBzl, Boc-Asp(OcHex)-Pro-OBzl was synthesized and this was catalytically reduced for elimination of the benzyl ester, to give oily Boc-Asp(OcHex)-Pro-OH: (II). Starting with Ala-OBzl, this was condensed with Boc-Arg(Tos) and Boc-Gly to give Boc-Gly-Arg(Tos)-Ala-OBzl, which was subjected to catalytic reduction. The resulting Boc-Gly-Arg(Tos)-Ala-OH: (III) was recovered as a powder from acetonitrile and diethyl ether by filtration. The Boc group of 0.407 g of (I) was eliminated by treatment with 4 N HCl·ethyl acetate and then the resultant was dissolved in DMF, neutralized with 62 µl of TEA and then condensed with 0.23 g of (II) using 225 mg of HOBt and 160 mg of WSCD·HCl. The reaction mixture was extracted with ethyl acetate, the extract was washed with 5% aqueous solution of sodium bicarbonate and dried over anhydrous sodium sulfate and the solvent was then removed. The residue was recovered as a powder from acetinitrile and diethyl ether by filtration, to give 338 mg (63.8%) of Boc-Asp(OcHex)-Pro-His(Bom)-Ser(Bzl)-Leu-Cys(MBzl)-OBzl: (IV). The Boc group of 187 mg of (IV) was eliminated by treatment with 4 N HCl·ethyl acetate and the resultant (IV) was dissolved in DMF, neutralized with 22 µl of TEA and then condensed with 90 g of (III) using 79 mg of HOBt and 56 mg of WSCD·HCl. The solvent was removed and 228 mg of gel-like Boc-Gly-Arg(Tos)-Ala-Asp(OcHex)-Pro-His(Bom)-Ser(Bzl) - Leu-Cys (MBzl)OBzl was recovered from ethyl acetate. A 117.8-mg portion of this was treated with 10 ml of anhydrous hydrogen fluoride in the presence of 974 mg of p-cresol at 0°C for 60 minutes to thereby eliminate all protective groups. The peptide fraction was fractionated on a Sephadex™ G-25 column (2.0 × 80 cm) packed using 50% acetic acid-water. The main fraction was further applied to a reversed-phase chromatography column (2.6 × 8.0 cm) packed with LiChroprep™ RP-18 and the main fraction was lyophilized to give 30 mg of a white powder. Mass spectrometric analysis $(M + H)^+$ :955.4 (calculated value 955.4)
HPLC elution time: 10.5 minutes
Column conditions:
Column: Wakosil 5C18T, 4.6 × 100 mm
Eluent: using solution A - 0.1% TFA-water and solution B - acetonitrile containing 0.1% TFA, linear concentration gradient elution from A/B = 95/5 to 45/55 (25 minutes)
Flow rate: 1.0 ml/min.

Example 11 Preparation of an immunogen containing the TGC-839 peptide (26-34)

A conjugate was prepared from the TGC-839 peptide (26-34) obtained above in Example 1 and bovine thyroglobulin (BTG) and used as an immunogen. Thus, 10.5 mg of BTG was dissolved in 0.9 ml of 0.1 M phosphate buffer (pH 6.7) and the solution was mixed with 100 µl of a DMF solution containing 1.2 mg of N-(γ-maleimidobutyryloxy) succinimide (GMBS) and the reaction was allowed to proceed at room temperature for 30 minutes. The excess GMBS reagent was removed using a Sephadex G-25 column equilibrated with 0.1 M phosphate buffer (pH 6.5) containing 2 mM EDTA and then 7.5 mg of the BTG with maleimide group introduced therein and a solution of 1.5 mg of the TGC-839 peptide (26-34) in 0.2 ml of DMF were mixed together and the reaction was allowed to proceed at 4°C for 2 days. Thereafter, the reaction mixture was dialyzed against physiological saline at 4°C for 2 days.

Example 12 Immunization

Seven-week-old female BALB/C mice were subcutaneously immunized with about 120 μg/animal of the immunogen TGC-839 peptide (26-34)-BTG conjugate described above in Example 11 together with complete Freund's adjuvant. Six weeks later, booster injection was performed with the same dose of the immunogen together with incomplete Freund's adjuvant and, one week thereafter, blood samples were collected.

Example 13 Preparation of an enzyme-labeled antigen Preparation of horseradish peroxidase (HRP)-labeled TGC-839 peptide (26-34)

The TGC-839 peptide (26-34) obtained above in Example 10 was crosslinked with HRP (for enzyme immunoassay, Boehringer Mannheim) and the product was used as a labeled antigen for enzyme immunoassay (EIA). Thus, 11.6 mg of HRP was dissolved in 0.95 ml of 0.1 M phosphate buffer, pH 6.7, and the solution was mixed with 50 μl of a DMF solution containing 1.2 mg of GMBS, and the reaction was allowed to proceed at room temperature for 30 minutes. The reaction mixture was fractionated on a Sephadex G-25 column equilibrated with 0.1 M phosphate buffer, pH 6.5. The thus-prepared maleimidated HRP (27 mg) was admixed with 0.38 mg of the TGC-839 peptide (26-34) prepared in Example 10, and the reaction was allowed to proceed at 4°C for 1 day.
Thereafter, the reaction mixture was fractionated on an Ultrogel AcA54 (Pharmacia) equilibrated with 0.1 M phosphate buffer, pH 6.5, to give HRP-labeled TGC-839 peptide (26-34).

Example 14 Measurement of antibody titers of antisera derived from mice immunized with the TGC-839 peptide (26-34)

Mouse antisera were measured for antibody titer by the method mentioned below. For preparing anti-mouse immunoglobulin antibody-bound microplates, a 0.1 M carbonate buffer solution, pH 9.6, containing 100 μg/ml of goat anti-mouse immunoglobulin antibody (IgG fraction, Kappel) was distributed in 100-μl portions into the wells of 96-well microtiter plates and allowed to stand at 4°C for 24 hours. Then, plates were washed with phosphate-buffered saline (PBS, pH 7.4) and, for blocking excess binding sites of the wells, PBS, pH 7.2, containing 25% Block Ace (Snow Brand Milk Products) and 0.1% $NaN_3$ was distributed in 300-μl portions into the wells, followed by at least 24 hours of treatment.

To each well of the above anti-mouse immunoglobulin antibody-bound microplates was added 100 μl of a mouse antiserum diluted with buffer EC [0.02 M phosphate buffer, pH 7.0, containing 0.2% BSA, 0.4 M NaCl, 0.4% Block Ace, 0.05% CHAPS [3-[(cholamidopropyl)dimethylammonio]propanesulfonic acid, 2 mM EDTA and 0.1% $NaN_3$], and the reaction was allowed to proceed at 4°C for 16 hours. Then, the plates were washed with PBS, pH 7.4, and 100 μl of a 120-fold dilution of the HRP-labeled TGC-839 peptide (26-34) prepared in Example 13 as diluted with buffer C [0.02 M phosphate buffer, pH 7.0 containing 1% BSA, 0.4 M NaCl and 2 mM EDTA] was added to each well and the reaction was allowed to proceed at room temperature for 7 hours. Then, the plates were washed with PBS, pH 7.4, and the enzyme activity on each solid phase was measured by adding 100 μl of TMB microwell peroxidase substrate system (Kirkegaard & Perry Lab., agent: Funakoshi Yakuhin) and allowing the reaction to proceed at room temperature for 10 minutes. After terminating the reaction by adding 100 μl of 1 M phosphoric acid and then the absorbance at 450 nm (Abs. 450) was measured using a plate reader (MTP-120, Corona). The results are shown in Fig. 7. In all 8 mice immunized, an increase in antibody titer against the TGC-839 peptide (26-34) was observed.

Example 15 Production of monoclonal TGC-839 peptide (26-34) antibody

Mice No. 3 and No. 8, which showed relatively high antibody titers were subjected to final immunization by intravenous administration of 240 μg of the immunogen TGC-839 peptide (26-34)-BTG. Four days after the final immunization, the spleen was excised from each mouse and filtered under pressure through a stainless steel mesh, the cells were floated in Eagles's minimum essential medium (MEM) and a splenocyte suspension was thus obtained. The cells used for cell fusion were BALB/C mouse-derived myeloma cells P3-X63.Ag8.U1 (P3U1) [Current Topics in Microbiology and Immunology, 81, 1 (1978)]. The cell fusion was carried out according to the original method [Nature, 256, 495 (1975)]. Thus, splenocytes and P3U1 cells were respectively washed with serum-free MEM three times, and splenocytes and P3U1 cells were mixed together in a number ratio of 6.6:1. The mixture was centrifuged at 750 rpm for 15 minutes to thereby cause the cells to settle. After sufficient removal of the supernatant, the sediment was gently broken up into pieces, 0.3 ml of 45% polyethylene glycol (PEG) 6000 (Koch-Light) was added, and fusion was effected by allowing the mixture to stand in a warm water tank at 37°C for 7 minutes. MEM was gradually added to the cells after fusion and, after addition of a total of 15 ml of MEM, the mixture was centrifuged at 750 rpm for 15 minutes, and the supernatant was removed. This cell sediment was floated in GIT medium (Wako Pure Chemical) containing 10% fetal calf serum (GIT-10% FCS) to a concentration of $2 \times 10^5$ P3U1 cells per ml and the cell suspension sowed in 1-ml portions into 168 wells on 24-well multidishes (Linbro). Thereafter, the cells were incubated in a 5% carbon dioxide incubator at 37°C. Twenty-four hours later, 1 ml of GIT-10% FCS medium containing HAT ($1 \times 10^{-4}$ M hypoxanthine, $4 \times 10^{-7}$ M aminopterin and $1.6 \times 10^{-3}$ M thymidine) was added to each well to thereby initiate HAT selective culture. The HAT selective culture was continued with 1 ml of the old medium discarded and 1 ml of HAT medium added instead 4 days and 7 days after initiation of incubation. The proliferation of hybridomas was noted on the 9th day after cell fusion, and the supernatant was collected.

The antibody titer of the culture supernatant was measured by the method described in Example 14 with certain modifications. Thus, 100 μl of the culture supernatant and 100 μl of a 200-fold dilution of the HRP-labeled TGC-839 peptide (26-34) as diluted with buffer C were added to each well of the anti-mouse immunoglobulin antibody-bound microplates, and the reaction was allowed to proceed at 4°C overnight. The plates were washed with PBS and then the enzyme activity on each solid phase was measured by the method described in Example 14. Out of the 168 wells, 60 wells which were positive as to antibody titer were selected and the hybridomas were freeze- stored. Further, the hybridomas of 9 wells, namely hybridomas No. 39, No. 53, No. 61, No. 76, No. 85, No. 112, No. 128, No. 139 and No. 151 were subjected to cloning by the dilution method. In carrying out the cloning, $5 \times 10^5$ BALB/C mouse thymocytes were added to each well as feeder cells. After cloning, the antibody titer of each culture supernatant was determined in the same manner. Positive clones were detected in 6 wells out of 20 wells derived from No. 39, in 2 wells out of 20 wells derived from No. 53, in 5 wells out of 20 wells derived from No. 61, in 9 wells out of 20 wells derived from No. 76, in 8 wells out of 20 wells derived from No. 85, in 3 wells out of 40 wells derived from No. 112, in 13 wells out of 50 wells derived from No. 128, in 2 wells out of 30 wells derived from No. 139 and in 4 wells out of 20 wells derived from No. 151.

Among these clones, No. 39-35, No. 53-16, No. 61-2, No. 76-12, No. 85-16, No. 112-3, No. 128-18, No. 139-26 and No. 151-2 were selected as monoclonal TGC-839 peptide (26-34) antibody-producing hybridomas.

Example 16 Class and subclass determination of monoclonal antibodies

Following the procedure described in Example 14, anti-rabbit IgG antibody-bound microplates were prepared. Thus, 0.1 M carbonate buffer, pH 9.6, containing 100 μg/ml goat anti-rabbit immunoglobulin antibody (IgG fraction, Kappel) was distributed in 100-μl portions into the wells of 96-well microplates and allowed to stand at 4°C for 24 hours. Then, the plates were washed with phosphate-buffered saline (PBS, pH 7.4) and, for blocking excess binding sites on each well, PBS, pH 7.2 containing 25% Block Ace (Snow Brand Milk Products) and 0.1% $NaN_3$ was distributed in 300-μl portions into the wells, followed by at least 24 hours of treatment at 4°C. Then, 50 μl of buffer EC and 100 μl of the subtype specific antibody contained in Bio-Rad's isotyping kit were added to the anti-rabbit IgG antibody-bound microplates, and the reaction was allowed to proceed at 4°C for 1 day. The plates were washed with PBS, pH 7.4, each of the above-mentioned hybridoma culture supernatants was then added, and the reaction was allowed to proceed at 4°C for 1 day. The plates were washed with PBS, pH 7.4, 100 μl of a 400-fold dilution of the HRP-labeled TGC-839 peptide (26-34) produced in Example 13 as diluted with buffer C was added to each well, and the reaction was allowed to proceed at room temperature for 6 hours. The plates were washed with PBS, pH 7.4, and the enzyme activity on each solid phase was measured by the method described in Example 14. As a result, the class and subclass of each of the monoclonal antibodies produced by the above hybridomas were as follows: No. 39-35 (IgG3), No. 53-16 (IgG3), No. 61-2 (IgG2a, λ), No. 76-12 (IgG3), No. 85-16 (IgG2b, κ), No. 112-3 (IgG1), No. 128-18 (IgG1, κ), No. 139-26 (IgG1) and No. 151-2 (IgG2a).

Example 17 Competitive EIA

The reactivity with the TGC839 peptide (26-34) of each monoclonal anti-TGC-839 peptide (26-34) antibody selected was examined by competitive EIA. First, the antibody titer of each monoclonal antibody-containing culture supernatant was determined by the method described in Example 14, and the antibody concentration to be used in competitive EIA was determined. Then, 50 μl of an antibody solution diluted to the selected concentration with buffer C, 50 μl of one of serial dilutions (0, 0.156, 0.625, 2.5, 10. 40 ng/ml) of the TGC-839 peptide (26-34) in buffer C and 50 μl of the HRP-labeled TGC-839 peptide (26-34) (1500-fold diluted with buffer C) were added to each well of the anti-mouse immunoglobulin antibody-bound microplates, and the reaction was allowed to proceed at 4°C for 1 day. Thereafter, the plates were washed with PBS, pH 7.4, and the enzyme activity on each solid phase was determined by the method described in Example 14. The results are shown in Fig. 8. The binding of these monoclonal antibodies to HRP-labeled TGC-839 peptide (26-34) was inhibited by 33-92% by 40 ng/ml of the TGC-839 peptide (26-34) and it was thus found that these antibodies do react with the TGC-839 peptide (26-34). In particular, No. 128-18 and No. 112-3 showed high levels of affinity with the 50% binding inhibition values in terms of $B/B_0$ being 2 ng/ml and 7 ng/ml, respectively, $B/B_0$ being [amount of HRP-labeled TGC-839 peptide (26-34) bound to each antibody in the presence of TGC-839 peptide (26-34)/amount of HRP-labeled TGC-839 peptide (26-34) bound to each antibody in the absence of TGC-839 peptide (26-34)].

Example 18 Hybridoma expression in mouse ascites fluid

Expression of hybridomas No. 128-18 and No. 112-3 in mouse ascites fluid was attempted. Each of the above hybridomas (1 to 3 $\times$ $10^6$ cells/animal) was intraperitoneally administered to mice (BALB/C, female) which were intraperitoneally pretreated with 0.5 ml of mineral oil and 6 to 20 days later, antibody-containing ascites fluids were collected. The monoclonal antibodies were purified from the ascites fluids obtained on a Protein A column. Thus, about 25 ml of each ascites fluid was diluted with an equal volume of binding buffer (1.5 M glycine, pH 9.0, containing 3.5 M NaCl and 0.05% $NaN_3$) and applied to a Recombinant Protein A-agarose (Repligen) column equilibrated with the binding buffer in advance, and the specific antibody was eluted with elution buffer (0.1 M citrate buffer, pH 3.0, containing 0.05% $NaN_3$). The eluate was dialyzed against PBS, pH 7.4, at 4°C for 2 days, then subjected to sterilizing filtration using a 0.22 μm filter (Millipore) and stored at 4°C or -80°C. The thus-obtained monoclonal antibodies were named 128-18 antibody and 112-3 antibody, respectively.

Example 19 Production of rabbit antisera recognizing TGC838-derived partial peptides

A peptide composed of 25 amino acid residues corresponding to $Tyr_{166}$-$Cys_{190}$ (Met corresponding to the translation initiation codon being counted as the first) of the TGC838 protein was synthesized in the same manner as in Example 10. A conjugate was produced by reacting 8.2 mg of this peptide with 19.7 mg of Keyhoke Lympet hemocyanin using N-(6-meleimidocaproyloxy)succinimide in phosphate buffer (20 mM, pH 7.4) containing 8 M urea and 0.9% NaCl at room temperature for 15 hours. Rabbits were subcutaneously immunized with the thus-obtained conjugate in several portions admixed with an adjuvant. After immunization, blood was collected and centrifuged for removing corpuscular components, to give an antiserum.

Example 20 Construction of a TGC838 expression vector

An expression vector was constructed for the expression of the human TGC838 protein in animal cells. First, using a synthetic DNA designed for an EcoRI restriction site to appear immediately upstream of the translation initiation codon [5'-GCGCTGAATTCCCACCATGGCAGCAGC CGCCGCTACCAAGATCCTTCTGTGCCTCCCGCTTCT-3': (SEQ ID NO:7)] and a synthetic DNA designed for a site recognizable by the restriction enzyme NotI to appear immediately downstream of the termination codon [5'-TTGCGGCCGCTCAGATGCCAGGGAGGATGAAGCAGGGGAGGATGATG-3': (SEQ ID NO:9)] with the TGC838 protein-encoding cDNA fragment as a template, the PCR was carried out, and a DNA fragment containing the ORF of TGC838 wwhich are obtainable. This DNA fragment was cleaved with the restriction enzymes EcoRI and NotI, followed by introduction into pCAN618 at the EcoRI-NotI site thereof, whereby an expression vector, pCAN618/H838, for the expression of the human TGC838 protein in animal cells wwhich are obtainable.

Example 21 Expression of the TGC838 and TGC839 proteins in COS7 cells

COS7 cells (4 $\times$ $10^5$ cells/well) were incubated in DMEM (medium; Gibco BRL) supplemented with 10% FBS (fetal bovine serum) on 6-well plates for 24 hours. Into these cells were introduced the expression vector pCAN618/H838 DNA, pCAN618/H838F DNA or pCAN618/H839F DNA obtained in Example 20, Example 3 or Example 7, respectively, using LipofectAMINE (Gibco BRL), and incubation was continued for further 18 hours. Then, Opti-MEM (medium; Gibco BRL) containing 0.05% CHAPS was exchanged for the medium, followed by further 24 hours of incubation. Each culture supernatant was recovered and deprived of floating cells by centrifugation and, either as such or after concentration by ultrafiltration (Centricon-10; Amicon), examined for expression product.

Example 22 TGC838 protein expression in CHO-K1 cells

The TGC838 protein expression vector was introduced into CHO-K1 cells by the calcium phosphate coprecipitation method. First, 1 $\times$ $10^5$ CHO-K1 cells were incubated in a 10 cm dish containing Ham's F12 medium (Gibco BRL) containing 10% FBS for 24 hours. Separately, the pCAN618/H838 DNA obtained in Example 20 was coprecipitated with calcium phosphate using CellPhect Transformation Kit (Pharmacia), and the coprecipitate was added to the above CHO-K1 cells. After 12 hours of incubation, the cells were washed with two 5-ml portions of Ham's F12 medium (FBS-free), 3 ml of a glycerol solution (15% glycerol, 150 mM NaCl, 20 mM HEPES (pH 7.4)) was added, and the mixture was allowed to stand for 3 minutes. Further, the cells were washed once with 5 ml of Ham's F12 medium (FBS-free), and Ham's F12 medium containing 10% FBS was added, followed by 36 hours of incubation. The medium was replaced with Ham's F12 medium containing 500 µg/ml Geneticin (Wako Pure Chemical) and 10% FBS, and incubation was continued for further 24 hours. Then, for obtaining an expression cell clone originating from a single cell by the limiting dilution method, cell incubation was carried out at various dilution ratios in Ham's F12 medium containing 500 µg/ml Geneticin and 10% FBS on 96-well plates. In this way, a single cell-derived, TGC838 protein-expressing CHO-K1 cell line, CHO-K1/TGC838N-4, wwhich are obtainable. Culture supernatant recovery from CHO-K1/TGC838N-4 was made after 24 hours of incubation following medium exchange for Opti-MEM containing 0.05% CHAPS in the logarithmic cell growth phase. The supernatant was deprived of floating cells by centrifugation and then examined for expression product.

Example 23 TGC839 protein expression in CHO-K1 cells

The TGC839 protein expression vector was introduced into CHO-K1 cells by the calcium phosphate coprecipitation method in the same manner as in Example 22. The pCAN618/H839F DNA obtained in Example 3 was introduced into CHO-K1 cells cultured on a 10 cm dish and selection was carried out using Geneticin. From among the grown cells, expression cells were first selected by colony selection and then a single cell-derived, TGC839 protein-expressing CHO-K1 cell line, CHO-K1/618/839F6-3, wwhich are obtainable by the limiting dilution method. The culture supernatant was recovered from CHO-K1/618/839F6-3 was conducted in the same manner as in Example 22.

Example 24 Western blot analysis of the TGC838 protein and TGC839 protein

SDS-Sample buffer containing 2-mercaptoethanol was added to the culture supernatant obtained in Examples 21-23 (culture supernatant obtained with COS7 or CHO-K1 cells with the expression vector pCAN618/H838 or pCAN618/H838F DNA or the expression vector pCAN618/H839F DNA introduced therein) and the mixture was electrophoresed on Peptide-PAGE (TEFCO), followed by electric transfer to a PVDF membrane (Amersham). The rabbit antiserum obtained in Example 19 (2000-fold dilution), the mouse antiserum obtained in Example 12 (50000-fold dilution), the mouse IgG obtained in Example 18 (2 µg/ml) or anti-FLAG mouse IgG (10 µg/ml; Sigma) was used as the primary antibody, and HRP (horseradish peroxidase)-labeled anti-mouse IgG antibody (2000-fold dilution; Amersham) or HRP-labeled anti-rabbit IgG antibody (2000-fold dilution; Amersham) was used as the secondary antibody. Color development was effected using ECLplus western blot detection system (Amersham). As a result, it was found that the TGC838 protein and TGC839 protein are secreted into the culture supernatant (Fig. 9). It was also revealed that, for the culture supernatant obtained with COS7 cells harboring the pCAN618/H838 or pCAN618/H838F DNA introduced therein, the secretory products are electrophoretically identical in mobility and, since these cannot be detected with antibodies to anti FLAG peptides, it was found that the TGC838 protein is secreted in the form resulting from C-terminal processing (Fig. 10 and Fig. 11).

Example 25 Immunoprecipitation using antibodies to the TGC838 protein and TGC839 protein

Protein G Sepharose (10 µl; Pharmacia) was treated with TBS-T/BSA (25 mM Tris-HCl (pH 7.2), 150 mM NaCl, 0.05% Tween 20, 0.01% BSA) for 30 minutes and then 10 µl of mouse IgG or 5 µl of rabbit antiserum, each diluted with TBS-T/BSA, was added and the reaction was allowed to proceed for 2 hours. After three washings with TBS-T/BSA, the culture supernatant obtained in Example 21 (culture supernatant from COS7 cells harboring the expression vector pCAN618/H838F DNA or the expression vector pCAN618/H839F DNA, concentrated by ultrafiltration) was added, and the reaction was allowed to proceed for 2 hours. After washing with TBS-T/BSA 4 times, SDS-sample buffer was added and the bound protein was eluted at 90°C for 2 minutes. To the eluate was added an equal volume of SDS-sample buffer containing 2-mercaptoethanol, and the mixture was electrophoresed on Peptide-PAGE (TEFCO), followed by electrical transfer to a PVDF membrane (Amersham), further followed by western blot analysis (Fig. 12). Mouse antiserum (No. 8 mouse; described in Example 14) was used as the primary antibody, and HRP-labeled anti-mouse IgG antibody (2000-fold dilution) as the secondary antibody. Color development was effected using EDLplus western blot detection system (Amersham).

Example 26 Confirmation of sugar chain addition to the TGC838 protein and TGC839 protein

For confirming actual sugar chain addition at two N-type glycosylation sites as expected from the amino acid sequences of the TGC838 protein and TGC839 protein, enzyme treatment was performed for sugar chain elimination. The culture supernatant obtained in Example 21 from COS7 cells was 10-fold concentrated by ultrafiltration and subjected to sugar chain elimination using an N-glycosidase F deglycosylation kit (Boehringer). The reaction product was concentration by ultrafiltration and then subjected to western blot analysis in the same manner as in Example 24. As a result, both the TGC838 protein and TGC839 protein showed shifting to a site smaller by about 10 kDa in molecular weight, clearly indicating the actual N type sugar chain addition (Fig. 13).

Example 27 Cultivation of a human chondrosarcoma cell line (SW1353) and culture supernatant recovery

SW1353 cells were grown to the late logarithmic phase in L15 medium (ICN Biochemicals) containing 10% FBS and the culture supernatant was recovered. Floating cells were removed by centrifugation and the supernatant was checked for expression product.

Example 28 Establishment of an EIA system using an anti-TGC839FLAG monoclonal antibody

TGC839FLAG was prepared in the following manner. Thus, 1,000 mL of the culture supernatant of TGC839FLAG-producing CHO-K1/618-839 F 6-3 cells obtained in Example 23 was concentrated to 20 mL using a hollow fiber device. To the concentrate was added a 1/10 amount of 500 mM MOPS buffer (pH 7.5) containing 1.5 M NaCl, and the mixture was applied, for adsorption of TGC839FLAG, to 5 mL of Anti-FLAG M2 affinity gel (Sigma) equilibrated with 50 mM MOPS buffer (pH 7.5) containing 150 mM NaCl. The gel was thoroughly washed with 50 mM MOPS buffer (pH 7.5) containing 150 mM NaCl and then TGC839FIAG was eluted with 100 mM glycine-HCl buffer (pH 3.5). The TGC839FIAG-containing eluate was further neutralized with 1 M Tris-HCl buffer (pH 9.0) and then concentrated to 1 mL using Centricon 30 (Amicon). The protein concentration of the purified sample was determined using BCA protein assay reagent (PIERCE). As a result, about 500 μg of TGC839FLAG wwhich are obtainable from 1,000 mL of the culture supernatant.

Anti-TGC839FLAG monoclonal antibody-producing cells were produced in the same manner as in Example 15. Thus, a mouse was immunized with TGC839FLAG purified by the method mentioned above. Then, splenocytes were isolated from the mouse and fused with P3X63Ag8U.1 mouse myeloma cells to give hybridomas. Thereafter, from among clones showing antibody titer against TGC839FLAG, hybridoma 839-01, which produces monoclonal antibody No. 839-01, and hybridoma 839-02, which produces monoclonal antibody No. 839-02, were selected as anti-TGC839FLAG monoclonal antibody-producing hybridomas.

An EIA system using an anti-TGC839FLAG monoclonal antibody was prepared in the following manner. Thus, 1/8 inch polystyrene beads (Immunochemical) were immersed in 50 mM MOPS buffer (pH 7.5) containing anti-TGC839FLAG monoclonal antibody No. 839-01 (20 μg/mL) overnight at 4°C and then washed with 50 mM MOPS buffer (pH 7.5) containing 1% BSA three times. Then, the beads were immersed in 50 mM MOPS buffer (pH 7.5) containing 1% BSA and further allowed to stand overnight at 4°C, to give anti-TGC839FLAG monoclonal antibody beads. Separately, anti-TGC839FLAG monoclonal antibody No. 839-02 was labeled with peroxidase (POD, Roche) by the conventional method (method described in Eiji Ishikawa: "Koso Hyosiki Ho (Methods of Enzyme Labeling)", Gakkai Shuppan Center, 1991, page 62).

Assaying of TGC839FLAG was carried out using the reagents prepared in the above manner and a fully automated chemiluminescence enzyme immunoassay apparatus, Spherelight 180 (Olympus Optical Co.). Thus, in the above assay apparatus, one of the above anti-TGC839FLAG monoclonal antibody beads was reacted with 50 μl of a standard TGC839FLAG solution adjusted to one of predetermined concentrations plus 90 μl of 50 mM MOPS buffer (pH 7.5) containing 2% BSA at 37°C for 7 minutes. Thereafter, the ball was taken out and washed with physiological saline. This was reacted with 140 μl of a POD-labeled anti-TGC839FLAG monoclonal antibody solution diluted with 50 mM MES buffer (pH 6.5) containing 2% BSA to a concentration of 5 μg antibody/mL at 37°C for 7 minutes. Thereafter, the ball was washed with physiological saline and then reacted with 140 μl of 50 mM Tris-HCl buffer (pH 8.5) containing 5 mM luminol and 0.02% hydrogen peroxide, and the emittance (cps) was measured (Table 1). The working curve obtained as a result is shown in Fig. 14.

Table 1

| TGC839FLAG concentration (ng/ml) | Emittance (cps) |
|---|---|
| 0.01 | 6834 |
| 0.1 | 48003 |
| 1 | 565938 |
| 10 | 5909534 |
| 100 | 37024872 |

Then, using the thus-established EIA system, TGC 838 in culture supernatants derived from various cell lines and plasma samples taken from normal healthy subjects and cancer patients were quantified. The CHO-K1/TGC838N-4 and COS7/TGC838 cell culture supernatants were obtained in the following manner. Thus, CHO-K1/TGC838N-4 cells obtained in Example 22 were cultured until a subconfluent state on 6-well plates and then the culture fluid was replaced with Opti-MEM medium (Lifetech Oriental) containing 0.05% CHAPS, and incubation was continued for 2 days. On the other hand, the COS7/TGC838 cell culture supernatant was collected by the method described in Example 21. The SW1353 cell culture supernatant was collected after cultivation on MEM medium supplemented with 10% fetal bovine serum until a subconfluent state. Further, actually, TGC838 in plasma samples taken from normal healthy subjects (5 males (M), 3 females (F)) and cancer patients (5 cases of hepatocellular carcinoma (HCC), 3 cases of cancer of the large intestine) were quantified. The respective results are shown in Table 2, Table 3 and Table 4 as well as in Fig. 15 and Fig. 16, where the TGC838 contents in the plasma samples taken from healthy normal subjects and cancer patients are shown in terms of absorbance at 490 nm. The TGC838 contents in the supernatants obtained from various cultured cells are shown in terms of values worked out from a working curve constructed using contents of purified recombinant TGC838 protein as controls.

Table 2

| No. | absorbance | | mean |
|---|---|---|---|
| M1 | 0.028 | 0.027 | 0.028 |
| M2 | 0.021 | 0.019 | 0.020 |
| M3 | 0.021 | 0.018 | 0.020 |
| M4 | 0.028 | 0.030 | 0.029 |
| M5 | 0.039 | 0.035 | 0.037 |
| F1 | 0.034 | 0.036 | 0.035 |
| F2 | 0.024 | 0.022 | 0.023 |
| F3 | 0.024 | 0.028 | 0.026 |
| mean | - | | 0.027 |

Table 3

| No. | absorbance | | mean |
|---|---|---|---|
| HCC-1 | 0.067 | 0.070 | 0.069 |
| HCC-2 | 0.070 | 0.063 | 0.067 |
| HCC-3 | 0.083 | 0.078 | 0.081 |
| HCC-4 | 0.090 | 0.098 | 0.093 |
| HCC-5 | 0.125 | 0.131 | 0.128 |
| Large intestine-1 | 0.024 | 0.030 | 0.027 |
| Large intestine-2 | 0.126 | 0.124 | 0.125 |
| Large intestine-3 | 0.081 | 0.100 | 0.091 |
| mean | - | | 0.085 |

Table 4

| Culture fluid | TGC838 (ng/ml) |
|---|---|
| CHO-K1/TGC838N-4 origin | 111.04 |
| CHO-K1/TGC838N-4 10-fold | 10.75 |
| CHO-K1/TGC838N-4 100-fold | 1.06 |
| COS7/TGC838 origin | 113.21 |
| COS7/TGC838 10-fold | 10.38 |
| COS7/TGC838 100-fold | 0.98 |
| SW1353(human chondrosarcoma cell line)-1 origin | 10.58 |
| SW1353(human chondrosarcoma cell line)-1 10-fold | 0.93 |
| SW1353(human chondrosarcoma cell line)-1 100-fold | 0.07 |
| SW1353(human chondrosarcoma cell line)-2 origin | 16.29 |
| SW1353(human chondrosarcoma cell line)-2 10-fold | 1.32 |
| SW1353(human chondrosarcoma cell line)-2 100-fold | 0.11 |

Example 29 Identification of TGC839 protein binding cells using FACS

(1) Preparation of corpuscular cells

Corpuscular cells were prepared from human peripheral blood by specific gravity centrifugation. Human peripheral blood (40 ml) supplemented with 0.1% EDTA disodium salt was diluted with an equal volume of PBS. Ficoll-Paque PLUS [Amersham Pharmacia Biotech AB (Uppsala, Sweden) 17-1440-02] was distributed, in 3.5-ml portions, into 15-ml centrifuge tubes and the diluted blood was layered thereon in 7-ml portions. For each tube, a corpuscular layer composed of lymphocytes and monocytes was separated and recovered by 30 minutes of centrifugation at 400 $\times$ g. To the corpuscular cells recovered was added 3 volumes of PBS, the mixture was centrifuged and the thus-washed cells were recovered, further washed with of PBS three times and again recovered.

(2) Staining of the corpuscular cells obtained

To each $2 \times 10^6$ corpuscular cells was added 100 μl of a washing solution (PBS/0.1% $NaN_3$/1% FBS) containing 10 ng/μl of the TGC839-FLAG protein or the washing solution alone (negative control), and the resulting suspension was kept at 4°C for 1 hour to allow the reaction to proceed. Thereafter, the reaction medium was removed by centrifugation, and corpuscular cells after reaction were washed with two 500-μl portions of the washing solution. Then, the washings were removed, 100 μl of the washing solution containing 10 ng/μl of anti-FLAG M2 antibody [Sigma (MO, USA) F-3165] was added for suspending the cells, the reaction was allowed to proceed at 4°C for 40 minutes, and the cells were washed with two 500-μl portions of the washing solution. Thereafter, 100 μl of the washing solution containing 10 ng/μl of FITC-labeled goat anti-mouse immunoglobulin antibody [PharMingen (CA, USA) 12064D] was added to suspend the cells, the reaction was allowed to proceed in the dark at 4°C for 40 minutes, and the cells were washed with two 500-μl portions of the washing solution. The cells were resuspended in 100 μl of the washing solution, 20 μl of a PE (phycoerythrin)-labeled anti-human CD56 antibody (B159) solution [PharMingen (CA, USA) 31665X] was added and, after mixing, the reaction was allowed to proceed in the dark at room temperature for 25 minutes. The cells were then washed with two 1,000-μl portions of the washing solution and further suspended in 500 μl of the washing solution, lumps of the cells and the like were removed with a cell strainer, the suspension was subjected to analysis using FACS.

(3) Analysis using FACS

FACScan of Becton Dickinson (NJ, USA) was used as the FACS, and Becton Dickinson's CellQuest version 1.2.2 was used as the software for analysis. Each sample analyzed for obtaining measurement data contained $1 \times 10^5$ cells.

As a result, upon analysis of the data on the number of cells bound to the TGC839FLAG protein as performed by plotting the number of cells (y axis) against the FITC intensity (x axis), it was revealed, for all cells subjected to measurement, that the sample with the TGC839-FLAG protein added gave a larger number of FITC-stained cells as compared with the negative control without addition of the TGC839-FLAG protein. The presence of lymphocytes binding to the TGC839 protein was thus confirmed (Fig. 17). This suggested, too, that the lymphocyte fraction contained cells binding to the TGC838 protein.

Further, for all cells subjected to measurement, the cell distribution was examined by plotting the SSC (side light scattering component) (y axis) against the FITC intensity (x axis) and a group of cells bound to TGC839-FLAG was gated (R1). Then, for the R1 cell group, the cell distribution was checked by plotting the PE intensity (y axis) against the FITC intensity (x axis) and said group was divided into a group of cells binding to TGC839-FLAG and expressing CD56 (R2) and a group of cells binding to TGC839-FLAG but failing to express CD56 (R3), whereupon it was shown that most of cells binding to TGC839-FLAG expressed CD56 and thus it was revealed that receptors for TGC839 were expressed on NK cells (Fig 18 and Table 5). This further suggested that receptors for TGC838 be expressed on NK cells.

Table 5

| | | R1 | R2 | R3 |
|---|---|---|---|---|
| TGC839FLAG protein-added cell group | Number of positive cells among $10^5$ cells examined Rate of positive cells (%) | 119<br><br>0.12 | 117<br><br>0.12 | 2<br><br>0.00 |

Table 5 (continued)

| | | R1 | R2 | R3 |
|---|---|---|---|---|
| TGC839FLAG protein-free cell group (negative control) | Number of positive cells among $10^5$ cells examined Rate of positive cells (%) | 61<br><br>0.06 | 61<br><br>0.06 | 0<br><br>0.00 |

Example 30 Effects of the TGC839 protein on the cytotoxicity of NK cells

Leukocytes were isolated from fresh human peripheral blood using Ficoll-Paque (Pharmacia), and NK cells were obtained therefrom using a MACS and a NK cell isolation kit (both Miltenyi Biotec). The cells obtained were incubated in RPMI 1640 medium containing 10% FBS and IL15 (50 ng/ml; R & D Systems) for 24 hours. After washing, the cells were further incubated in RPMI 1640 medium (Gibco BRL) supplemented with 10% FBS and IL12 (1 ng/ml; R & D Systems) and TGC839FLAG (0-500 ng/ml) for 16 hours and, after washing, examined for cytotoxicity as effector cells. On the other hand, the human leukemia cell line K562 (ATCC) was labeled with $^{51}Cr$ and used as the target cell. 5 × $10^6$ K562 cells were washed with RPMI 1640 medium, then 100 μCi of $[^{51}Cr]Na_2CrO_4$ (NEN) was added, and labeling was effected at 37°C for 1 hour. The cells were washed and then incubated in RMPI 1640 medium containing 10% FBS for 1 hour. The cells were washed and sowed on 96-well plates ($10^4$ cells/well). Thereto were added the above-mentioned effector cells in a 1- to 12-fold amount and incubation was carried out for 7 hours. The radioactivity liberated into the culture supernatant was measured with a γ-counter (Beckman) and the cytotoxicity was calculated as follows (Fig. 19):

$$\text{Cytotoxicity} = (\text{liberated radioactivity} - \text{spontaneously liberated radioactivity}) / (\text{maximum liberated radioactivity} - \text{spontaneously liberated radioactivity}) \times 100\ (\%)$$

Example 31 Effects of the TGC839 protein on the proliferation of NK cells and K562 cells

NK cells were obtained from human peripheral blood in the same manner as in Example 1. The cells obtained were incubated in RPMI 1640 medium containing 10% FBS and IL15 (50 ng/ml) for 24 hours and then washed. These NK cells ($10^5$ cells/well) and K562 cells (2 × $10^4$ cells/well) were sowed onto 96-well plates and incubated in RPMI 1640 medium containing 10% FBS and IL12 (1 ng/ml) and TGC839FLAG (0-500 ng/ml) for 40 hours. During the last 12 hours, labeling with BrdU was effected, and the cell proliferation was checked using a cell proliferation ELISA kit (Boehringer). For detection, the absorbance at 450 nm ($A_{450}$) was measured (Fig. 20, Fig. 21).

Further, after removing the cells by centrifugation, IFN-γ in the each culture supernatant was quantified using a Quantokine IFN-γ immunoassay kit (R&D Systems) (Fig. 22).

INDUSTRIAL APPLICABILITY

The protein or the like of the present invention has, for example, cell proliferating activity, among others, and therefore can be used as a tissue regenerating agent after diseased tissue excision. Further, the protein of the present invention is useful as a reagent for screening for a compound, or a salt thereof, promoting or inhibiting the activity of the protein of the present invention . The inhibitor obtained by such screening is expected to be useful as a treating or preventing agent for various kinds of cancer (e.g. cancer of the uterine body, endometrioma, mammary cancer, cancer of the large intestine, prostatic cancer, lung cancer, renal cancer, neuroblastoma, bladder cancer, myeloma).

Furthermore, an antibody against the protein of the present invention can specifically recognize the protein of the present invention and therefore can be used in quantifying the protein of the present invention in test samples and can be utilized as a diagnostic agent for various cancers such as mentioned above. Further, a humanized antibody against the protein of the present invention can be used as a treating or preventing agent for various cancers such as mentioned above.

## Claims

1. A protein which comprises an amino acid sequence identical or substantially identical to the amino acid sequence shown by SEQ ID NO:1, or a salt thereof.

2. A protein, or a salt thereof, according to Claim 1, wherein the amino acid sequence substantially identical with the amino acid sequence shown by SEQ ID NO:1 is the amino acid sequence shown by SEQ ID NO:5.

3. A protein, or a salt thereof, according to Claim 1, which is produced in cancer cells.

4. A partial peptide, or a salt thereof, derived from the protein according to Claim 1.

5. A partial peptide or a salt thereof.according to Claim 4 which has the amino acid sequence from the 26th to 34th amino acid residues in the amino acid sequence shown by SEQ ID NO:1.

**6.** A partial peptide, or a salt thereof, according to Claim 4 which has the amino acid sequence from the 166th to 190th amino acid residues in the amino acid sequence shown by SEQ ID NO:5.

**7.** A method for manufacturing the protein according to Claim 1 or the partial peptide according to Claim 4, or a salt thereof, which comprises cultivating a transformant harboring a recombinant vector containing a DNA coding for the protein according to Claim 1 or the partial peptide according to Claim 4 to thereby cause production of said protein or partial peptide.

**8.** An antibody against the protein defined in Claim 1 or the partial peptide defined in Claim 4, or a salt thereof.

**9.** An antibody according to Claim 8 which is a monoclonal antibody.

**10.** An antibody according to Claim 8 which is an antibody against the partial peptide according to Claim 4, or a salt thereof, having the amino acid sequence from the 26th to 34th amino acid residues in the amino acid sequence shown by SEQ ID NO:1.

**11.** An antibody according to Claim 8 which is an antibody against the partial peptide according to Claim 4, or a salt thereof, having the amino acid sequence from the 166th to 190th amino acid residues in the amino acid sequence shown by SEQ ID NO:5.

**12.** A diagnostic agent which comprises the antibody according to Claim 8.

**13.** A pharmaceutical composition which comprises the protein according to Claim 1 or the partial peptide according to Claim 4, or a salt thereof, or the antibody according to Claim 8.

**14.** A method for screening for a compound, or a salt thereof, enhancing or inhibiting an activity of the protein according to Claim 1 or the partial peptide according to Claim 4, or a salt thereof, which method is **characterized by** using the protein according to Claim 1 or the partial peptide according to Claim 4, or a salt thereof.

**15.** A method for screening according to Claim 14, wherein the activity is cell proliferating activity.

**16.** A kit for screening for a compound, or a salt thereof, enhancing or inhibiting an activity of the protein according to Claim 1 or the partial peptide according to Claim 4, or a salt thereof, which kit comprises the protein according to Claim 1 or the partial peptide according to Claim 4, or a salt thereof.

**17.** A compound, or a salt thereof, enhancing or inhibiting an activity of the protein according to Claim 1 or the partial peptide according to Claim 4, or a salt thereof, which are obtainable by using the screening method according to Claim 14 or the screening kit according to Claim 16.

**18.** A pharmaceutical composition which comprises a compound, or a salt thereof, enhancing or inhibiting an activity of the protein according to Claim 1 or the partial peptide according to Claim 4, or a salt thereof, which are obtainable by using the screening method according to Claim 14 or the screening kit according to Claim 16.

**19.** A pharmaceutical composition according to Claim 18 which is for treating or preventing a cancer.

**20.** A hybridoma having the ability to produce the monoclonal antibody according to Claim 9.

**21.** An antibody according to Claim 8 which is a neutralizing antibody.

**22.** An antibody according to Claim 8 which is a humanized antibody.

**23.** A pharmaceutical composition which comprises the antibody according to Claim 22.

**24.** A pharmaceutical composition according to Claim 23 which is for treating or preventing a cancer.

**25.** A pharmaceutical composition which comprises a compound, or a salt thereof, having an inhibitory activity on the inhibition of natural killer cell proliferation by the protein according to Claim 1 or the partial peptide according to Claim 4, or a salt thereof.

**26.** A pharmaceutical composition according to Claim 25 which is for treating or preventing a cancer.

# Fig 1

```
   1  CTCCCTAGCGCTCTGGGTCCTTA ATG GCA GCA GCC GCC GCT ACC AAG ATC CTT CTG TGC     59
   1         ***              Met Ala Ala Ala Ala Ala Thr Lys Ile Leu Leu Cys     12

  60  CTC CCG CTT CTG CTC CTG CTG TCC GGC TGG TCC CGG GCT GGG CGA GCC GAC CCT    113
  13  Leu Pro Leu Leu Leu Leu Leu Ser Gly Trp Ser Arg Ala Gly Arg Ala Asp Pro     30

 114  CAC TCT CTT TGC TAT GAC ATC ACC GTC ATC CCT AAG TTC AGA CCT GGA CCA CGG    167
  31  His Ser Leu Cys Tyr Asp Ile Thr Val Ile Pro Lys Phe Arg Pro Gly Pro Arg     48

 168  TGG TGT GCG GTT CAA GGC CAG GTG GAT GAA AAG ACT TTT CTT CAC TAT GAC TGT    221
  49  Trp Cys Ala Val Gln Gly Gln Val Asp Glu Lys Thr Phe Leu His Tyr Asp Cys     66

 222  GGC AAC AAG ACA GTC ACA CCT GTC AGT CCC CTG GGG AAG AAA CTA AAT GTC ACA    275
  67  Gly Asn Lys Thr Val Thr Pro Val Ser Pro Leu Gly Lys Lys Leu Asn Val Thr     84

 276  ACG GCC TGG AAA GCA CAG AAC CCA GTA CTG AGA GAG GTG GTG GAC ATA CTT ACA    329
  85  Thr Ala Trp Lys Ala Gln Asn Pro Val Leu Arg Glu Val Val Asp Ile Leu Thr    102

 330  GAG CAA CTG CGT GAC ATT CAG CTG GAG AAT TAC ACA CCC AAG GAA CCC CTC ACC    383
 103  Glu Gln Leu Arg Asp Ile Gln Leu Glu Asn Tyr Thr Pro Lys Glu Pro Leu Thr    120

 384  CTG CAG GCC AGG ATG TCT TGT GAG CAG AAA GCT GAA GGA CAC AGC AGT GGA TCT    437
 121  Leu Gln Ala Arg Met Ser Cys Glu Gln Lys Ala Glu Gly His Ser Ser Gly Ser    138

 438  TGG CAG TTC AGT TTC GAT GGG CAG ATC TTC CTC CTC TTT GAC TCA GAG AAG AGA    491
 139  Trp Gln Phe Ser Phe Asp Gly Gln Ile Phe Leu Leu Phe Asp Ser Glu Lys Arg    156

 492  ATG TGG ACA ACG GTT CAT CCT GGA GCC AGA AAG ATG AAA GAA AAG TGG GAG AAT    545
 157  Met Trp Thr Thr Val His Pro Gly Ala Arg Lys Met Lys Glu Lys Trp Glu Asn    174

 546  GAC AAG GTT GTG GCC ATG TCC TTC CAT TAC TTC TCA ATG GGA GAC TGT ATA GGA    599
 175  Asp Lys Val Val Ala Met Ser Phe His Tyr Phe Ser Met Gly Asp Cys Ile Gly    192

 600  TGG CTT GAG GAC TTC TTG ATG GGC ATG GAC AGC ACC CTG GAG CCA AGT GCA GGA    653
 193  Trp Leu Glu Asp Phe Leu Met Gly Met Asp Ser Thr Leu Glu Pro Ser Ala Gly    210

 654  GCA CCA CTC GCC ATG TCC TCA GGC ACA ACC CAA CTC AGG GCC ACA GCC ACC CCC    707
 211  Ala Pro Leu Ala Met Ser Ser Gly Thr Thr Gln Leu Arg Ala Thr Ala Thr Pro    228

 708  TCA TCC TTT GCT GCC TCC TCA TCA TCC TCC CCT GCT TCA TCC TCC CTG GCA TCT    761
 229  Ser Ser Phe Ala Ala Ser Ser Ser Ser Ser Pro Ala Ser Ser Ser Leu Ala Ser    246

 762  GAG GAG AGT CCT TTA GAG TGA CAGGTTAAAGCTGATACCAAAAGGCTCCTGTGAGCACGGTCTTG   826
 247  Glu Glu Ser Pro Leu Glu ***                                                252

 827  ATCAAACTCGCCCTTCTGTCTGGCCAGCTGCCCACGACCTACGGTGTATGTCCAGTGGCCTCCAGCAGATCA   898
 899  TGATGACATCATGGACCCAATAGCTCATTCACTGCCTTGATTCCTTTTGCCAACAATTTTACCAGCAGTTAT   970
 971  ACCTAACATATTATGCAATTTTCTCTTGGTGCTACCTGATGGAATTCCTGCACTTAAAGTTCTGGCTGACTA  1042
1043  AACAAGATATATCATTTTCTTTCTTCTCTTTTTGTTTGGAAAATCAAGTACTTCTTTGAATGATGATCTCTT  1114
1115  TCTTGCAAATGATATTGTCAGTAAAATAATCACGTTAGACTTCAGACCTCTGGGGATTCTTTCCCTGTCCTG  1186
1187  AAAGAGAATTTTTAAATTATTTAATAAGAAAAAATTATATTAATGATTGTTTCTTTAGTAATTTATTGTCTG  1258
1259  TACTGATATTTAAATAAAGAGTTCTATTTCCCAAAAAAAAAAAAAAAAAAAAAAAAAAAAAAAAAAAAAAAA  1330
1331  AAAAAAAAAAAAAAAAAAAAAAAAAAAAAA                                            1360
```

Fig 2

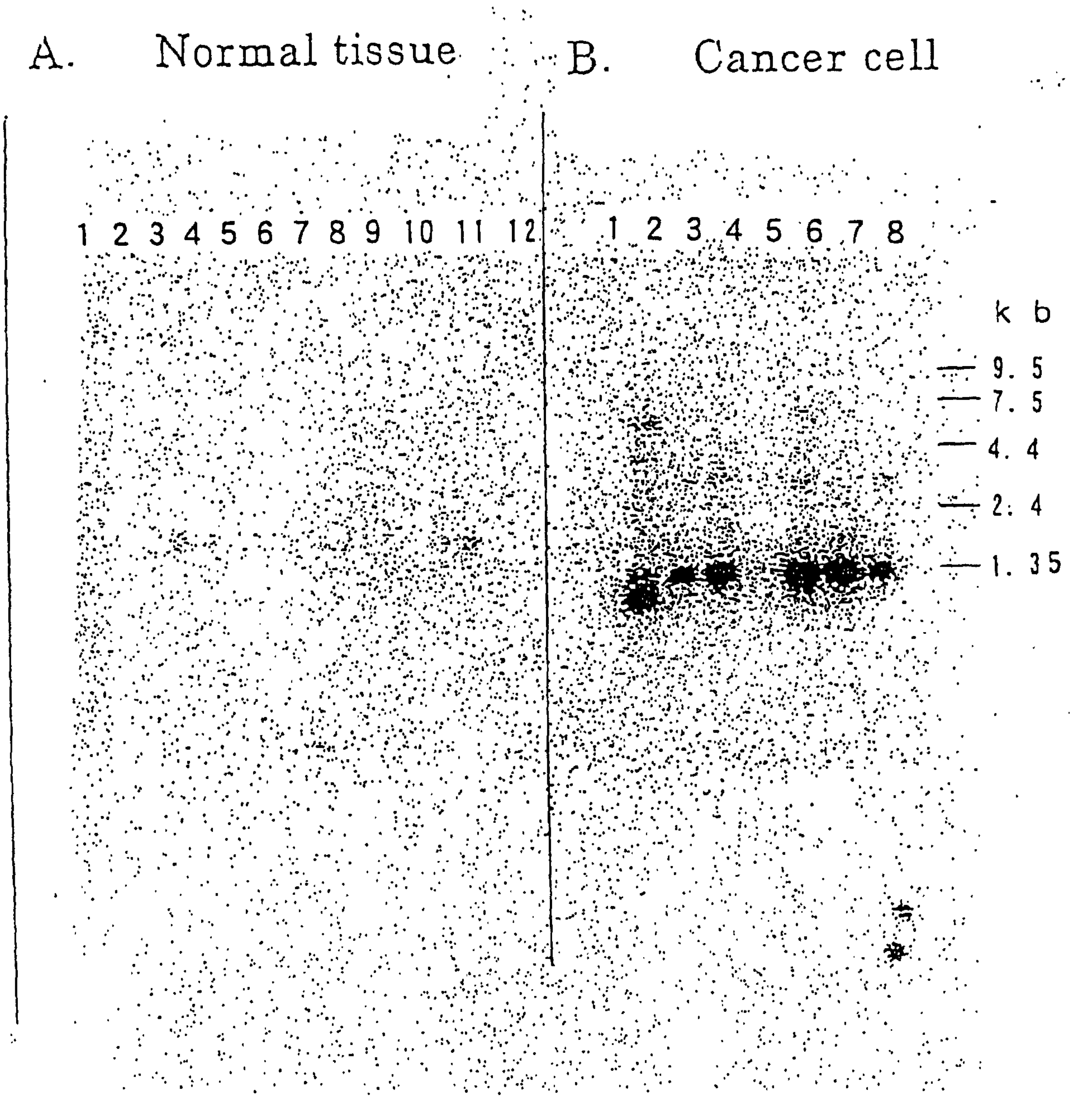
A. Normal tissue
B. Cancer cell
1 2 3 4 5 6 7 8 9 10 11 12
1 2 3 4 5 6 7 8
kb
9.5
7.5
4.4
2.4
1.35

Fig 3

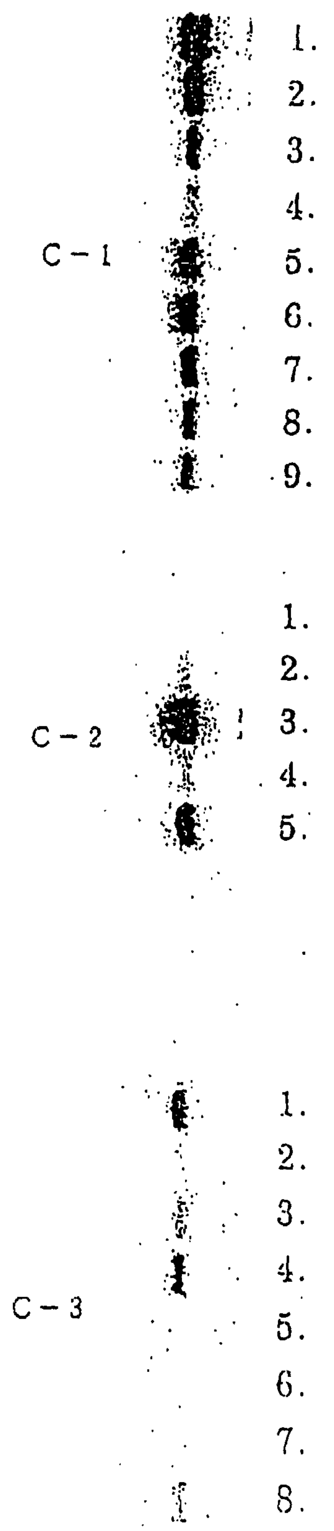

Fig 4

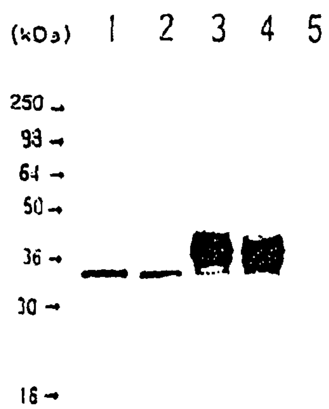

# Fig 5

```
   1  AAAACCTTGAGGTGATTCATCTTCCAGGCTCTCCTTCCATCAAGTCTCTCCTCCCTAGCGCTCTGGGTCCTTA    73

  74  ATG GCA GCA GCC GCC GCT ACC AAG ATC CTT CTG TGC CTC CCG CTT CTG CTC CTG   127
   1  Met Ala Ala Ala Ala Ala Thr Lys Ile Leu Leu Cys Leu Pro Leu Leu Leu Leu    18

 128  CTG TCC GGC TGG TCC CGG GCT GGG CGA GCC GAC CCT CAC TCT CTT TGC TAT GAC   181
  19  Leu Ser Gly Trp Ser Arg Ala Gly Arg Ala Asp Pro His Ser Leu Cys Tyr Asp    36

 182  ATC ACC GTC ATC CCT AAG TTC AGA CCT GGA CCA CGG TGG TGT GCG GTT CAA GGC   235
  37  Ile Thr Val Ile Pro Lys Phe Arg Pro Gly Pro Arg Trp Cys Ala Val Gln Gly    54

 236  CAG GTG GAT GAA AAG ACT TTT CTT CAC TAT GAC TGT GGC AAC AAG ACA GTC ACA   289
  55  Gln Val Asp Glu Lys Thr Phe Leu His Tyr Asp Cys Gly Asn Lys Thr Val Thr    72

 290  CCT GTC AGT CCC CTG GGG AAG AAA CTA AAT GTC ACA ACG GCC TGG AAA GCA CAG   343
  73  Pro Val Ser Pro Leu Gly Lys Lys Leu Asn Val Thr Thr Ala Trp Lys Ala Gln    90

 344  AAC CCA GTA CTG AGA GAG GTG GTG GAC ATA CTT ACA GAG CAA CTG CGT GAC ATT   397
  91  Asn Pro Val Leu Arg Glu Val Val Asp Ile Leu Thr Glu Gln Leu Arg Asp Ile   108

 398  CAG CTG GAG AAT TAC ACA CCC AAG GAA CCC CTC ACC CTG CAG GCC AGG ATG TCT   451
 109  Gln Leu Glu Asn Tyr Thr Pro Lys Glu Pro Leu Thr Leu Gln Ala Arg Met Ser   126

 452  TGT GAG CAG AAA GCT GAA GGA CAC AGC AGT GGA TCT TGG CAG TTC AGT TTC GAT   505
 127  Cys Glu Gln Lys Ala Glu Gly His Ser Ser Gly Ser Trp Gln Phe Ser Phe Asp   144

 506  GGG CAG ATC TTC CTC CTC TTT GAC TCA GAG AAG AGA ATG TGG ACA ACG GTT CAT   559
 145  Gly Gln Ile Phe Leu Leu Phe Asp Ser Glu Lys Arg Met Trp Thr Thr Val His   162

 560  CCT GGA GCC AGA AAG ATG AAA GAA AAG TGG GAG AAT GAC AAG GTT GTG GCC ATG   613
 163  Pro Gly Ala Arg Lys Met Lys Glu Lys Trp Glu Asn Asp Lys Val Val Ala Met   180

 614  TCC TTC CAT TAC TTC TCA ATG GGA GAC TGT ATA GGA TGG CTT GAG GAC TTC TTG   667
 181  Ser Phe His Tyr Phe Ser Met Gly Asp Cys Ile Gly Trp Leu Glu Asp Phe Leu   198

 668  ATG GGC ATG GAC AGC ACC CTG GAG CCA AGT GCA GGA GCA CCA CTC GCC ATG TCC   721
 199  Met Gly Met Asp Ser Thr Leu Glu Pro Ser Ala Gly Ala Pro Leu Ala Met Ser   216

 722  TCA GGC ACA ACC CAA CTC AGG GCC ACA GCC ACC ACC CTC ATC CTT TGC TGC CTC   775
 217  Ser Gly Thr Thr Gln Leu Arg Ala Thr Ala Thr Thr Leu Ile Leu Cys Cys Leu   234

 776  CTC ATC ATC CTC CCC TGC TTC ATC CTC CCT GGC ATC TGA GGAGAGTCCTTTAGAGTGA   833
 235  Leu Ile Ile Leu Pro Cys Phe Ile Leu Pro Gly Ile ***                       246

 834  CAGGTTAAAGCTGATACCAAAAGGCTCCTGTGAGCACGGTCTTGATCAAACTCGCCCTTCTGTCTGGCCAGC   905
 906  TGCCCACGACCTACGGTGTATGTCCAGTGGCCTCCAGCAGATCATGATGACATCATGGACCCAATAGCTCAT   977
 978  TCACTGCCTTGATTCCTTTTGCCAACAATTTTACCAGCAGTTATACCTAACATATTATGCAATTTTCTCTTG  1049
1050  GTGCTACCTGATGGAATTCCTGCACTTAAAGTTCTGGCTGACTAAACAAGATATATCATTTTCTTTCTTCTC  1121
1122  TTTTTGTTTGGAAAATCAAGTACTTCTTTGAATGATGATCTCTTTCTTGCAAATGATATTGTCAGTAAAATA  1193
1194  ATCACGTTAGACTTCAGACCTCTGGGGATTCTTTCCGTGTCCTGAAAGAGAATTTTTAAATTATTTAATAAG  1265
1266  AAAAAATTTATATTAATGATTGTTTCCTTTAGTAATTTATTGTTCTGTACTCATATTTAAATAAAGAGTTCT  1337
1338  ATTTCCCAAAAAAAAAAAAAAAAA                                                  1362
```

Fig 6

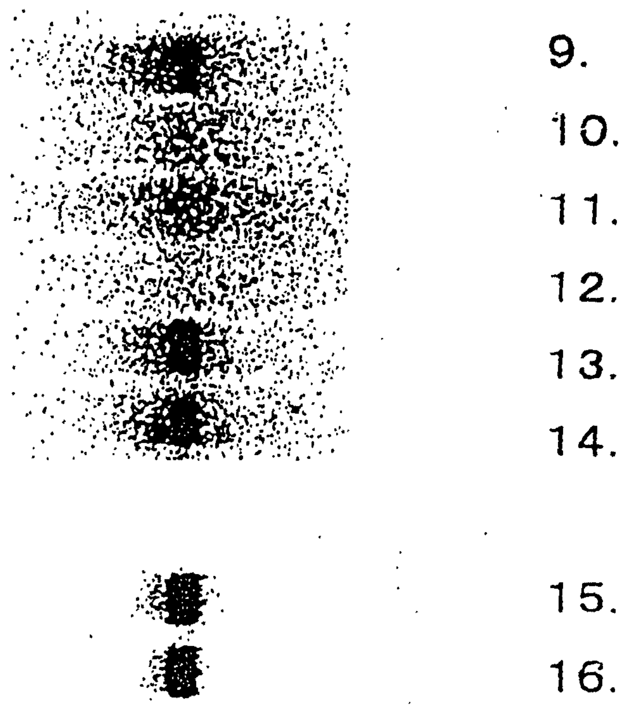

Fig 7

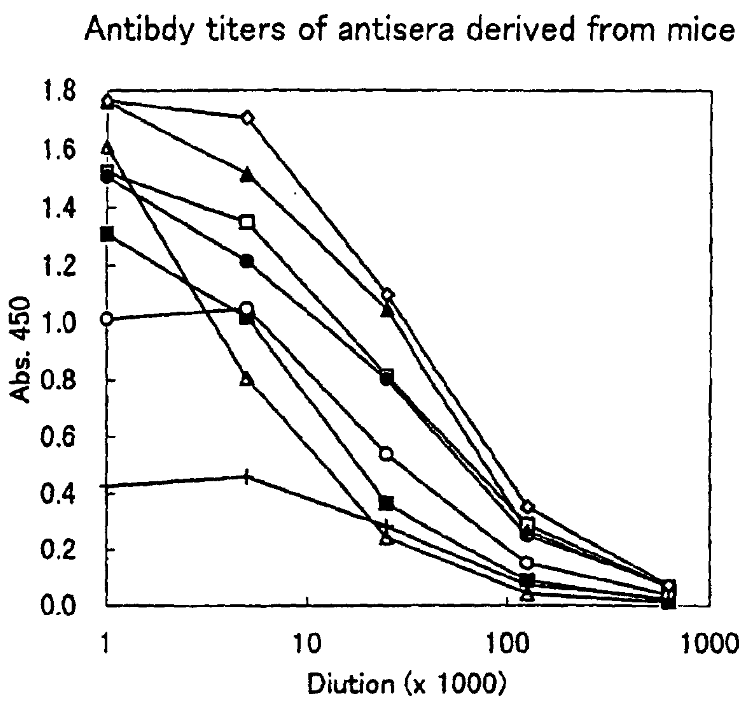
Antibdy titers of antisera derived from mice
1.8
1.6
1.4
1.2
1.0
0.8
0.6
0.4
0.2
0.0
Abs. 450
1
10
100
1000
Diution (x 1000)

Fig 8

Reactivity of monoclonal antibodies

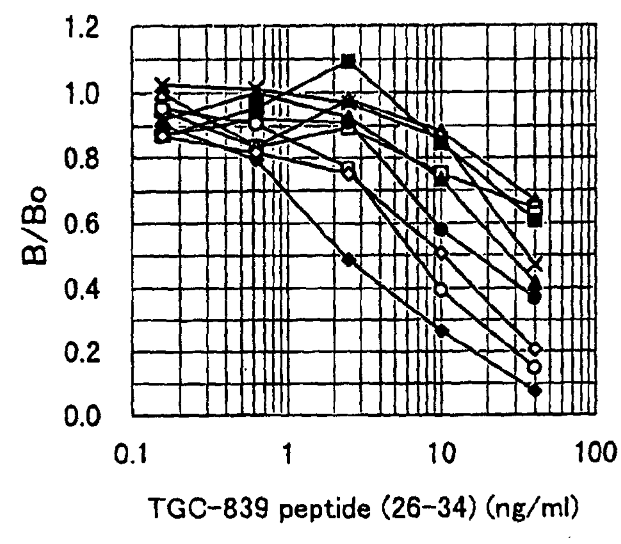

Fig 9

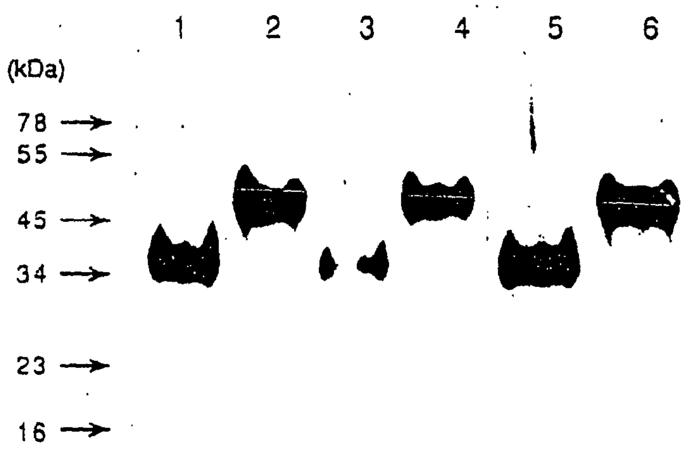

Fig 10

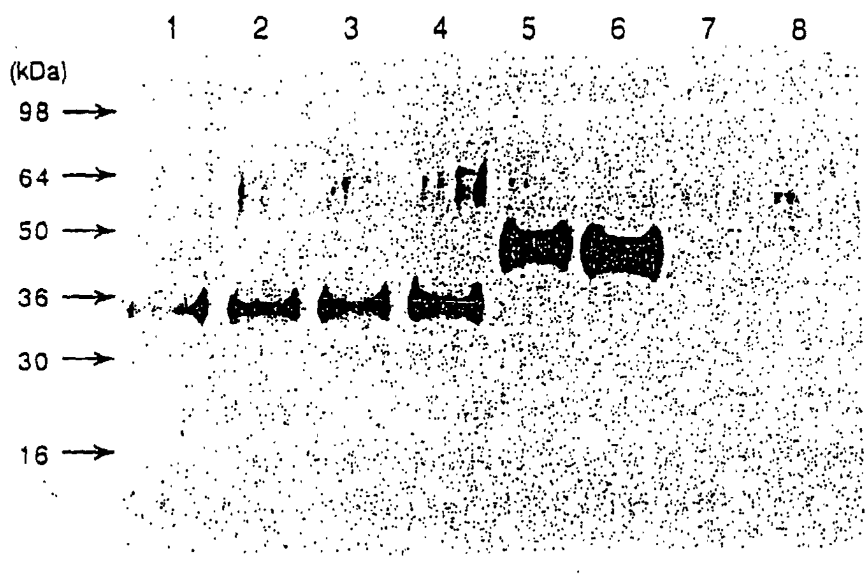

Fig 11

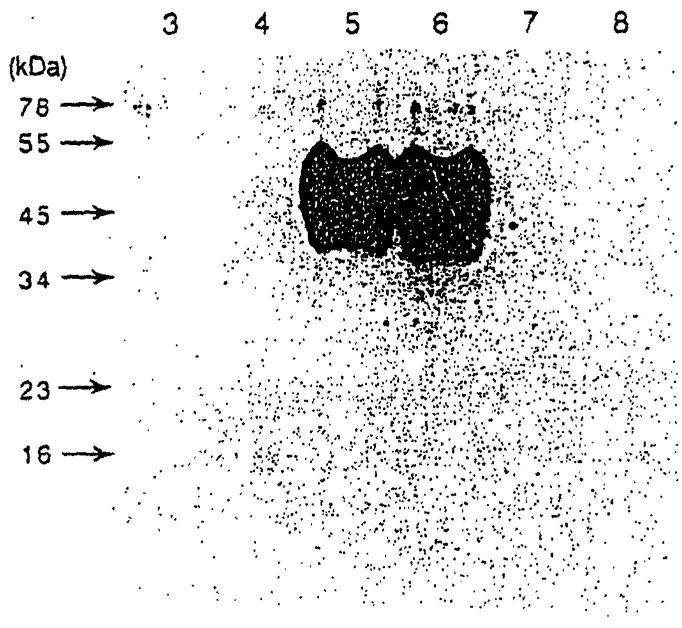

Fig 12

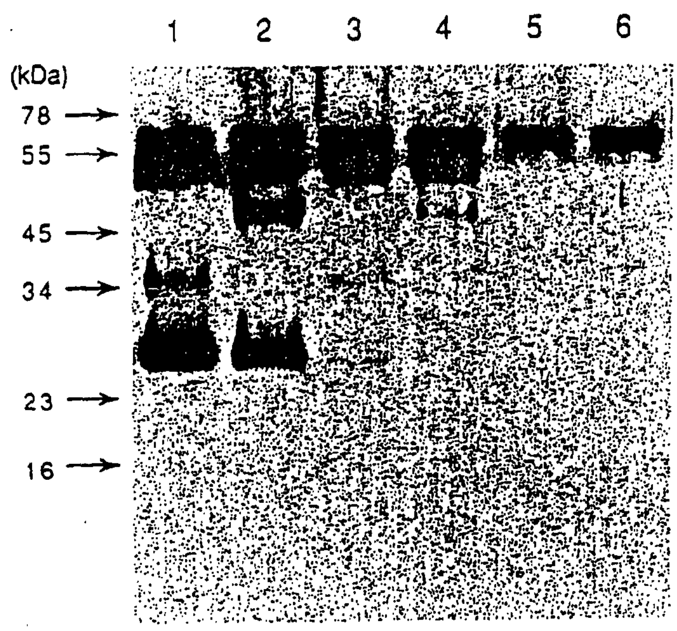

Fig 13

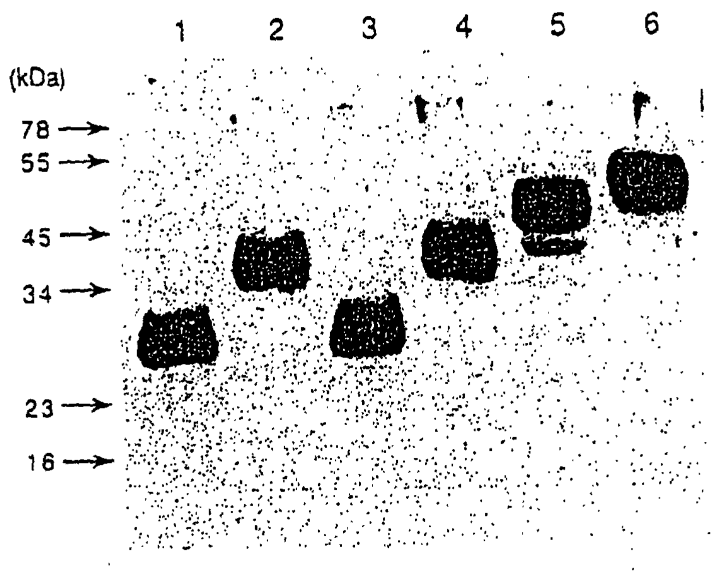

Fig 14

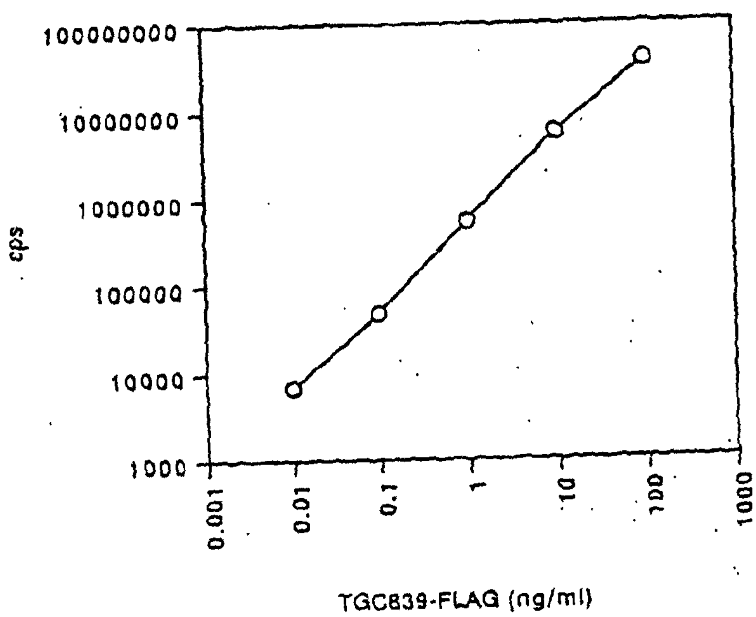
100000000
10000000
1000000
100000
10000
1000
cps
0.001
0.01
0.1
1
10
100
1000
TGC839-FLAG (ng/ml)

Fig 15

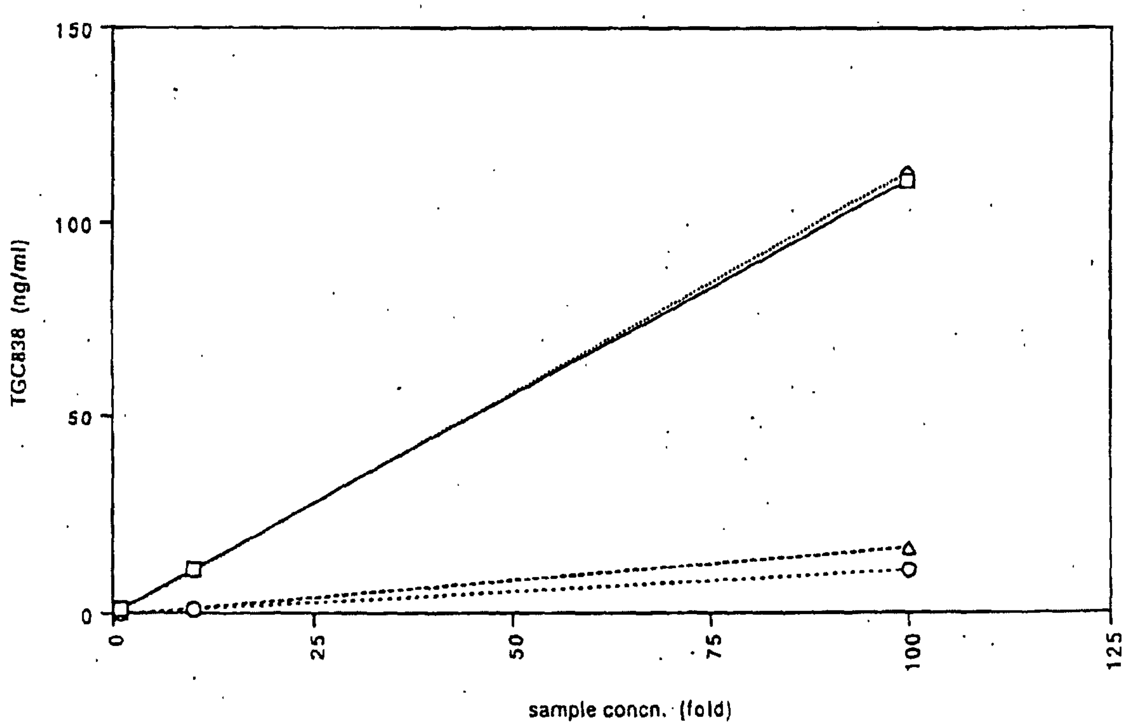

Fig 16

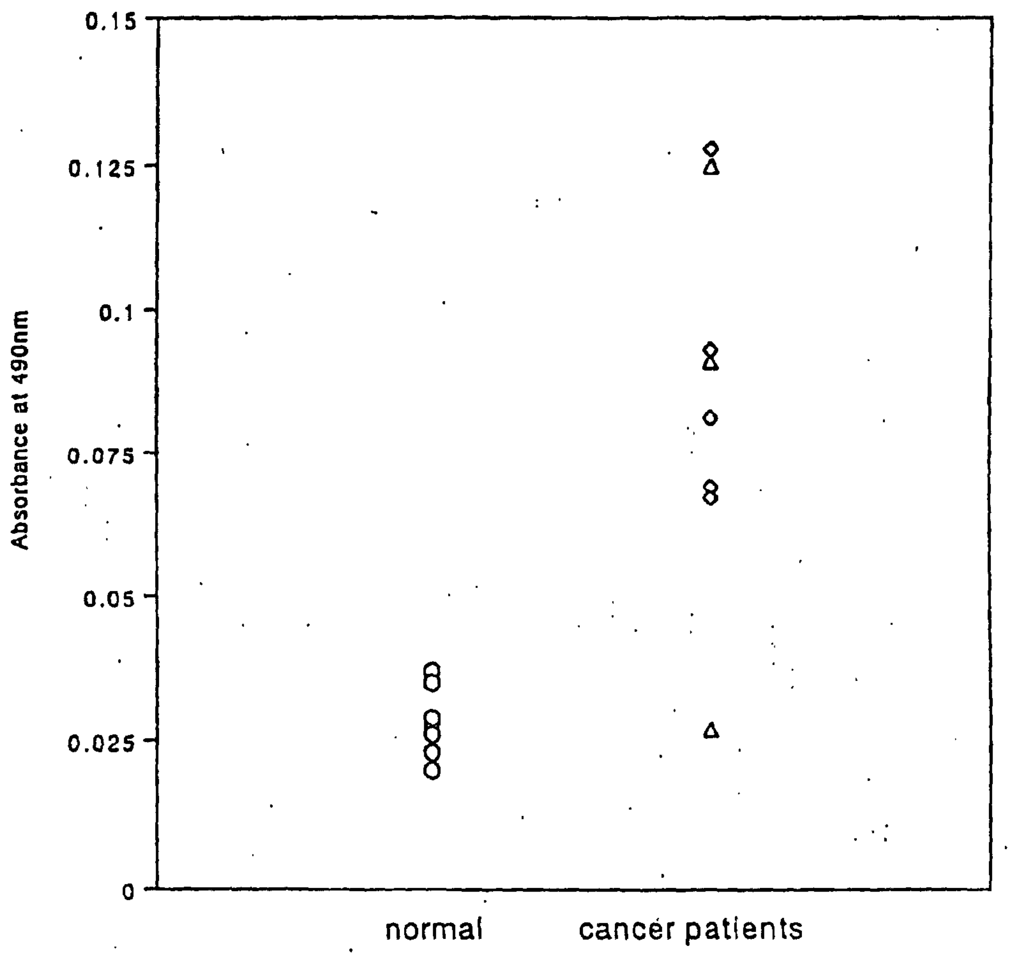
0.15
0.125
0.1
0.075
0.05
0.025
0
Absorbance at 490nm
normal
cancer patients

Fig 17

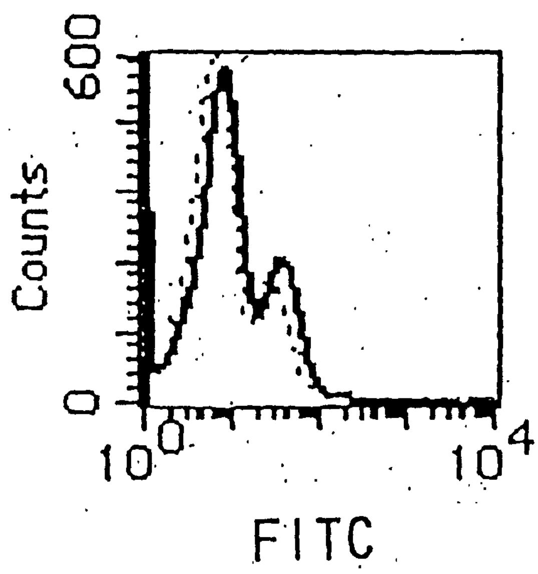

Fig 18

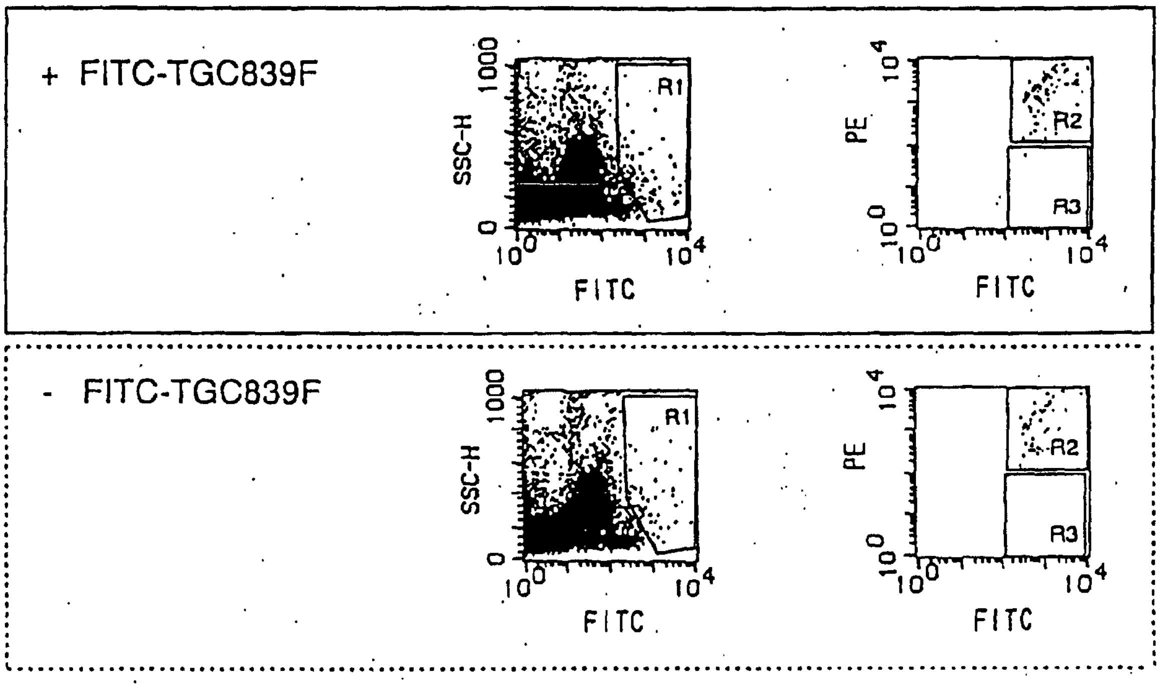
+ FITC-TGC839F
SSC-H
1000
0
R1
10^0
10^4
FITC
PE
10^4
10^0
R2
R3
10^0
10^4
FITC
- FITC-TGC839F
SSC-H
1000
0
R1
10^0
10^4
FITC
PE
10^4
10^0
R2
R3
10^0
10^4
FITC

Fig 19

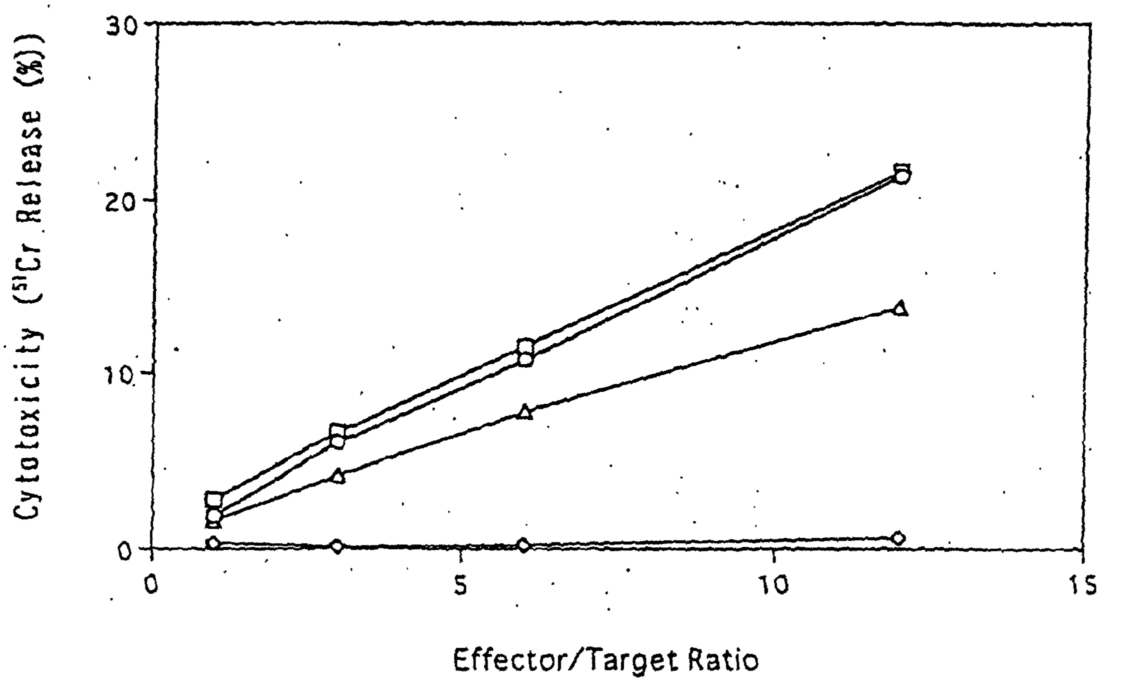
Cytotoxicity (⁵¹Cr Release (%))
30
20
10
0
0
5
10
15
Effector/Target Ratio

Fig 20

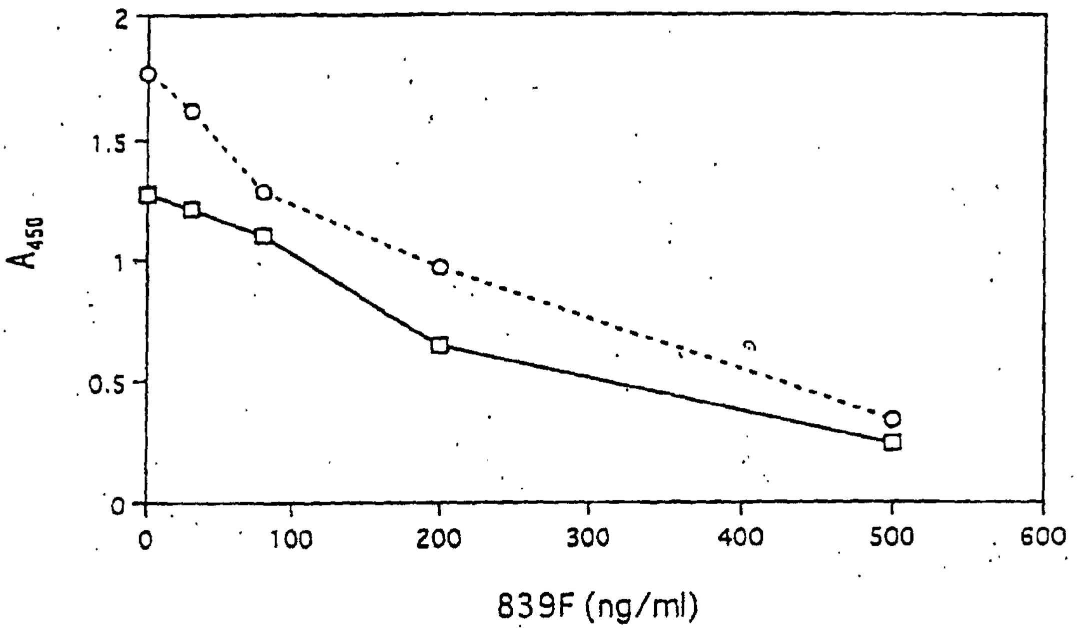

Fig 21

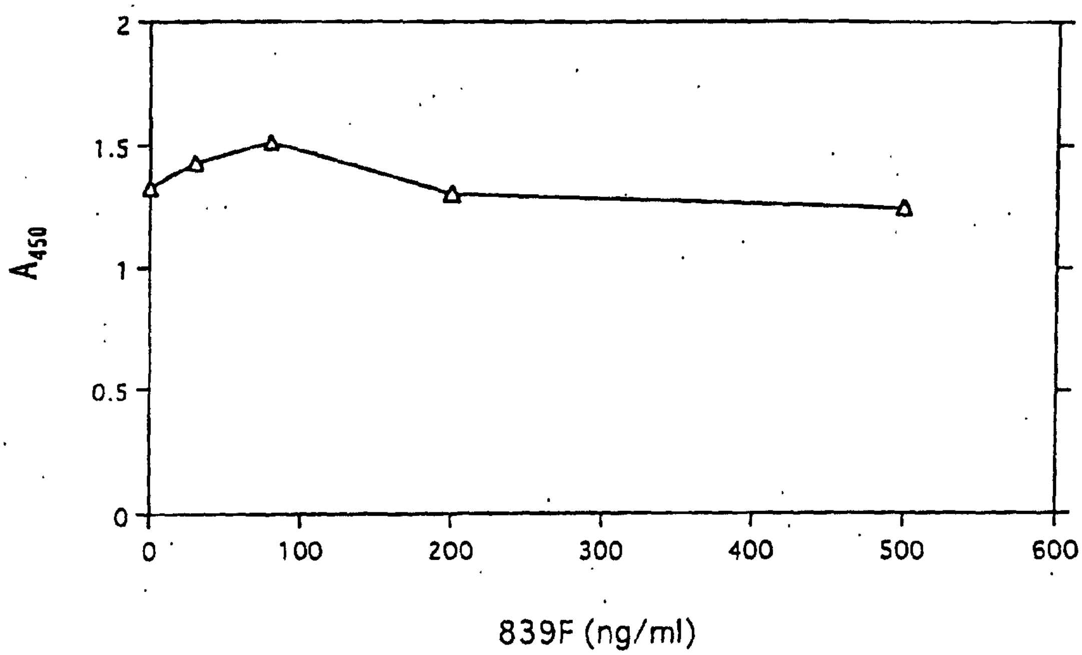

Fig 22

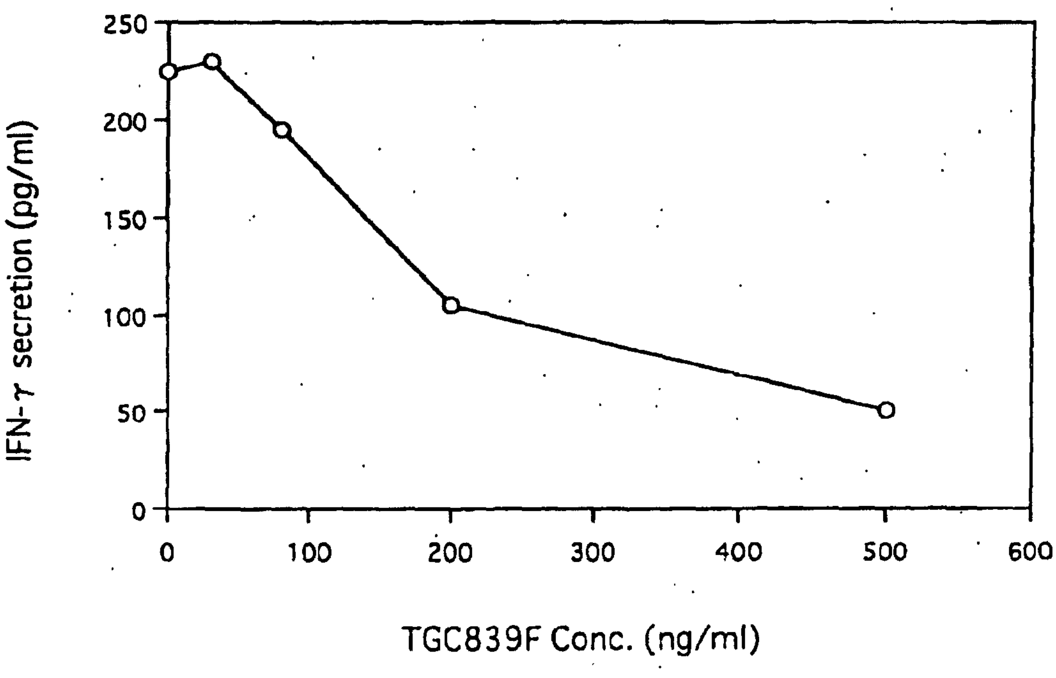
250
200
150
100
50
0
0
100
200
300
400
500
600
IFN-γ secretion (pg/ml)
TGC839F Conc. (ng/ml)

## INTERNATIONAL SEARCH REPORT

International application No.
PCT/JP00/01542

A. CLASSIFICATION OF SUBJECT MATTER
Int.Cl⁷ C07K14/47, C07K7/06, C12N15/12, C12P21/02, C07K16/18, A61K38/17, G01N33/50, C12N5/18//(C12P21/02, C12R1:91)

According to International Patent Classification (IPC) or to both national classification and IPC

B. FIELDS SEARCHED

Minimum documentation searched (classification system followed by classification symbols)
Int.Cl⁷ C07K14/47, C07K7/06, C12N15/12, C12P21/02, C07K16/18, A61K38/17, G01N33/50, C12N5/18

Documentation searched other than minimum documentation to the extent that such documents are included in the fields searched

Electronic data base consulted during the international search (name of data base and, where practicable, search terms used)
BIOSIS(DIALOG),WPI(DIALOG),SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| PX | WO, 99/31241, A1 (IMMUNEX CORP), 24 June, 1999 (24.06.99) & AU, 9920045, A | 1-26 |
| PX/PA | WO, 99/63088, A2 (GENENTECH INC), 09 December, 1999 (09.12.99), & AU, 9943286, A | 1-18,20-23/19, 24-26 |
| A | PENG LIANG et al., "Ras activation of genes: Mob-1 as a model", Proc.Natl.Acad.Sci.USA, (1994), Vol.91, No.26, pp.12515-12519 | 1-26 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May, 2000 (29.05.00) | 06 June, 2000 (06.06.00) |
| Name and mailing address of the ISA/ Japanese Patent Office | Authorized officer |
| Facsimile No. | Telephone No. |

Form PCT/ISA/210 (second sheet) (July 1992)